# EUROPÄISCHE PATENTSCHRIFT

(11) **EP 2 433 617 B1**
(45) Veröffentlichungstag und Bekanntmachung des Hinweises auf die Patenterteilung: **15.05.2019**
(21) Anmeldenummer: 11180715.2
(22) Anmeldetag: 09.09.2011
(51) Int. Cl.: A61K 8/11, A61K 8/84, A61K 8/81, A61Q 5/02, A61Q 5/10, A61Q 5/12, A61Q 15/00, A61Q 19/10

(54) **Kosmetisches Mittel mit Mikrokapseln**
Cosmetic composition containing microcapsules
Produit cosmétique doté de microcapsules

(30) Priorität: 10.09.2010 DE 102010040567
(43) Veröffentlichungstag der Anmeldung: 28.03.2012
(73) Patentinhaber: Henkel AG & Co. KGaA, 40589 Düsseldorf (DE)
(72) Erfinder: Huchel, Ursula, 50670 Köln (DE); Bauer, Andreas, 41564 Kaarst (DE); Sunder, Matthias, 40593 Düsseldorf (DE)

(56) Entgegenhaltungen:
- EP-A1- 2 364 773
- WO-A1-2009/100553
- WO-A2-2010/102830
- JP-A- H06 312 128
- US-A- 3 755 190
- US-A- 5 071 706

## Beschreibung

Die Erfindung liegt auf dem Gebiet der kosmetischen Mittel und betrifft Kosmetika, die spezielle Mikrokapseln enthalten.

Eine Vielzahl von Kosmetika bestehen aus einer flüssigen oder pastösen Phase, worin die Aktivstoffe in gelöster oder fein dispergierter Form vorliegen. Solche Kosmetika, insbesondere flüssige Mittel, haben den Nachteil, dass darin enthaltende mit der Formulierung unverträgliche, wirksame Bestandteile, wie Farb-, Duftstoffe, Pflegeöle, Vitamine, Enzyme, antibakterielle Wirkstoffe, Säuren, Basen und/oder Oxidationsmittel, die in solchen Formulierungen eingesetzt werden, häufig schon bei der Lagerung und/oder vor deren gewünschten Anwendungszeitpunktes ihre Aktivität durch chemische Reaktionen und/oder physikalische Einflüsse, verlieren oder zumindest stark reduziert werden.

Es existieren bereits zahlreiche kommerzielle Verkapselungssysteme, die auf natürlichen oder künstlichen Polymeren basieren. Diese können einen Wirkstoff oder dessen Lösung umschliessen und dann in der Hülle physikalisch oder chemisch vernetzt werden oder durch einen Koazervationsprozess mit einem anderen Polymer ausgefällt werden. Weiterhin existieren Verkapselungen durch Liposome, z. B. "Nanotopes" von der Firma Ciba-Geigy, oder schwammartige Partikel wie "Mikrosponges" von der Firma Advanced Polymer Systems.

Beispielsweise werden mikroverkapselte Formkörper zur Stabilitätserhöhung von pharmazeutischen Wirkstoffen, zur Geschmacksbeeinflussung, zur gezielten organspezifischen Wirkstoffabgabe sowie zur Vermeidung von Unverträglichkeiten mit anderen Hilfs- und Wirkstoffen eingesetzt. Darüber hinaus finden Mikrokapseln in der Klebstofftechnologie Anwendung. Bekannt sind außerdem auch Duftstoffkapseln mit Gelatine als Wandmaterial, aus denen durch mechanische Zerstörung Parfümöle freigesetzt werden. Außer "echten" Mikrokapseln, die eine Hülle/Kern-Struktur besitzen, gibt es kugelförmige Trägerpartikel z. B. aus Alginat, Gelatine oder Polyvinylalkohol (PVAI), in die ein Wirkstoff, lebende Zellen oder Enzyme eingebettet werden können. Diese Kapseln können z. B. durch ein Vertropfungsverfahren hergestellt werden. Allgemein handelt es sich bei Mikrokapseln um Teilchen mit Durchmessern von < 1 mm. Neben dem Einschluss in Kapseln verschiedener Grösse können Substanzen auch auf geeigneten Trägermaterialien adsorbiert oder chemisch modifiziert werden.

Im Stand der Technik wurden, wie oben beschrieben, zahlreiche Anstrengungen unternommen, solche Wirkbestandteile durch Verkapselung, Beschichtung oder dergleichen zu schützen.

Nachteilig ist, dass derartig geschützte Bestandteile in den Mitteln schon während der Lagerung, insbesondere in flüssigen Waschmitteln, Reinigungsmitteln und/oder Pflegemitteln, an Aktivität verlieren, da die Materialien der Verkapselungen, Beschichtungen oder dergleichen in erhöhtem Umfang in Lösung gehen und dadurch ihre Schutzwirkung verlieren. Außerdem tragen verstärkt Migrations- und Diffusionseffekte zur Stofffreisetzung, wie Schlierenbildung in Lösungen, und/oder zu Aktivitätsverlusten von Wirkstoffbestandteilen bei.

Ferner haben die im Stand der Technik bekannten Verkapselungen, Beschichtungen, Träger oder dergleichen den Nachteil, dass die auf diese Weise geschützten, adsorbierten oder chemisch modifizierten Bestandteile bereits während der Lagerung freigesetzt werden, und dass eine Freisetzung von Wirksubstanzen am Ort der spezifischen Anwendung nicht gesteuert werden kann.

Für viele kosmetische Mittel ist es auch aus ästhetischen Gründen gewünscht, Bestandteile des Mittels separat in abgegrenzter Form dem Mittel zuzugeben, beispielsweise in Form von Kapseln, Kugeln, Tropfen, als zweite Phase bzw. als weitere Phase und/oder dergleichen. Neben ästhetischen Effekten, werden durch solche räumlichen Abgrenzungen, wie oben aufgeführt, verbesserte Aktivitätsstabilitäten während der Lagerung und/oder bei Verdünnung angestrebt. Insbesondere bei Farbstoffen, die häufig Bestandteile von Schutzschichten, wie Umhüllungen, Beschichtungen und dergleichen sind, lässt sich bei solchen Mitteln des Standes der Technik, selbst in hochkonzentrierten Lösungen, bei Lagerung über einen verlängerten Zeitraum, d. h. Wochen oder Monate, eine Schlierenbildung - "so genanntes Ausbluten" - um den Formkörper beobachten. Neben den Farbstoffen, deren Schlierenbildung für den Anwender bereits mit bloßem Auge erkennbar ist, bereitet der ungewollte, vorzeitige Austritt von Duftstoffen große Probleme.

Aus dem Stand der Technik sind Mikrokapseln bekannt, die als Kernmaterial flüssige, feste oder gasförmige Stoffe enthalten können. Als Material für die Kapselwände sind beispielsweise Phenol-Formaldehyd-Polymere, Melamin-Formaldehyd-Polymere, Polyurethan, Gelatine, Polyamide oder Polyharnstoffe gebräuchlich. Weit verbreitet ist beispielsweise die Verwendung Leukofarbstoff-gefüllter Mikrokapseln zur Herstellung selbstdurchschreibender Papiere.

Aus US 3,755,190 ist bekannt, dass Kapseln aus Phenol-Formaldehyd-Polymeren brüchige Wände aufweisen. Um dies zu vermeiden, wird ein Herstellverfahren beschrieben, bei welchem vollständig hydrolysierter Polyvinylalkohol eingesetzt wird.

Dispersionen von Mikrokapseln aus Aminoplastharzen, wie Melamin-Formaldehyd-Harzen, enthalten herstellungsbedingt einen gewissen Anteil freien Formaldehyds. Es ist in kosmetischen Mitteln unerlässlich, den Formaldehydgehalt so niedrig wie möglich zu halten, wenn möglich Formaldehyd ganz zu vermeiden. Zur Verringerung des Formaldehyd-Gehalts ist es üblich, Mikrokapseldispersionen auf der Basis von Melamin-Formaldehyd-Harzen Formaldehydfänger zuzusetzen. Zu den am häufigsten verwendeten Formaldehydfängern gehören Ammoniak, Harnstoff, Ethylenharnstoff und Melamin, die den Restgehalt an Formaldehyd in der Kapseldispersion reduzieren.

Aus EP 383358 und DE 3814250 sind lichtempfindliche Materialien bekannt, die aus Mikrokapseln bestehen, deren Wände aus Melamin-Formaldehyd-Harzen gebildet werden. Zur Entfernung überschüssigen Formaldehyds wird bei der Härtung Harnstoff zugesetzt. Bei den in EP 319337 und US 4918317 beschriebenen Verfahren wird Harnstoff gegen Ende der Härtung zugesetzt. EP 415273 beschreibt die Herstellung und Verwendung mono- und polydisperser Vollkugelteilchen aus Melamin-Formaldehyd-Kondensat. Zur Bindung des bei der Kondensation freiwerdenden Formaldehyds wird die Verwendung von Ammoniak, Harnstoff oder Ethylenharnstoff vorgeschlagen.

DE 19833347 beschreibt ein Verfahren zur Herstellung von Mikrokapseln durch Kondensation von Melamin-Formaldehyd-Harzen und/oder deren Methylethern, wobei vor der Härtung Harnstoff oder Harnstoff, dessen Aminogruppen mit einer Ethylen- oder Propylenbrücke verbunden sind, als Formaldehydfänger zugesetzt wird. Die erhaltenen Dispersionen sind zwar formaldehydarm, durch den Zusatz von Harnstoff vor der Härtung werden jedoch die Stabilität der Mikrokapseln und die Viskosität der Mikrokapseldispersion ungünstig beeinflusst.

WO 01/51197 lehrt ein Verfahren zur Herstellung von Mikrokapseln durch Kondensation von Melamin-Formaldehyd-Harzen, bei dem während der Härtung ein Gemisch aus Melamin und Harnstoff zugesetzt wird.

Die WO 2010/102830 A2 offenbart Mikrokapseln, die durch Umsetzung von einem aromatischen Alkohol mit einem Aldehyd in Gegenwart eines Homo- oder Copolymers von 2-Acrylamido-2-methyl-propansulfonsäure erhältlich sind.

Die EP 2364773 A1 beschreibt Mikrokapseln, die durch Umsetzung von einem aromatischen Alkohol mit einem Aldehyd in Gegenwart eines Homo- oder Copolymers von 2-Acrylamido-2-methyl-propansulfonsäure erhältlich sind.

Aus der JP 06-312128 A sind Mikrokapseln, die durch Umsetzung von einem aromatischen Alkohol mit einem Aldehyd in Gegenwart eines Säurekatalysators erhältlich sind, bekannt.

Durch den Zusatz der genannten Formaldehydfänger zur fertigen Mikrokapseldispersion bzw. bei der Herstellung der Mikrokapseldispersion wird regelmäßig der Formaldehydgehalt der Dispersion gesenkt. Oft lässt sich jedoch der Formaldehydgehalt von Produkten, die derartige Mikrokapseldispersionen enthalten oder damit behandelt wurden, auch bei Zugabe großer Mengen an Formaldehydfänger nicht unter eine bestimmte Grenze senken.

Aufgabe der vorliegenden Erfindung ist es deshalb, Kosmetika bereitzustellen, die Mikrokapseln mit einem möglichst niedrigen Formaldehydgehalt beinhalten bzw. bei denen bevorzugt auf die Verwendung von Formaldehyd ganz verzichtet wurde.

Eine weitere Aufgabe der vorliegenden Erfindung bestand darin, kosmetische Mittel bereitzustellen, die lager- und ausblutungsstabil eingearbeitete formaldehydarme bzw. -freie Kapseln enthalten. Dabei sollte eine größtmögliche Lager- und Ausblutungsstabilität auch in hoch-wasserhaltigen Formulierungen erreicht werden.

Überraschend wurde gefunden, dass bestimmte Kapselmaterialien in kosmetischen Mitteln hervorragend stabile Kapseln liefern und zudem eine Kontamination des Kosmetikums mit Formaldehyd gänzlich ausschließen.

Ein erster Gegenstand der vorliegenden Erfindung sind kosmetische Zusammensetzungen, enthaltend - bezogen auf ihr Gewicht -
a) Mikrokapseln, deren Kapselwände ein Harz umfassen, welches durch Umsetzung
   i) mindestens eines aromatischen Alkohols oder dessen Ether oder Ester und
   ii) mindestens einer aldehydischen Komponente, die mindestens zwei C-Atome pro Molekül aufweist, und
   iii) in Gegenwart mindestens eines Homo- oder Copolymers von 2-Acrylamido-2-methyl-propansulfonsäure oder dessen Salzen,
      erhältlich ist
b) 0,01 bis 70 Gew.-% mindestens eines kosmetischen Wirkstoffes aus der Gruppe
   b1) der Oxidationsfarbstoffvorprodukte und/oder
   b2) der direktziehenden Farbstoffe und/oder
   b3) der Oxidationsmittel, ausgewählt aus Wasserstoffperoxid und dessen Anlagerungsverbindungen an feste Träger, und/oder
   b4) der Tenside und/oder Emulgatoren und/oder
   b5) der haarkonditionierenden Wirkstoffe und/oder
   b6) der desodorierenden und/oder der schweißhemmenden Wirkstoffe und/oder
   b7) der hautaufhellenden und/oder hautberuhigenden und/oder feuchtigkeitsspendenden Wirkstoffe und/oder
   b8) der anorganischen und/oder organischen UV-Filtersubstanzen und/oder
   b9) der sebumregulierenden Wirkstoffe und/oder der mechanischen Exfoliationsmittel und/oder der antimikrobiellen Wirkstoffe und/oder
   b10) der haarfestigenden oder Haarstyling-Wirkstoffe und/oder
   b11) der Antikaries-Wirkstoffe und/oder
   b12) der Wirkstoffe gegen Zahnsteinbildung und/oder
   b13) der Mischungen dieser Wirkstoffe b1) - b12).

Als ersten Inhaltsstoff enthalten die erfindungsgemäßen kosmetischen Mittel Mikrokapseln, deren Kapselwände ein Harz umfassen, welches durch Umsetzung spezieller Komponenten erhältlich ist. Diese Komponenten a)i), a)ii) und a)iii) werden nachstehend beschrieben.

Als aromatische Alkohole a)i) sind im Rahmen der vorliegenden Erfindung Aryloxyalkanole, Arylalkanole und Oligoalkanolarylether bevorzugt. Ebenfalls bevorzugt sind aromatische Verbindungen, bei denen mindestens eine freie Hydroxygruppe, besonders bevorzugt mindestens zwei freie Hydroxy-Gruppen unmittelbar aromatisch gebunden sind, wobei es besonders bevorzugt ist, wenn mindestens zwei freie Hydroxy-Gruppen unmittelbar an einen aromatischen Ring gebunden sind und ganz besonders bevorzugt in meta-Stellung zueinander angeordnet sind. Es ist bevorzugt, dass die aromatischen Alkohole ausgewählt sind aus Phenol, den Kresolen (o-, m- und p-Kresol), den Naphtholen (α- und β-Naphthol) und Thymol, sowie aus den Ethylphenolen, Propylphenolen, Fluorphenolen und Methoxyphenolen.

Erfindungsgemäß bevorzugte aromatische Alkohole sind außerdem solche, die bei der Herstellung von Polycarbonat-Kunststoffen und Epoxydharzlacken verwendet werden, insbesondere 2,2-Bis-(4-hydroxyphenyl)-propan (Bisphenol A). Ganz besonders bevorzugt ist es, wenn der erfindungsgemäß vorliegende aromatische Alkohol ausgewählt ist aus den Phenolen mit zwei oder mehr Hydroxygruppen, vorzugsweise aus Brenzcatechin, Resorcin, Hydrochinon und 1,4-Naphthohydrochinon, Phloroglucin, Pyrogallol, Hydroxyhydrochinon, wobei insbesondere Resorcin und/oder Phloroglucin als aromatische Alkohole bevorzugt sind.

Zusammenfassend sind erfindungsgemäße Zusammensetzungen bevorzugt, bei denen der mindestens eine aromatische Alkohol a)i) ausgewählt ist aus Phenol, den Kresolen (*o*-, *m*- und *p-*Kresol), den Naphtholen (α- und β-Naphthol), Thymol, Brenzcatechin, Resorcin, Hydrochinon und 1,4-Naphthohydrochinon, Phloroglucin, Pyrogallol, Hydroxyhydrochinon.

In einer weiteren Ausführungsform der vorliegenden Erfindung enthalten die kosmetischen Mittel Mikrokapseln, bei deren Herstellung der aromatische Alkohol als Ether eingesetzt wird, wobei der Ether in einer bevorzugten Ausführungsform ein Derivat der jeweiligen freien Form des umzusetzenden aromatischen Alkohols a)i) ist. Der freie Alkohol kann dabei ebenso vorhanden sein; dann liegt demnach eine Mischung vor. Für diesen Fall kann das molare Verhältnis zwischen der freien Form des erfindungsgemäß umzusetzenden aromatischem Alkohols und der genannten zusätzlichen Komponente (Etherform eines aromatischen Alkohols) zwischen 0:100, bevorzugt, bevorzugt 1:1, oder 1:2 oder 1:4 betragen.

Der Vorteil der Mischung des aromatischen Alkohols mit einer Ether-Form ist darin begründet, dass damit die Reaktivität des Systems beeinflussbar ist. Insbesondere lässt sich mit der geeigneten Auswahl des Verhältnisses ein System schaffen, dessen Reaktivität in einem ausgewogenen Verhältnis zu der Lagerstabilität des Systems steht. Der aromatische Alkohol kann in Form eines Ester eingesetzt werden.

Als Aldehyde a)ii) mit mindestens 2 C-Atomen sind gemäß der vorliegenden Erfindung sowohl aliphatische als auch aromatische Aldehyde bevorzugt. Besonders bevorzugte Aldehyde sind eine oder mehrere ausgewählt aus der folgenden Gruppe Valeraldehyd, Capronaldehyd, Caprylaldehyd, Decanal, Succindialdehyd, Cyclohexancarbaldehyd, Cyclopentancarbaldehyd, 2-Methyl-1-propanal, 2-Methylpropionaldehyd, Acetaldehyd, Acrolein, Aldosteron, Antimycin A, 8'-Apo-β-caro-ten-8'-al, Benzaldehyd, Butanal, Chloral, Citral, Citronellal, Crotonaldehyd, Dimethylaminobenzaldehyd, Folinsäure, Fosmidomycin, Furfural, Glutaraldehyd, Glycerinaldehyd, Glykolaldehyd, Glyoxal, Glyoxylsäure, Heptanal, 2-Hydroxybenzaldehyd, 3-Hydroxybutanal, Hydroxymethylfurfural, 4-Hydroxynonenal, Isobutanal, Isobutyraldehyd, Methacrolein, 2-Methylundecanal, Mucochlorsäure, N-Methylformamid, 2-Nitrobenzaldehyd, Nonanal, Octanal, Oleocanthal, Orlistat, Pentanal, Phenylethanal, Phycocyanin, Piperonal, Propanal, Propenal, Protocatechualdehyd, Retinal, Salicylaldehyd, Secologanin, Streptomycin, Strophanthidin, Tylosin, Vanillin, Zimtaldehyd.

Im Sinne der vorliegenden Erfindung kann die aldehydische Komponente mindestens ein oder zwei, besonders bevorzugt zwei, drei oder vier, ganz besonders bevorzugt zwei freie Aldehyd-Gruppen pro Molekül, aufweisen, wobei es bevorzugt ist, wenn als aldehydische Komponente mindestens Glyoxal, Glutar- und/oder Succindialdehyd vorliegt, besonders bevorzugt ist Glutardialdehyd.

In den erfindungsgemäßen Mikrokapseln kann das molare Verhältnis von a) dem mindestens einen aromatischen Alkohol oder (Ether oder Ester davon), zu b) der mindestens einen aldehydischen Komponente im Allgemeinen zwischen 1:1 und 1:5, besonders bevorzugt zwischen 1 zu 2 und 1 zu 3 und ganz besonders bevorzugt bei Resorcin bei etwa 1 zu 2,6 liegen. Das Gewichts-Verhältnis der Komponenten a) + b) zu c), d.h. das Verhältnis der Gewichts-Summe von a) + b)) zum Gewicht der Komponente c) liegt im Allgemeinen zwischen 1 : 1 und 1: 0,01, besonders bevorzugt zwischen 1 : 0,2 und 1 : 0,05.

Zusammenfassend sind erfindungsgemäße Zusammensetzungen bevorzugt, bei denen die aldehydische Komponente a)ii) ausgewählt ist aus Valeraldehyd, Capronaldehyd, Caprylaldehyd, Decanal, Succindialdehyd, Cyclohexancarbaldehyd, Cyclopentancarbaldehyd, 2-Methyl-1-propanal, 2-Methylpropionaldehyd, Acetaldehyd, Acrolein, Aldosteron, Antimycin A, 8'-Apo-β-caroten-8'-al, Benzaldehyd, Butanal, Chloral, Citral, Citronellal, Crotonaldehyd, Dimethylaminobenzaldehyd, Folinsäure, Fosmidomycin, Furfural, Glutaraldehyd, Glycerinaldehyd, Glykolaldehyd, Glyoxal, Glyoxylsäure, Heptanal, 2-Hydroxybenzaldehyd, 3-Hydroxybutanal, Hydroxymethylfurfural, 4-Hydroxynonenal, Isobutanal, Isobutyraldehyd, Methacrolein, 2-Methylundecanal, Mucochlorsäure, N-Methylformamid, 2-Nitrobenzaldehyd, Nonanal, Octanal, Oleocanthal, Orlistat, Pentanal, Phenylethanal, Phycocyanin, Piperonal, Propanal, Propenal, Protocatechualdehyd, Retinal, Salicylaldehyd, Secologanin, Streptomycin, Strophanthidin, Tylosin, Vanillin, Zimtaldehyd. Bevorzugte Homo- oder Copolymerevon 2-Acrylamido-2-methyl-propansulfonsäure oder dessen Salzen (AMPS sind Copolymere von 2-Acrylamido-2-methyl-propansulfonsäure oder dessen Salzen, z.B. Copolymere mit einem oder mehreren Comonomeren aus der Gruppe der (Meth)Acrylate, der Vinylverbindungen wie Vinylester oder Styrole, der ungesättigten Di- oder Polycarbonsäuren wie Maleinsäureester, oder der Salze von Amylverbindungen oder Allylverbindungen. Nachfolgend werden bevorzugte.

Comonomere für AMPS genannt, diese Comonomere können jedoch auch mit anderen polar funktionalisierten (Meth)Acrylat-monomeren copolymerisiert werden:
1) Vinylverbindungen, z.B. Vinylester wie Vinylacetat, Vinyllaurat, Vinylpropionat oder Vinylester der Neononansäure, oder aromatische Vinylverbindungen wie Styrol-Comonomere, beispielsweise Styrol, alpha-Methylstyrol oder polar funktionalisierte Styrole wie Styrole mit Hydroxy-, Amino-, Nitril-, Carbonsäure-, Phosphonsäure-, Phosphorsäure-, Nitro- oder SulfonsäureGruppen und deren Salze, wobei die Styrole bevorzugt in para-Position polar funktionalisiert sind.
2) Ungesättigte Di- oder Polycarbonsäuren, z.B. Maleinsäureester wie Dibutylmaleinat oder Dioctylmaleinat, als Salze von Allylverbindungen z.B. Natriumallylsulfonat, als Salze von Amylderivaten z.B. Natriumamylsulfonat.
3) (Meth)Acrylat-Comonomere, dies sind Ester der Acrylsäure und Methacrylsäure, wobei die Estergruppen z.B. gesättigte oder ungesättigte, geradkettige, verzweigte oder cylische Kohlenwasserstoffreste sind, welche eines oder mehrere Heteroatome wie N, O, S, P, F, Cl, Br, I enthalten können. Beispiele solcher Kohlenwasserstoffreste sind geradkettiges, verzweigtes oder cyclisches Alkyl, geradkettiges, verzweigtes oder cylisches Alkenyl, Aryl wie Phenyl oder Heterocyclyl wie Tetrahydrofurfuryl.

Als (Meth)Acrylat-Comonomere, vorzugsweise für AMPS, kommen beispielsweise in Frage:
a) Acrylsäure, C₁-C₁₄-Alkyl-Acrylsäure wie Methacrylsäure,
b) (Meth)Acrylamide wie Acrylamid, Methacrylamid, Diaceton-Acrylamid, Diaceton-Methacrylamid, N-Butoxymethyl-Acrylamid, N-iso-Butoxymethyl-Acrylamid, N-Butoxymethyl-Methacrylamid, N-iso-Butoxymethyl-Methacrylamid, N-Methylol-Acrylamid, N-Methylol-Methacrylamid;
c) Heterocyclyl-(Meth)Acrylate wie Tetrahydrofurfuryl-acrylat und Tetrahydrofurfuryl-methacrylat oder carbocyclische (Meth)Acrylate wie Isobornyl-acrylat und Isobornyl-methacrylat,
d) Urethan(Meth)Acrylate wie Diurethandiacrylat und Diurathanmethacrylat (CAS: 72869-86-4),
e) C₁-C₁₄-Alkylacrylate wie Methyl-, Ethyl-, n-Propyl-, iso-Propyl-, n-Butyl-, sec. Butyl-, iso-Butyl-, tert. Butyl-, n-Pentyl-, iso-Pentyl-, Hexyl- (z.B. n-Hexyl, iso-Hexyl oder Cyclohexyl), Heptyl-, Octyl-(z.B. 2-Ethylhexyl), Nonyl-, Decyl- (z.B. 2-Propylheptyl oder iso-Decyl), Undecyl-, Dodecyl-, Tridecyl- (z.B. iso-Tridecyl), und Tetradecyl-Acrylat; die Alkylgruppen können optional mit einem oder mehreren Halogenatomen (z.B. Fluor, Chlor, Brom oder Iod) substituiert sein, z.B. Trifluoroethyl-Acrylat, oder mit einer oder mehreren Aminogruppen, z.B. Diethylaminoethyl-Acrylat, oder mit einer oder mehreren Alkoxygruppen wie Methoxypropyl-Acrylat, oder mit einer oder mehreren Aryloxygruppen wie Phenoxyethyl-Acrylat.
f) C₂-C₁₄-Alkenylacrylate wie Ethenyl-, n-Propenyl-, iso-Propenyl-, n-Butenyl-, sec. Butenyl-, iso-Butenyl-, tert. Butenyl-, n-Pentenyl-, iso-Pentenyl-, Hexenyl- (z.B. n-Hexenyl, iso-Hexenyl oder Cyclohexenyl), Heptenyl-, Octenyl- (z.B. 2-Ethylhexenyl), Nonenyl-, Decenyl- (z.B. 2-Propenylheptyl oder iso-Decenyl), Undecenyl-, Dodecenyl-, Tridecenyl- (z.B. iso-Tridecenyl), und Tetradecenyl-Acrylat, und deren Epoxide wie Glycidyl-Acrylat oder Aziridine wie Aziridin-Acrylat;
g) C₁-C₁₄-Hydroxyalkylacrylate wie Hydroxymethyl-, Hydroxyethyl-, Hydroxy-n-Propyl-, Hydroxy-iso-Propyl-, Hydroxy-n-Butyl-, Hydroxy-sec.-Butyl-, Hydroxy-iso-Butyl-, Hydroxy-tert.-Butyl-, Hydroxy-n-Pentyl-, Hydroxy-iso-Pentyl-, Hydroxyhexyl- (z.B. Hydroxy-n-Hexyl, Hydroxy-iso-Hexyl oder Hydroxy-Cyclohexyl), Hydroxyheptyl-, Hydroxyoctyl- (z.B. 2-Ethylhexyl), Hydroxynonyl-, Hydroxydecyl- (z.B. Hydroxy-2-Propylheptyl oder Hydroxy-iso-Decyl), Hydroxyundecyl-, Hydroxydodecyl-, Hydroxytridecyl- (z.B. Hydroxy-iso-Tridecyl), und Hydroxytetradecyl-Acrylat, wobei sich die Hydroxy-Gruppe bevorzugt in endständiger Position (ω-Position) (z.B. 4-Hydroxy-n-butylacrylat) oder in (ω-1)-Position (z.B. 2-Hydroxy-n-propylacrylat) des Alkylrests befindet;
h) Alkylenglykolacrylate, die eine oder mehrere Alkylenglykol-Einheiten enthalten. Beispiele sind i) Monoalkylenglykolacrylate, wie Acrylate von Ethylenglykol, Propylenglykol (z.B. 1,2- oder 1,3-Propandiol), Butylenglykol (z.B. 1,2-, 1,3- oder 1,4-Butandiol, Pentylenglykol (z.B. 1,5-Pentandiol) oder Hexylenglykol (z.B. 1,6-Hexandiol), bei denen die zweite Hydroxy-Gruppe verethert oder verestert ist, z.B. durch Schwefelsäure, Phosphorsäure, Acrylsäure oder Methacrylsäure, oder ii) Polyalkylenglykolacrylate wie Polyethylenglykolacrylate, Polypropylenglykolacrylate, Polybutylenglykolacrylate, Polypentylenglykolacrylate oder Polyhexylenglykolacrylate, deren zweite Hydroxy-Gruppe optional verethert oder verestert sein kann, z.B. durch Schwefelsäure, Phosphorsäure, Acrylsäure oder Methacrylsäure;
   Beispiele von (Poly)Alkylenglykol-Einheiten mit veretherten Hydroxygruppen sind C₁-C₁₄-Alkyloxy-(poly)alkylenglykole (z.B. C₁-C₁₄-Alkyloxy-(poly)alkylenglykolacrylate), Beispiele von (Poly)Alkylenglykol-Einheiten mit veresterten Hydroxygruppen sind Sulfonium-(poly)alkylenglykole (z.B. Sulfonium-(poly)alkylenglykolacrylate) und deren Salze, (Poly)Alkylenglykoldiacrylate wie 1,4-Butandioldiacrylat oder 1,6-Hexandioldiacrylat oder (Poly)Alkylenglykolmethacrylatacrylate wie 1,4-Butandiolmethacrylatacrylat oder 1,6-Hexandiolmethacrylatacrylat;
   Die Polyalkylenglykolacrylate können eine Acrylatgruppe tragen (z.B. Polyethylenglykolmonoacrylat, Polypropylenglykolmonoacrylat, Polybutylenglykol-monoacrylat, Polypentylenglykolmonoacrylat oder Polyhexylenglykolmonoacrylat) oder zwei oder mehr, bevorzugt zwei, Acrylatgruppen tragen wie Polyethylenglykoldiacrylat, Polypropylenglykoldiacrylat, Polybutylenglykoldiacrylat, Polypentylenglykoldiacrylat oder Polyhexylenglykoldiacrylat;
   Die Polyalkylenglykolacrylate können auch zwei oder mehr voneinander verschiedene Polyalkylenglykol-Blöcke enthalten, z.B. Blöcke von Polymethylenglykol und Polyethylenglykol oder Blöcke von Polyethylenglykol und Polypropylenglykol;
   Der Polymerisationsgrad der Polyalkylenglykol-Einheiten oder Polyalkylenglykol-Blöcke liegt im Allgemeinen im Bereich von 1 bis 20, vorzugsweise im Bereich von 3 bis 10, besonders bevorzugt im Bereich von 3 bis 6.
i) C₁-C₁₄-Alkylmethacrylate wie Methyl-, Ethyl-, n-Propyl-, iso-Propyl-, n-Butyl-, sec. Butyl-, iso-Butyl-, tert. Butyl-, n-Pentyl-, iso-Pentyl-, Hexyl- (z.B. n-Hexyl, iso-Hexyl oder Cyclohexyl), Heptyl-, Octyl- (z.B. 2-Ethylhexyl), Nonyl-, Decyl- (z.B. 2-Propylheptyl oder iso-Decyl), Undecyl-, Dodecyl-, Tridecyl- (z.B. iso-Tridecyl), und Tetradecyl-Methacrylat; die Alkylgruppen können optional mit einem oder mehreren Halogenatomen (z.B. Fluor, Chlor, Brom oder Iod) substituiert sein, z.B. Trifluoroethyl-Methacrylat, oder mit einer oder mehreren Aminogruppen, z.B. DiethylaminoethylMethacrylat, oder mit einer oder mehreren Alkoxygruppen wie Methoxypropyl-Methacrylat, oder mit einer oder mehreren Aryloxygruppen wie Phenoxyethyl-Methacrylat.
j) C₂-C₁₄-Alkenylmethacrylate wie Ethenyl-, n-Propenyl-, iso-Propenyl-, n-Butenyl-, sec. Butenyl-, iso-Butenyl-, tert. Butenyl-, n-Pentenyl-, iso-Pentenyl-, Hexenyl- (z.B. n-Hexenyl, iso-Hexenyl oder Cyclohexenyl), Heptenyl-, Octenyl- (z.B. 2-Ethylhexenyl), Nonenyl-, Decenyl- (z.B. 2-Propenylheptyl oder iso-Decenyl), Undecenyl-, Dodecenyl-, Tridecenyl- (z.B. iso-Tridecenyl), und Tetradecenyl-Methacrylat, und deren Epoxide wie Glycidyl-Methacrylat oder Aziridine wie Aziridin-Methacrylat.
k) C₁-C₁₄-Hydroxyalkylmethacrylate wie Hydroxymethyl-, Hydroxyethyl-, Hydroxy-n-Propyl-, Hydroxy-iso-Propyl-, Hydroxy-n-Butyl-, Hydroxy-sec.-Butyl-, Hydroxy-iso-Butyl-, Hydroxy-tert.-Butyl-, Hydroxy-n-Pentyl-, Hydroxy-iso-Pentyl-, Hydroxyhexyl- (z.B. Hydroxy-n-Hexyl, Hydroxy-iso-Hexyl oder Hydroxy-Cyclohexyl), Hydroxyheptyl-, Hydroxyoctyl- (z.B. 2-Ethylhexyl), Hydroxynonyl-, Hydroxydecyl- (z.B. Hydroxy-2-Propylheptyl oder Hydroxy-iso-Decyl), Hydroxyundecyl-, Hydroxydodecyl-, Hydroxytridecyl- (z.B. Hydroxy-iso-Tridecyl), und Hydroxytetradecyl-Methacrylat, wobei sich die Hydroxy-Gruppe bevorzugt in endständiger Position (ω-Position) (z.B. 4-Hydroxy-n-butylmethacrylat) oder in (ω-1)-Position (z.B. 2-Hydroxy-n-propylmethacrylat) des Alkylrests befindet;
l) Alkylenglykolmethacrylate, die eine oder mehrere Alkylenglykol-Einheiten enthalten. Beispiele sind i) Monoalkylenglykolmethacrylate, wie Methacrylate von Ethylenglykol, Propylenglykol (z.B. 1,2- oder 1,3-Propandiol), Butylenglykol (z.B. 1,2-, 1,3- oder 1,4-Butandiol, Pentylenglykol (z.B. 1,5-Pentandiol) oder Hexylenglykol (z.B. 1,6-Hexandiol), bei denen die zweite Hydroxy-Gruppe verethert oder verestert ist, z.B. durch Schwefelsäure, Phosphorsäure, Acrylsäure oder Methacrylsäure, oder ii) Polyalkylenglykolmethacrylate wie Polyethylenglykolmethacrylate, Polypropylenglykol-methacrylate, Polybutylenglykolmethacrylate, Polypentylenglykolmethacrylate oder Polyhexylenglykolmethacrylate, deren zweite Hydroxy-Gruppe optional verethert oder verestert sein kann, z.B. durch Schwefelsäure, Phosphorsäure, Acrylsäure oder Methacrylsäure;
   Beispiele von (Poly)Alkylenglykol-Einheiten mit veretherten Hydroxygruppen sind C₁-C₁₄-Alkyloxy-(poly)alkylenglykole (z.B. C₁-C₁₄-Alkyloxy-(poly)alkylenglykolmeth-acrylate), Beispiele von (Poly)Alkylenglykol-Einheiten mit veresterten Hydroxygruppen sind Sulfonium-(poly)alkylen-glykole (z.B. Sulfonium-(poly)alkylenglykolmethacrylate) und deren Salze oder (Poly)Alkylen-glykoldimethacrylate wie 1,4-Butandioldimethacrylat;
   Die Polyalkylenglykolmethacrylate können eine Methacrylatgruppe tragen (z.B. Polyethylenglykolmonomethacrylat, Polypropylen-glykolmonomethacrylat, Polybutylenglykol-monomethacrylat, Polypentylenglykolmono-methacrylat oder Polyhexylenglykolmonomethacrylat) zwei oder mehr, bevorzugt zwei, Methacrylatgruppen tragen, wie Polyethylenglykoldimethacrylat, Polypropylenglykoldi-methacrylat, Polybutylenglykoldimethacrylat, Polypentylenglykoldi-methacrylat oder Polyhexylenglykoldimethacrylat;
   Die Polyalkylenglykolmethacrylate können auch zwei oder mehr voneinander verschiedene Polyalkylenglykol-Blöcke enthalten, z.B. Blöcke von Polymethylenglykol und Polyethylenglykol oder Blöcke von von Polyethylenglykol und Polypropylenglykol (z.B. Bisomer PEM63PHD (Cognis), CAS 58916-75-9).

Der Polymerisationsgrad der Polyalkylenglykol-Einheiten oder Polyalkylenglykol-Blöcke liegt im Allgemeinen im Bereich von 1 bis 20, vorzugsweise im Bereich von 3 bis 10, besonders bevorzugt im Bereich von 3 bis 6.

Beispiele bevorzugter (Meth)Acrylat-Comonomere sind nachfolgend aufgeführt:
4-Hydroxybutylacrylat 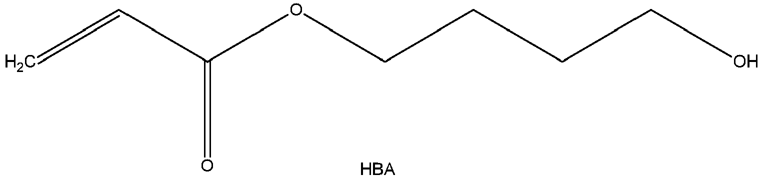
2-Hydroxypropylmethacrylat 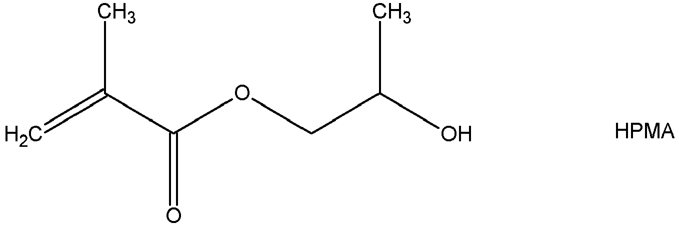
Ammoniumsulfatoethylmethacrylat 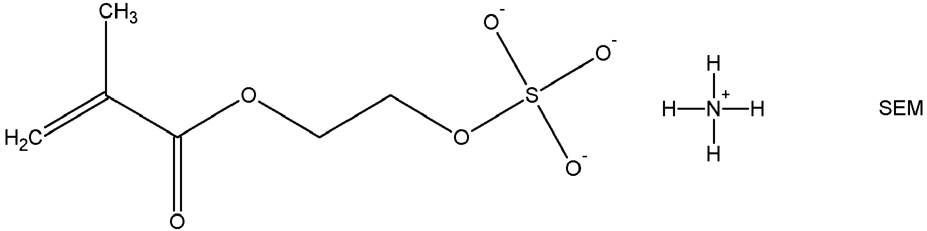
Pentapropylenglykolmethacrylat 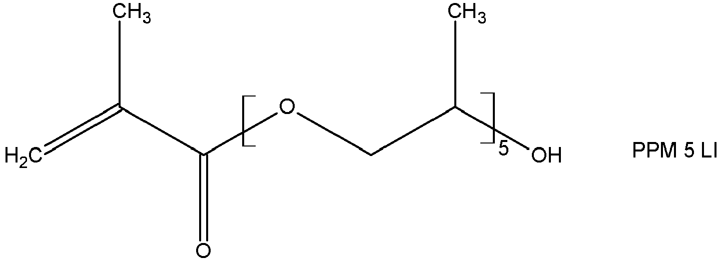
Acrylsäure 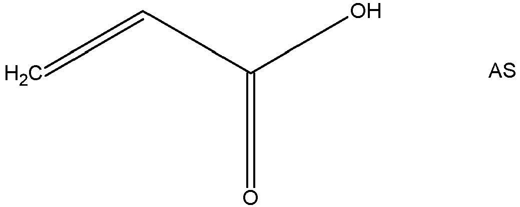
Hexaethylenglykolmethyacrylat 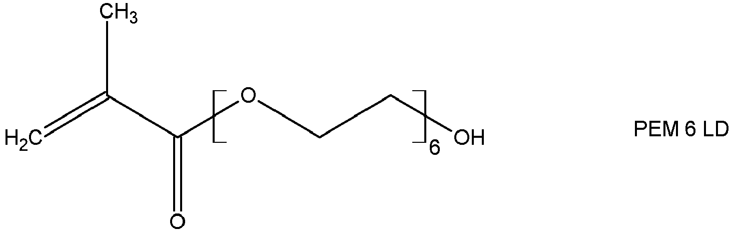
Hexapropylenglykolacrylat 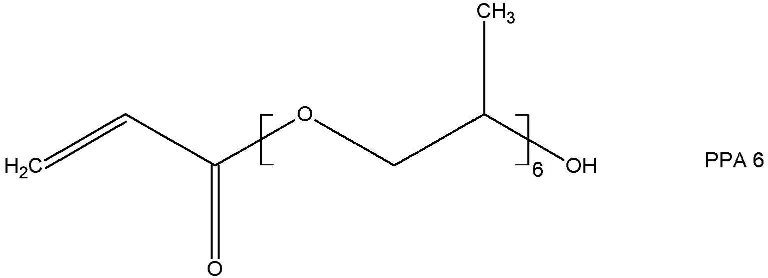
Hexaethylenglykolacrylat 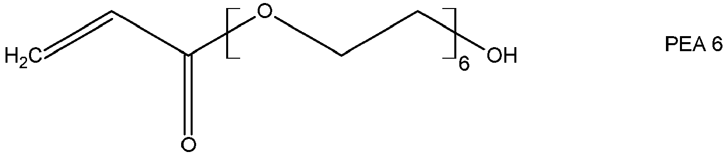
Hydroxyethylmethacrylat 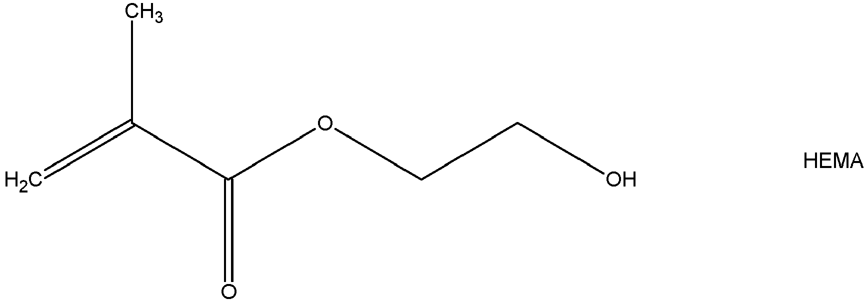
Polyalkylenglycolmethacrylat (CAS-Nr. 589-75-9) 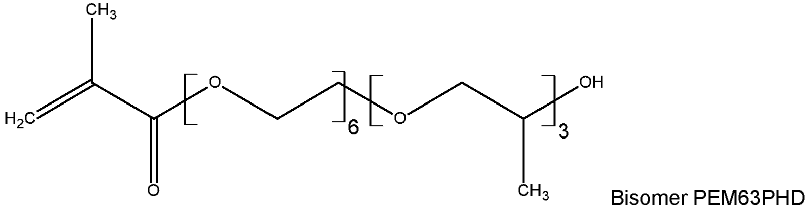
Methoxy-polyethylenglycolmethacrylat 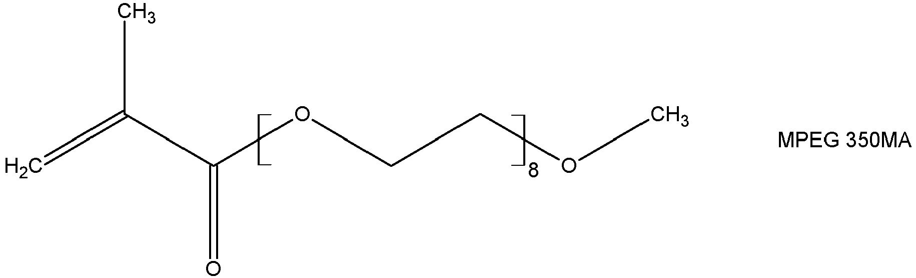
2-Propylheptylacrylat (2-PHA) 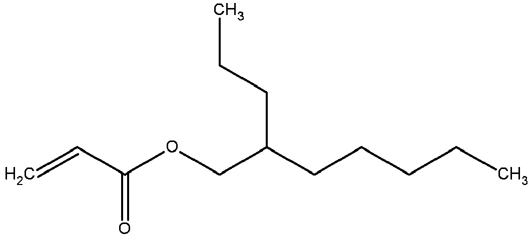
1,3-Butandioldimethacrylat (BDDMA) 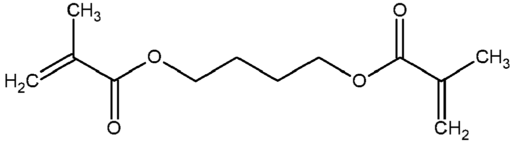
Triethylenglykoldimethacrylat (TEGDMA) 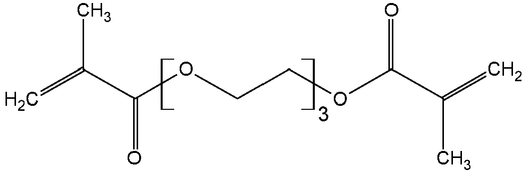
Hydroxyethylacrylat (HEA) 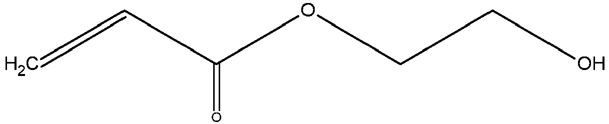
2-Hydroxypropylacrylat (HPA) 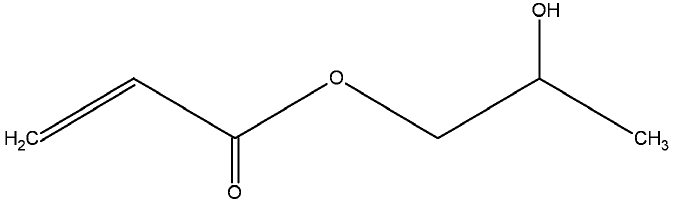
Ethylenglykoldimethacrylat (EGDMA) 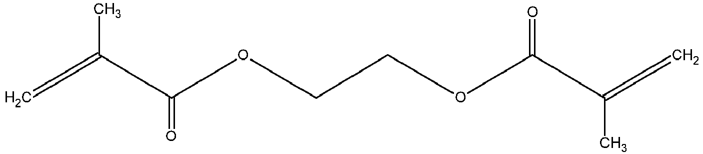
Glycidylmethacrylat (GMA) 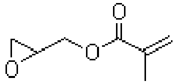
Allylmethacrylat (ALMA)

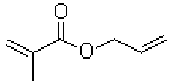

Die AMPS-Copolymere weisen im Allgemeinen einen Anteil an AMPS-Einheiten von größer 50 Mol-% auf, vorzugsweise im Bereich von 60 - 95 Mol-%, besonders bevorzugt von 80 bis 99 Mol-%, der Anteil an Comonomeren liegt im Allgemeinen kleiner 50 Mol-%, vorzugsweise im Bereich von 5 bis 40 Mol-%, besonders bevorzugt von 1 bis 20 Mol-%.

Die Copolymere können durch an sich bekannte Verfahren erhalten werden, beispielsweise im Batch- oder im Semibatch-Verfahren. Beispielsweise werden zunächst entsprechende Mengen Wasser und Monomeren in einen temperierbaren Reaktor geleitet und unter Inertgas-Atmosphäre gesetzt. Die Vorlage dann gerührt, auf Reaktionstemperatur gebracht (vorzugsweise im Bereich von ca. 70 - 80 °C) und Initiator zugesetzt, vorzugsweise in Form einer wässrigen Lösung. Als Initiator eignen sich bekannte Initiatoren für radikalische Polymerisationen, beispielsweise Natrium-, Kalium- oder Ammoniumperoxodisulfat, oder H₂O₂ basierte Mischungen, z.B. Mischungen von H₂O₂ mit Zitronensäure. Die maximale Temperatur wird abgewartet und sobald die Temperatur im Reaktor sinkt erfolgt entweder a) die Zudosierung der restlichen Monomere und anschließend eine Nachreaktion (Semibatch-Verfahren), oder b) direkt die Nachreaktion (Batch-Verfahren). Danach wird das erhaltene Reaktionsgemisch auf Raumtemperatur abgekühlt und das Copolymer aus der wässrigen Lösung isoliert, z.B. durch Extraktion mit organischen Lösungsmitteln wie Hexan oder Methylenchlorid und anschließendes Abdestillieren des Lösungsmittels. Danach kann das Copolymer mit organischem Lösungsmittel gewaschen und getrocknet werden. Das erhaltene Reaktionsgemisch kann auch direkt weiterverarbeitet werden, in diesem Fall ist es von Vorteil, der wässrigen Copolymer-Lösung ein Konservierungsmittel zuzusetzen.

Die AMPS-Copolymere eignen sich als Schutzkolloide bei der Herstellung von Mikrokapseln. Zusammenfassend sind erfindungsgemäße Zusammensetzungen, enthaltend ein Copolymer von 2-Acrylamido-2-methyl-propansulfonsäure oder deren Salzen ist mit einem oder mehreren weiteren (Meth)Acrylatmonomeren, ausgewählt aus der Gruppe der (Meth)Acrylate, der Vinylverbindungen, der ungesättigen Di- oder Polycarbonsäuren und der Salze von Amylverbindungen oder Allylverbindungen, bevorzugt.

In besonders bevorzugten erfindungsgemäßen Zusammensetzungen liegt das molare Verhältnis von dem mindestens einen aromatischen Alkohol a)i) zu der mindestens einen aldehydischen Komponente a)ii), die mindestens zwei C-Atome pro Molekül aufweist, zwischen 1 zu 2 und 1 zu 3,5, bevorzugt zwischen 1 zu 2,4 und 1 zu 2,8 und besonders bevorzugt bei 1 zu 2,6.

Bevorzugte erfindungsgemäße kosmetische Zusammensetzungen enthalten Mikrokapseln aus folgenden Komponenten a)i), a)ii), und a)iii):
- Phloroglucin, Glutardialdehyd, AMPS/Hydroxyethylmethacrylat-Copolymer;
- Phloroglucin, Succindialaldehyd, AMPS/Hydroxyethylmethacrylat-Copolymer;
- Phloroglucin, Glyoxal, AMPS/Hydroxyethylmethacrylat-Copolymer;
- Phloroglucin, Glutardialdehyd, AMPS/Hydroxyethylacrylat-Copolymer;
- Phloroglucin, Succindialaldehyd, AMPS/Hydroxyethylacrylat-Copolymer;
- Phloroglucin, Glyoxal, AMPS/Hydroxyethylacrylat-Copolymer;
- Phloroglucin, Glutardialdehyd, AMPS/Hydroxypropylmethacrylat-Copolymer;
- Phloroglucin, Succindialaldehyd, AMPS/Hydroxypropylmethacrylat-Copolymer;
- Phloroglucin, Glyoxal, AMPS/Hydroxypropylmethacrylat-Copolymer;
- Phloroglucin, Glutardialdehyd, AMPS/Hydroxypropylacrylat-Copolymer;
- Phloroglucin, Succindialaldehyd, AMPS/Hydroxypropylacrylat-Copolymer;
- Phloroglucin, Glyoxal, AMPS/Hydroxypropylacrylat-Copolymer;
- Phloroglucin, Glutardialdehyd, AMPS/Hydroxybutylmethacrylat-Copolymer;
- Phloroglucin, Succindialaldehyd, AMPS/Hydroxybutylmethacrylat-Copolymer;
- Phloroglucin, Glyoxal, AMPS/Hydroxybutylmethacrylat-Copolymer;
- Phloroglucin, Glutardialdehyd, AMPS/Hydroxybutylacrylat-Copolymer;
- Phloroglucin, Succindialaldehyd, AMPS/Hydroxybutylacrylat-Copolymer;
- Phloroglucin, Glyoxal, AMPS/Hydroxybutylacrylat-Copolymer;
- Phloroglucin, Glutardialdehyd, AMPS/Polyethylenglykolmonomethacrylat-Copolymer;
- Phloroglucin, Succindialaldehyd, AMPS/Polyethylenglykolmonomethacrylat-Copolymer;
- Phloroglucin, Glyoxal, AMPS/ Polyethylenglykolmonomethacrylat-Copolymer;
- Phloroglucin, Glutardialdehyd, AMPS/Polyethylenglykolmonoacrylat-Copolymer;
- Phloroglucin, Succindialaldehyd, AMPS/Polyethylenglykolmonoacrylat-Copolymer;
- Phloroglucin, Glyoxal, AMPS/ Polyethylenglykolmonoacrylat-Copolymer;
- Phloroglucin, Glutardialdehyd, AMPS/Polypropylenglykolmonomethacrylat-Copolymer;
- Phloroglucin, Succindialaldehyd, AMPS/Polypropylenglykolmonomethacrylat-Copolymer;
- Phloroglucin, Glyoxal, AMPS/ Polypropylenglykolmonomethacrylat-Copolymer;
- Phloroglucin, Glutardialdehyd, AMPS/Polypropylenglykolmonoacrylat-Copolymer;
- Phloroglucin, Succindialaldehyd, AMPS/Polypropylenglykolmonoacrylat-Copolymer;
- Phloroglucin, Glyoxal, AMPS/ Polypropylenglykolmonoacrylat-Copolymer;
- Phloroglucin, Glutardialdehyd, AMPS/Methoxy-polyethylenglykolmonomethacrylat-Copolymer;
- Phloroglucin, Succindialaldehyd, AMPS/Methoxy-polyethylenglykolmonomethacrylat-Copolymer;
- Phloroglucin, Glyoxal, AMPS/ Methoxy-polyethylenglykolmonomethacrylat-Copolymer;
- Phloroglucin, Glutardialdehyd, AMPS/Methoxy-polyethylenglykolmonoacrylat-Copolymer;
- Phloroglucin, Succindialaldehyd, AMPS/Methoxy-polyethylenglykolmonoacrylat-Copolymer;
- Phloroglucin, Glyoxal, AMPS/ Methoxy-polyethylenglykolmonoacrylat-Copolymer;
- Resorcin, Glutardialdehyd, AMPS/Hydroxyethylmethacrylat-Copolymer;
- Resorcin, Succindialaldehyd, AMPS/Hydroxyethylmethacrylat-Copolymer;
- Resorcin, Glyoxal, AMPS/Hydroxyethylmethacrylat-Copolymer;
- Resorcin, Glutardialdehyd, AMPS/Hydroxyethylacrylat-Copolymer;
- Resorcin, Succindialaldehyd, AMPS/Hydroxyethylacrylat-Copolymer;
- Resorcin, Glyoxal, AMPS/Hydroxyethylacrylat-Copolymer;
- Resorcin, Glutardialdehyd, AMPS/Hydroxypropylmethacrylat-Copolymer;
- Resorcin, Succindialaldehyd, AMPS/Hydroxypropylmethacrylat-Copolymer;
- Resorcin, Glyoxal, AMPS/Hydroxypropylmethacrylat-Copolymer;
- Resorcin, Glutardialdehyd, AMPS/Hydroxypropylacrylat-Copolymer;
- Resorcin, Succindialaldehyd, AMPS/Hydroxypropylacrylat-Copolymer;
- Resorcin, Glyoxal, AMPS/Hydroxypropylacrylat-Copolymer;
- Resorcin, Glutardialdehyd, AMPS/Hydroxybutylmethacrylat-Copolymer;
- Resorcin, Succindialaldehyd, AMPS/Hydroxybutylmethacrylat-Copolymer;
- Resorcin, Glyoxal, AMPS/Hydroxybutylmethacrylat-Copolymer;
- Resorcin, Glutardialdehyd, AMPS/Hydroxybutylacrylat-Copolymer;
- Resorcin, Succindialaldehyd, AMPS/Hydroxybutylacrylat-Copolymer;
- Resorcin, Glyoxal, AMPS/Hydroxybutylacrylat-Copolymer;
- Resorcin, Glutardialdehyd, AMPS/Polyethylenglykolmonomethacrylat-Copolymer;
- Resorcin, Succindialaldehyd, AMPS/Polyethylenglykolmonomethacrylat-Copolymer;
- Resorcin, Glyoxal, AMPS/ Polyethylenglykolmonomethacrylat-Copolymer;
- Resorcin, Glutardialdehyd, AMPS/Polyethylenglykolmonoacrylat-Copolymer;
- Resorcin, Succindialaldehyd, AMPS/Polyethylenglykolmonoacrylat-Copolymer;
- Resorcin, Glyoxal, AMPS/ Polyethylenglykolmonoacrylat-Copolymer;
- Resorcin, Glutardialdehyd, AMPS/Polypropylenglykolmonomethacrylat-Copolymer;
- Resorcin, Succindialaldehyd, AMPS/Polypropylenglykolmonomethacrylat-Copolymer;
- Resorcin, Glyoxal, AMPS/ Polypropylenglykolmonomethacrylat-Copolymer;
- Resorcin, Glutardialdehyd, AMPS/Polypropylenglykolmonoacrylat-Copolymer;
- Resorcin, Succindialaldehyd, AMPS/Polypropylenglykolmonoacrylat-Copolymer;
- Resorcin, Glyoxal, AMPS/ Polypropylenglykolmonoacrylat-Copolymer;
- Resorcin, Glutardialdehyd, AMPS/Methoxy-polyethylenglykolmonomethacrylat-Copolymer;
- Resorcin, Succindialaldehyd, AMPS/Methoxy-polyethylenglykolmonomethacrylat-Copolymer;
- Resorcin, Glyoxal, AMPS/ Methoxy-polyethylenglykolmonomethacrylat-Copolymer;
- Resorcin, Glutardialdehyd, AMPS/Methoxy-polyethylenglykolmonoacrylat-Copolymer;
- Resorcin, Succindialaldehyd, AMPS/Methoxy-polyethylenglykolmonoacrylat-Copolymer;
- Resorcin, Glyoxal, AMPS/ Methoxy-polyethylenglykolmonoacrylat-Copolymer;

In einer weiteren Ausführung der Erfindung können zur Herstellung der Mikrokapseln zusätzlich eines oder mehrere stickstoffhaltige oder siliciumdioxidhaltige Agentien verwendet werden. Dabei können die stickstoffhaltigen Agentien in das Harz einpolymerisiert werden (z.B. um das Eigenschaftsprofil des Harzes abzurunden) oder zur Nachbehandlung verwendet werden. Dabei kommen bevorzugt heterocyclische Verbindungen mit mindestens einem Stickstoffatom als Heteroatom, welches entweder mit einem aminosubstituierten Kohlenstoffatom oder einer Carbonylgruppe benachbart ist, wie zum Beispiel Pyridazin, Pyrimidin, Pyrazin, Pyrrolidon, Aminopyridin und davon abgeleitete Verbindungen zum Einsatz. Vorteilhafte Verbindungen dieser Gattung sind Aminopyridin und davon abgeleitete Verbindungen. Geeignet sind prinzipiell alle Aminopyridine, wie zum Beispiel Melamin, 2,6-Diaminopyridin, substituierte und dimere Aminopyridine und aus diesen Verbindungen hergestellte Mischungen. Vorteilhaft sind weiterhin Polyamide und Dicyandiamid, Harnstoff und seine Derivate sowie Pyrrolidon und davon abgeleitete Verbindungen. Beispiele für geeignete Pyrrolidone sind zum Beispiel Imidazolidinon und davon abgeleitete Verbindungen, wie zum Beispiel Hydantoin, dessen Derivate besonders vorteilhaft sind, insbesondere vorteilhaft sind von diesen Verbindungen Allantoin und seine Derivate. Besonders vorteilhaft sind weiter Triamino-1,3,5-Triazin (Melamin) und seine Derivate.

Es sei besonders betont, dass es sich bei der Nachbehandlung um eine "reine" Nachbehandlung der Oberfläche handelt, um zu dieser bevorzugten Ausführungsform der erfindungsgemäß eingesetzten Mikrokapseln zu gelangen. Mit anderen Worten: in dieser bevorzugten Ausführungsform ist das besagte stickstoffhaltige Agens nicht gleichmäßig am Aufbau der gesamten Kapselwand beteiligt, sondern überwiegend auf die äußere Oberfläche der Kapselwände konzentriert. Die Nachbehandlung kann auch mit Kieselgel (insb. amorphem hydrophobem Kieselgel) oder mit aromatischen Alkoholen a)i) erfolgen, wobei diese bevorzugt als Schlämmen eingesetzt werden. Die Herstellung der in den erfindungsgemäßen kosmetischen Mitteln enthaltenen Mikrokapseln oder Mikrokapseldispersionen erfolgt vorzugsweise, indem der mindestens eine erfindungsgemäß umzusetzende aromatische Alkohol, die mindestens eine erfindungsgemäß umzusetzende aldehydische Komponente, die mindestens zwei C-Atome pro Molekül aufweist, und mindestens einemHomo- oder Copolymer von 2-Acrylamido-2-methyl-propansulfonsäure oder dessen Salzen , gegebenenfalls in Gegenwart mindestens einer zu verkapselnde Substanz (Kernmaterial), zusammen- und zur Reaktion gebracht werden und durch spätere Temperaturerhöhung die Aushärtung der Kapseln erfolgt. Dabei ist es besonders bevorzugt, dass der pH-Wert im Laufe des Verfahrens erhöht wird.

Vorzugsweise wird im Rahmen eines solchen Verfahrens zunächst
a) der mindestens eine aromatische Alkohol und/oder Ester oder Ether und die mindestens eine aldehydische Komponente und mindestens eine zu verkapselnde Substanz, in Gegenwart mindestens eines Homo- oder Copolymers von 2-Acrylamido-2-methyl-propansulfonsäure oder dessen Salzen, bei einer Temperatur von 40 bis 65°C und einem pH-Wert zwischen 6 und 9, vorzugsweise 7 und 8,5 zusammengebracht und
b) in einem späteren Verfahrensschritt bei einer Temperatur von 40 bis 65 °C der pH-Wert auf über 9, vorzugsweise zwischen 9,5 und 11 angehoben, wobei
c) später die Aushärtung der Kapseln durch Temperaturerhöhung auf 60°C bis 110°C, vorzugsweise 70°C bis 90°C, insbesondere 80 °C durchgeführt wird.

Wird jedoch Phloroglucin als Alkoholkomponente verwendet, wird vorteilhafter im Sauren gehärtet; bevorzugt liegt der pH Wert dann bei höchstens 4, insbesondere bevorzugt zwischen 3 und 4, zum Beispiel zwischen 3,2 - 3,5.

Durch die gewählten Parameter der Temperatur, des pH-Wertes und/oder der Rührgeschwindigkeit können die Ausbeute und Qualität der erfindungsgemäß eingesetzten Mikrokapseln oder Mikrokapseldispersionen beeinflusst werden. Insbesondere kann eine zu niedrige Temperatur dazu führen, dass die Kapselwand weniger dicht ausgebildet wird.

Die Alkalität kann ebenfalls von Bedeutung für die Qualität der erfindungsgemäß eingesetzten Mikrokapseln sein. Daneben beeinflusst der pH-Wert im Rahmen der Prozessführung die Tendenz zum Gelieren des Ansatzes. Sofern die Teilchenbildung (Schritt b) oben) bei einem pH-Wert von 9 oder niedriger durchgeführt wird, könnte der Ansatz gelieren.

In einer Ausführungsform des Verfahrens wird zum Einstellen der Alkalität ein Alkalisalz, vorzugsweise Alkalicarbonat, insbesondere Natriumcarbonat verwendet. Natriumcarbonat ist bevorzugt, da damit die Gefahr des Gelierens verringert wird. Im Rahmen des Verfahrens kann zu Beginn der Umsetzung (Verfahrensschritt a)) des aromatischen Alkohols mit der aldehydischen Komponente gerührt werden, wobei die Rührgeschwindigkeit bei 500 bis 2500 U/min, insbesondere bei 1000 bis 2000 U/min liegen kann. Zu dem erhaltenen Vorkondensat kann anschließend das

Homo- oder Copolymer von 2-Acrylamido-2-methyl-propansulfonsäure oder dessen Salz und die zu verkapselnde Substanz zugeben werden. Vorzugsweise wird später und zwar unmittelbar vor oder während der Anhebung der Alkalität (Verfahrensschritt b) die Rührgeschwindigkeit erhöht, wobei sie dann bei 3000 bis 5000 U/min, insbesondere bei 3500 bis 4500 U/min, vor allem bei 4000 U/min liegen kann. Vorzugsweise wird die so erhöhte Rührgeschwindigkeit so lange beibehalten, bis die Viskositätswerte der Mischung sinken, wobei nach Einsetzen einer Viskositätsabnahme die Rührgeschwindigkeit gesenkt wird, vorzugsweise auf 500 bis 2500 U/min, besonders bevorzugt auf 1000 bis 2000 U/min. Ein früheres Absenken der Rührgeschwindigkeit kann ebenfalls zum unerwünschten Gelieren des Ansatzes führen.

Vorzugsweise wird nach Beginn der beschriebenen Viskositätsabnahme mindestens 20 Minuten, besonders bevorzugt zwischen 30 und 180 Minuten bei einer Rührgeschwindigkeit von 1000 bis 2000 U/min und einer Temperatur von 40 bis 65°C weiter gerührt, bevor in Verfahrensschritt c) die Aushärtung der Kapseln durch Temperaturerhöhung erfolgt. Diese Phase nach Beginn der beschriebenen Viskositätsabnahme und vor Aushärtung der Kapseln wird in der vorliegenden Erfindung auch als Ruhephase bezeichnet. Die Ruhephase kann vorzugsweise dazu dienen, um die Präformierung ausreichend stabiler Kapselwände zu erreichen, mit anderen Worten, die Kapselwände so stabil auszubilden, dass kein Kernmaterial mehr entweicht.

Es ist auch die Herstellung von Vollkugeln möglich, also Kapseln, die kein Kernmaterial umschließen. Diese Vollkugeln können sogar einen mittleren Durchmesser von unter 500 nm (vorzugsweise zwischen 300 und 400 nm) aufweisen. Dabei kann es sich vorzugsweise um monodisperse Vollkugeln handeln. Zur Herstellung dieser Vollkugeln kann in einer Ausführungsform Phloroglucin verwendet werden.

Die Mikrokapseln weisen im Allgemeinen Durchmesser im Bereich von 1-1000 µm auf. Im Rahmen der vorliegenden Erfindung sind vom Begriff Mikrokapsel auch Nanokapseln umfasst, d.h. Kapseln mit einem Durchmesser < 1 µm. Die Kapseln weisen vorzugsweise einen mittleren Durchmesser von 0,1 bis 100 µm auf. Die Wandstärke kann zum Beispiel 0,05 bis 10 µm betragen.

Die erfindungsgemäßen kosmetischen Zusammensetzungen enthalten die Mikrokapseln vorzugsweise in Mengen von 0,01 und 50 Gew.-%, jeweils bezogen auf das Gewicht des kosmetischen Mittels. Bevorzugte erfindungsgemäße Zusammensetzungen sind dadurch gekennzeichnet, dass sie - bezogen auf ihr Gewicht - 0,1 bis 25 Gew.-%, vorzugsweise 0,25 bis 20 Gew.-%, weiter bevorzugt 0,5 bis 15 Gew.-% und insbesondere 1 bis 10 Gew.-% Mikrokapseln a) enthalten. Weitere, besonders bevorzugte erfindungsgemäße Zusammensetzungen enthalten - bezogen auf ihr Gewicht - 3 bis 5 Gew.-% und insbesondere 1 bis 2 Gew.-% erfindungsgemäße Mikrokapseln a). Bei der Herstellung der erfindungsgemäßen kosmetischen Mittel, egal ob fest oder flüssig, ist es vorteilhaft, die einzubringenden Mikrokapseln in Form eines Mikrokapsel-Slurries (wässrige Dispersion der Mikrokapseln) einzubringen. Dabei hat es sich als sehr vorteilhaft erwiesen, den Mikrokapsel-Slurry zur Stabilisierung mit Tensid zu versetzen, wobei als Tensid kationisches, anionisches und/oder nichtionisches Tensid eingesetzt wird, vorzugsweise nichtionisches Tensid, insbesondere ethoxylierter Oxoalkohol geeignet ist. Derart stabilisierte Mikrokapsel-Slurries sind besser verarbeitbar. Ansonsten kann die Verarbeitbarkeit der Mikrokapsel-Slurries durch eine reversible Flockulation erschwert werden.

Anionische Tenside können dabei vorteilhafterweise in Mengen von 1 bis 40 Gew.-%, beispielsweise 2 bis 30 Gew.-%, insbesondere 3 bis 20 Gew.-% zur Stabilisierung der Dispersionen eingesetzt werden, Gew.-% bezogen auf dies gesamte Dispersion. Kationische Tenside können vorteilhafterweise in Mengen von 0,001 bis 4 Gew.-%, beispielsweise 0,01 bis 3 Gew.-% und insbesondere 0,1 bis 2 Gew.-% zur Stabilisierung der Dispersionen eingesetzt werden, Gew.-% bezogen auf dies gesamte Dispersion. Nichtionische Tenside können vorteilhafterweise in Mengen von 0,01 bis 20 Gew.-%, beispielsweise 0,1 bis15 Gew.-%, insbesondere 1 bis 10 Gew.-% zur Stabilisierung der Dispersionen eingesetzt werden, Gew.-% bezogen auf dies gesamte Dispersion. Geeignete anionische Tenside sind z.B. Alkylbenzolsulfonate, vorzugsweise sekundäres C10-C13-n-Alkylbenzolsulfonat, Alkansulfonate, Methylestersulfonate, α-Olefinsulfonate, Alkylsulfate, vorzugsweise Fettalkoholsulfat, Alkylethersulfate, vorzugsweise Fettalkoholethersulfat und Sulfosuccinate. Geeignete kationische Tenside sind z.B. quartäre Ammonium-Verbindungen, insbesondere quartäre Ammonium-Verbindungen mit einem oder zwei hydrophoben Alkyl-Resten, quartäre Phosphonium-Salze oder tertiäre Sulfonium-Salze. Besonders bevorzugt sind so genannte Esterquats. Esterquat ist die Sammelbezeichnung für kationische grenzflächenaktive Verbindungen mit vorzugsweise zwei hydrophoben Gruppen, die über Ester-Bindungen mit einem quaternierten Di(Tri-)ethanolamin oder einer analogen Verbindung verknüpft sind.

Der Einsatz nichtionischer Tenside zur Stabilisierung wässriger Mikrokapseldispersionen hat sich als besonders vorteilhaft erwiesen. Mit Vorteil einsetzbar sind insbesondere Fettalkoholethoxylate, Oxoalkoholethoxylate, Alkylphenolpolyglycolether, Fettsäureethoxylate, Fettaminethoxylate, ethoxylierte Triacylglycerole und Mischether (beidseitig alkylierte Polyethylenglycolether) sowie Alkylpolyglucoside, Saccharoseester, Sorbitanester, Fettsäureglucamide sowie Aminoxide. Besonders aber der Einsatz von Oxoalkoholethoxylaten ist vorteilhaft hinsichtlich der gewünschten Stabilisierung der wässrigen Mikrokapseldispersionen. Diese ermöglichen die besten Ergebnisse im Sinne der Erfindung. Bevorzugte Oxoalkoholethoxylate leiten sich von Oxoalkoholen mit 9 bis 15 Kohlenstoffatomen ab, an die vorzugsweise 3 bis 15 Mol Ethylenoxid angelagert sind. Ein besonders bevorzugtes Oxoalkoholethoxylat im Sinne der Erfindung ist C₁₃-C₁₅-Oxoalkohol, an welchen 7 Mol Ethylenoxid angelagert sind. Ein geeignetes Handelsprodukt ist z.B. Lutensol® AO 7 von der BASF. Durch den Einsatz von Oxoalkoholethoxylaten kann eine auftretende reversible Flockulation völlig zurückgedrängt werden. Auch Alkylpolyglucoside sind zur Verringerung der Flockulation bevorzugt.

Besonders vorteilhaft sind die vorstehend beschriebenen stabilisierten Mikrokapseldispersionen bei der Herstellung von flüssigen kosmetischen Mitteln. Ein erfindungsgemäßes Verfahren, bei welchem man ein flüssiges kosmetisches Mittel mit einer Mikrokapseldispersion, wie zuvor beschrieben, vermengt, vorzugsweise durch Einrühren der Mikrokapseldispersion in die Produktmatrix oder durch kontinuierliche Zugabe in ein flüssiges Produkt und Vermischen über statische Mischelemente, entspricht daher einer bevorzugten Ausführungsform der Erfindung.

Zusätzlich zu den Mikrokapseln enthalten die erfindungsgemäßen Zusammensetzungen bezogen auf ihr Gewicht, 0,01 bis 70 Gew.-% mindestens eines kosmetischen Wirkstoffes aus der Gruppe
b1) der Oxidationsfarbstoffvorprodukte und/oder
b2) der direktziehenden Farbstoffe und/oder
b3) der Oxidationsmittel, ausgewählt aus Wasserstoffperoxid und dessen Anlagerungsverbindungen an feste Träger, und/oder
b4) der Tenside und/oder Emulgatoren und/oder
b5) der haarkonditionierenden Wirkstoffe und/oder
b6) der desodorierenden und/oder der schweißhemmenden Wirkstoffe und/oder
b7) der hautaufhellenden und/oder hautberuhigenden und/oder feuchtigkeitsspendenden Wirkstoffe und/oder
b8) der anorganischen und/oder organischen UV-Filtersubstanzen und/oder
b9) der sebumregulierenden Wirkstoffe und/oder der mechanischen Exfoliationsmittel und/oder der antimikrobiellen Wirkstoffe und/oder
b10) der haarfestigenden oder Haarstyling-Wirkstoffe und/oder
b11) der Antikaries-Wirkstoffe und/oder
b12) der Wirkstoffe gegen Zahnsteinbildung und/oder
b13) der Mischungen dieser Wirkstoffe b1) - b12).

Erfindungsgemäße Mittel, die Oxidationsfarbstoffvorprodukte und gegebenenfalls direktziehende Farbstoffe enthalten, können als Mittel zum Färben und/oder als Mittel zum Aufhellen keratinischer Fasern konfektioniert werden. Sind nur direktziehende Farbstoffe vorhanden, ist beispielsweise auch die Formulierung eines Färbeshampoos möglich. Unter keratinischen bzw. keratinhaltigen Fasern werden im Rahmen dieser Anmeldung Pelze, Wolle, Federn und insbesondere menschliche Haare verstanden.

Bevorzugte erfindungsgemäße Mittel sind Färbemittel, d.h. Mittel zur Veränderung der Farbe keratinischer Fasern. Unter diesen sind insbesondere die sogenannten Oxidationsfärbemittel bevorzugt. Die erfindungsgemäßen Oxidationsfärbemittel enthalten mindestens eine Kuppler- und mindestens eine Entwicklerkomponente. Kuppler- und Entwicklerkomponenten werden auch als Oxidationsfarbstoffvorprodukte bezeichnet. Zudem können die erfindungsgemäßen Oxidationsfärbemittel auch noch direktziehende Farbstoffe als Nuanceure enthalten.

Erfindungsgemäß bevorzugte Mittel zum Färben und/oder Aufhellen keratinischer Fasern sind solche, wie sie in der dieser Anmeldung zu Grunde liegenden deutschen Prioritätsanmeldung DE 102010040567.1 auf den ursprünglich eingereichten Seiten 25, Zeile 25 bis Seite 63, Zeile 2, beschrieben sind. Dieser und die folgenden Verweise auf die Prioritätsanmeldung dienen lediglich der Illustration der Gegenstände der Anmeldung/des Patents, ergänzen diese aber nicht.

Die erfindungsgemäßen Mittel können - wie weiter oben erwähnt - auch als Duschgele, Duschbäder, Zahnreinigungsmittel, Mundwässer, Haarshampoos, Haarkonditionierer, konditionierende Shampoos, Haarsprays, Haarspülungen, Haarkuren, Haarpackungen, Haar-Tonics, Haarfestiger, Haarlegemittel, Haarstyling-Zubereitungen, Fönwell-Lotionen, Schaumfestiger, Haargele, Haarwachse, Deodorantien, Antitranspirantien, Cremes, Lotionen, Sonnenschutzmittel oder deren Kombinationen formuliert werden. Ein Einsatz von Oxidationsfarbstoffvorprodukten und/oder direktziehenden Farbstoffen ist in solchen kosmetischen Mitteln nicht zwingend notwendig.

Die genannten Produkte enthalten hingegen mindestens einen Inhaltsstoff aus den Gruppen
b4) der Tenside und/oder Emulgatoren und/oder
b5) der haarkonditionierenden Wirkstoffe und/oder
b6) der desodorierenden und/oder der schweißhemmenden Wirkstoffe und/oder
b7) der hautaufhellenden und/oder hautberuhigenden und/oder feuchtigkeitsspendenden Wirkstoffe und/oder
b8) der anorganischen und/oder organischen UV-Filtersubstanzen und/oder
b9) der sebumregulierenden Wirkstoffe und/oder der mechanischen Exfoliationsmittel und/oder der antimikrobiellen Wirkstoffe und/oder
b10) der haarfestigenden oder Haarstyling-Wirkstoffe und/oder
b11) der Antikaries-Wirkstoffe und/oder
b12) der Wirkstoffe gegen Zahnsteinbildung und/oder
b13) der Mischungen dieser Wirkstoffe b4) - b12).

Die Wirkstoffe bzw. Wirkstoffgruppen werden nachstehend beschrieben.

In vielen Fällen enthalten die erfindungsgemäßen Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside und Emulgatoren eignen sich für die erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
   - Acylglutamate der Formel (I), 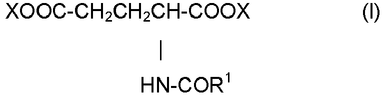 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
   - Ester einer hydroxysubstituierten Di- oder Tricarbonsäure der allgemeinen Formel (II), 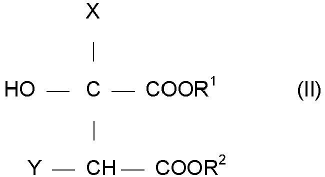 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, dass wenigstens eine der Gruppen R, R¹ oder R² ein Rest Z ist,
- Ester der Sulfobernsteinsäure oder der Sulfosuccinate der allgemeinen Formel (III), 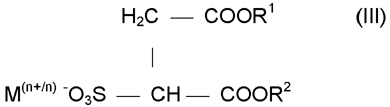 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkyl-polyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Tenside und Emulgatoren sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.

Als zwitterionische Tenside und Emulgatoren werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside und Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammonium-glycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamidderivat.

Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside und Emulgatoren enthalten als hydrophile Gruppe z. B. eine Polyolgruppe, eine Polyalkylenglycolethergruppe oder eine Kombination aus Polyol- und Polyglycolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diester von Anlagerungsprodukten von 1 bis 30 Mol Ethylenoxid an Polyole mit 3 bis 6 Kohlenstoffatomen, insbesondere an Glycerin,
- Anlagerungsprodukte von 5 bis 60 Mol Ethylenoxid an Rizinusöl und gehärtetes Rizinusöl,
- Polyolfettsäure(partial)ester, wie Hydagen® HSP (Cognis) oder Sovermol® - Typen (Cognis), insbesondere von gesättigten C₈₋₃₀-Fettsäuren,
- alkoxylierte Triglyceride,
- alkoxylierte Fettsäurealkylester,
- Aminoxide,
- Fettsäurealkanolamide, Fettsäure-N-alkylglucamide und Fettamine sowie deren Ethylenoxid- oder Polyglycerin-Anlagerungsprodukte,
- Sorbitanfettsäureester und Anlagerungsprodukte von Ethylenoxid an Sorbitanfettsäureester wie beispielsweise die Polysorbate,
- Zuckerfettsäureester und Methylglucosid-Fettsäureester sowie deren Ethylenoxid- oder Polyglycerin-Anlagerungsprodukte,
- Alkylpolyglycoside entsprechend der allgemeinen Formel RO-(Z)ₓ wobei R für Alkyl, Z für Zucker sowie x für die Anzahl der Zuckereinheiten steht. Besonders bevorzugt sind solche Alkylpolyglycoside, bei denen R
   - im wesentlichen aus C₈- und C₁₀-Alkylgruppen oder
   - im wesentlichen aus C₁₂- und C₁₄-Alkylgruppen oder
   - im wesentlichen aus C₈- bis C₁₆-Alkylgruppen oder
   - im wesentlichen aus C₁₂- bis C₁₆-Alkylgruppen oder
   - im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Die erfindungsgemäß verwendbaren Alkylpolyglycoside enthalten im Schnitt 1,1 bis 5 Zuckereinheiten. Alkylpolyglycoside mit x-Werten von 1,1 bis 2,0 sind bevorzugt. Ganz besonders bevorzugt sind Alkylglycoside, bei denen x 1,1 bis 1,8 beträgt.
- Gemische aus Alkyl-(oligo)-glucosiden und Fettalkoholen, z. B. Montanov®68,
- Sterine, z. B. Ergosterin, Stigmasterin, Sitosterin und Mykosterine,
- Phospholipide, z. B. Lecithine bzw. Phosphatidylcholine,
- Polyglycerine und Polyglycerinderivate wie beispielsweise Polyglycerinpoly-12-hydroxystearat (Dehymuls® PGPH) oder Triglycerindiisostearat (Lameform® TGI),
- alkoxylierte Polydialkylsiloxane (INCI-Bezeichnung: Dimethicone Copolyol).

Als bevorzugte nichtionische oberflächenaktive Substanzen haben sich die Alkylpolyglycoside, gegebenenfalls im Gemisch mit Fettalkoholen, alkoxylierte Polydialkylsiloxane, Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen.

Bevorzugte erfindungsgemäße Zusammensetzungen enthalten - jeweils bezogen auf ihr Gesamtgewicht - neben den Mikrokapseln a) mindestens ein nichtionisches Tensid in einer Gesamtmenge von 0,05 - 10 Gew.-%, bevorzugt 0,1 - 5 Gew.-%, besonders bevorzugt 0,5 - 4 Gew.-%, außerordentlich bevorzugt 1 - 3 Gew.-%.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen. Bevorzugte erfindungsgemäße Zusammensetzungen enthalten neben den Mikrokapseln a) mindestens ein kationisches Tensid in einer Gesamtmenge von 0,05 - 10 Gew.-%, bevorzugt 0,1 - 5 Gew.-%, besonders bevorzugt 0,5 - 3 Gew.-%.

Bevorzugte erfindungsgemäße Zusammensetzungen enthalten neben den Mikrokapseln a) mindestens ein Tensid in einer Gesamtmenge von - jeweils bezogen auf ihr Gewicht - 0,05 - 30 Gew.-%, bevorzugt 0,1 - 20 Gew.-%, besonders bevorzugt 0,5 - 15 Gew.-%, außerordentlich bevorzugt 1 - 10 Gew.-%, weiter bevorzugt 2 - 5 Gew.-%.

In einer weiteren bevorzugten Ausführungsform können die erfindungsgemäßen Mittel Emulgatoren enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 - 5, insbesondere 1,2 - 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in einer Gesamtmenge von 0,1 - 25 Gew.-%, insbesondere 0,5 - 15 Gew.-%, jeweils bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Zusammenfassend sind erfindungsgemäße Zusammensetzungen bevorzugt, die als Haarreinigungsmittel konfektioniert ist und - bezogen auf ihr Gewicht - 0,05 bis 30 Gew.-%, bevorzugt mindestens eines anionischen und/oder amphoteren Tensids enthalten. Bevorzugte erfindungsgemäße Zusammensetzungen, die als Haarreinigungsmittel konfektioniert sind, enthalten bevorzugt - jeweils bezogen auf ihr Gewicht - 2 bis 20, bevorzugt 5 bis 15 und insbesondere 8 bis 14 Gew.-% Tenside, die bevorzugt aus der Gruppe der anionischen, amphoteren, zwitterionischen und nichtionischen Tenside sowie aus Gemischen dieser Tensidklassen ausgewählt sind, sowie 0,1 bis 10 Gew.-%, vorzugsweise 0,25 bis 5 Gew.-%, weiter bevorzugt 0,5 bis 3 Gew.-% und insbesondere 1 bis 2 Gew.-% erfindungsgemäße Mikrokapseln a).

Erfindungsgemäße Zusammensetzungen, die als Körperreinigungsmittel, insbesondere als Duschgel, Duschbad oder Flüssigseife, konfektioniert sind, enthalten bevorzugt - bezogen auf ihr Gewicht - 5 bis 30, vorzugsweise 7 bis 20 und insbesondere 8 bis 14 Gew.-% Tenside, die bevorzugt aus der Gruppe der anionischen, amphoteren, zwitterionischen und nichtionischen Tenside sowie aus Gemischen dieser Tensidklassen ausgewählt sind.

Bevorzugte erfindungsgemäße Zusammensetzungen, die als Körperreinigungsmittel, insbesondere als Duschgel, Duschbad oder Flüssigseife, konfektioniert sind, enthalten bevorzugt - jeweils bezogen auf ihr Gewicht - 5 bis 30, vorzugsweise 7 bis 20 und insbesondere 8 bis 14 Gew.-% Tenside, die bevorzugt aus der Gruppe der anionischen, amphoteren, zwitterionischen und nichtionischen Tenside sowie aus Gemischen dieser Tensidklassen ausgewählt sind, sowie 0,1 bis 10 Gew.-%, vorzugsweise 0,25 bis 5 Gew.-%, weiter bevorzugt 0,5 bis 3 Gew.-% und insbesondere 1 bis 2 Gew.-% erfindungsgemäße Mikrokapseln a).

Die erfindungsgemäßen Mittel können auch als Deodorantien und/oder Antitranspirantien formuliert werden. In diesem Falle enthalten sie mindestens einen desodorierenden und/oder schweißhemmenden Wirkstoff. Erfindungsgemäß einsetzbare Antitranspirant-Wirkstoffe sind die wasserlöslichen adstringierenden anorganischen und organischen Salze des Aluminiums, Zirkoniums und Zinks bzw. beliebige Mischungen dieser Salze. Geeignete Antitranspirant-Wirkstoffe sind ausgewählt aus den Aluminiumchlorhydraten, zum Beispiel Aluminiumsesquichlorhydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat. Erfindungsgemäß wird unter Wasserlöslichkeit der Antitranspirant-Wirkstoffe eine Löslichkeit von wenigstens 5 Gew.-% bei 20 °C verstanden, das heißt, dass Mengen von wenigstens 5 g des Antitranspirant-Wirkstoffes in 95 g Wasser bei 20 °C löslich sind.

Es ist in den vorstehend genannten Zusammensetzungen außerordentlich bevorzugt, dass die Mikrokapseln a) bestimmte verkapselte Inhaltsstoffe beinhalten.

Erfindungsgemäß bevorzugte Mikrokapseln a) enthalten als verkapselten Inhaltsstoff mindestens einen deodorierend wirkenden aromatischen Alkohol der Struktur (AA-1), 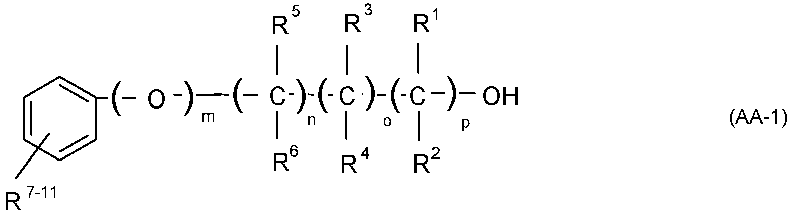
wobei R¹ - R⁶ = unabhängig voneinander ein Wasserstoffatom, eine Alkylgruppe mit 1 bis 10 Kohlenstoffatomen, die linear oder verzweigt sein kann und die substituiert sein kann mit OH-Gruppen oder Alkoxy-Gruppen mit 1 bis 5 Kohlenstoffatomen, oder eine Alkenylgruppe mit 2 bis 10 Kohlenstoffatomen, die linear oder verzweigt sein kann und die substituiert sein kann mit OH-Gruppen oder Alkoxy-Gruppen mit 1 bis 5 Kohlenstoffatomen,
R⁷ - R¹¹ = unabhängig voneinander ein Wasserstoffatom, ein Halogenatom, insbesondere ein Chloratom, oder eine Alkylgruppe mit 1 bis 10 Kohlenstoffatomen, die linear oder verzweigt sein kann und die substituiert sein kann mit OH-Gruppen oder Alkoxy-Gruppen mit 1 bis 5 Kohlenstoffatomen, insbesondere mit einer Methoxygruppe, m = 0 oder 1 ist, n, o, p = unabhängig voneinander ganze Zahlen von 0 bis 10 sein können, wobei mindestens einer der Werte n, o, p ≠ 0 ist. Erfindungsgemäß besonders bevorzugte Mikrokapseln a) sind dadurch gekennzeichnet, dass der deodorierend wirkende aromatische Alkohol der Struktur (AA-1) ausgewählt ist aus Phenoxyethanol, Phenoxyisopropanol (3-Phenoxy-propan-2-ol), Anisalkohol, 2-Methyl-5-phenyl-pentan-1-ol, 1,1-Dimethyl-3-phenyl-propan-1-ol, Benzylalkohol, 2-Phenylethan-1-ol, 3-Phenylpropan-1-ol, 4-Phenylbutan-1-ol, 5-Phenylpentan-1-ol, 2-Benzylheptan-1-ol, 2,2-Dimethyl-3-phenylpropan-1-ol, 2,2-Dimethyl-3-(3'-methylphenyl)-propan-1 -ol, 2-Ethyl-3-phenylpropan-1-ol, 2-Ethyl-3-(3'-methylphenyl)-propan-1-ol, 3-(3'-Chlorphenyl)-2-ethylpropan-1-ol, 3-(2'-Chlorphenyl)-2-ethylpropan-1-ol, 3-(4'-Chlorphenyl)-2-ethylpropan-1-ol, 3-(3',4'-Dichlorphenyl)-2-ethylpropan-1-ol, 2-Ethyl-3-(2'-methylphenyl)-propan-1-ol, 2-Ethyl-3-(4'-methylphenyl)-propan-1-ol, 3-(3',4'-Dimethylphenyl)-2-ethylpropan-1-ol, 2-Ethyl-3-(4'-methoxyphenyl)-propan-1-ol, 3-(3',4'-Dimethoxyphenyl)-2-ethyl-propan-1-ol, 2-Allyl-3-phenylpropan-1-ol und 2-n-Pentyl-3-phenylpropan-1-ol.

| | m | n | o | p | R¹ | R² | R³ | R⁴ | R⁵ | R⁶ | R⁷⁻¹¹ | |
|---|---|---|---|---|---|---|---|---|---|---|---|---|
| Phenoxyethanol | 1 | 0 | 0 | 2 | H | H | - | - | - | - | H | |
| 3-Phenoxy-propan-2-ol | 1 | 0 | 1 | 1 | H | CH₃ | H | H | - | - | H | |
| 2-Benzylheptan-1-ol | 0 | 1 | 1 | 1 | H | H | H | n-C₅H₁₁ | H | H | H | |
| Anisalkohol | 0 | 0 | 0 | 1 | H | H | - | - | - | - | R^{7,8,10,11} = H | R⁹=OCH₃ |
| 2-Methyl-5-phenyl-pentan-1-ol | 0 | 3 | 1 | 1 | H | H | H | CH₃ | H | H | H | |
| 1,1-Dimethyl-3-phenyl-pro pan-1-ol | 0 | 0 | 1 | 1 | CH₃ | CH₃ | H | H | - | - | H | |
| Benzylalkohol | 0 | 0 | 0 | 1 | H | H | - | - | - | - | H | |
| 2-Phenylethan-1-ol | 0 | 0 | 0 | 2 | H | H | - | - | - | - | H | |
| 3-Phenylpropan-1-ol | 0 | 0 | 0 | 3 | H | H | - | - | - | - | H | |
| 3-(3'-Chlorphenyl)-2-ethyl-propan-1-ol | 0 | 1 | 1 | 1 | H | H | C₂H₅ | H | H | H | R^{7,9,10,11} = H | R⁸ = Cl |
| 2,2-Dimethyl-3-phenylpropan-1-ol | 0 | 1 | 1 | 1 | H | H | CH₃ | CH₃ | H | H | H | |
| 2,2-Dimethyl-3-(3'-methylphenyl)-propan-1-ol | 0 | 1 | 1 | 1 | H | H | CH₃ | CH₃ | H | H | R^{7,9,10,11} = H | R⁸ = CH₃ |
| 2-Ethyl-3-(3'-methylphenyl)-propan-1-ol | 0 | 1 | 1 | 1 | H | H | C₂H₅ | H | H | H | R^{7,9,10,11} = H | R⁸ = CH₃ |
| 4-Phenylbutan-1-ol | 0 | 0 | 0 | 4 | H | H | - | - | - | - | H | |
| 3-(2'-Chlorphenyl)-2-ethylpropan-1-ol | 0 | 1 | 1 | 1 | H | H | C₂H₅ | H | H | H | R^{8,9,10,11} = H | R⁷ = Cl |
| 5-Phenylpentan-1-ol | 0 | 0 | 0 | 5 | H | H | - | - | - | - | H | |
| 3-(4'-Chlorphenyl)-2-ethyl-propan-1-ol | 0 | 1 | 1 | 1 | H | H | C₂H₅ | H | H | H | R^{7,8,10,11} = H | R⁹ = Cl |
| 2-Allyl-3-phenylpropan-1-ol | 0 | 1 | 1 | 1 | H | H | C₃H₅ | H | H | H | H | |
| 3-(3',4'-Dichlorphenyl)-2-ethylpropan-1-ol | 0 | 1 | 1 | 1 | H | H | C₂H₅ | H | H | H | R^{7,10,11} = H | R^{8,9} = Cl |
| 2-Ethyl-3-phenylpropan-1-ol | 0 | 1 | 1 | 1 | H | H | C₂H₅ | H | H | H | H | |
| 2-Ethyl-3-(2'-methylphenyl)-propan-1-ol | 0 | 1 | 1 | 1 | H | H | C₂H₅ | H | H | H | R^{8,9,10,11} = H | R⁷ = CH₃ |
| 2-Ethyl-3-(4'-methylphenyl)-propan-1-ol | 0 | 1 | 1 | 1 | H | H | C₂H₅ | H | H | H | R^{7,8,10,11} = H | R⁹ = CH₃ |
| 3-(3',4'-Dimethylphenyl)-2-ethylpropan-1-ol | 0 | 1 | 1 | 1 | H | H | C₂H₅ | H | H | H | R^{7,10,11} = H | R^{8,9} = CH₃ |
| 2-Ethyl-3-(4'-methoxyphenyl)-propan-1-ol | 0 | 1 | 1 | 1 | H | H | C₂H₅ | H | H | H | R^{7,8,10,11} = H | R⁹ = OCH₃ |
| 3-(3',4'-Dimethoxyphenyl)-2-ethylpropan-1-ol | 0 | 1 | 1 | 1 | H | H | C₂H₅ | H | H | H | R^{7,10,11} = H | R^{8,9} = OCH₃ |
| 2-n-Pentyl-3-phenylpropan-1-ol | 0 | 1 | 1 | 1 | H | H | n-C₅H₁₁ | H | H | H | H | |

Besonders bevorzugte Alkohole der Struktur AA-1 sind solche, bei denen m = 0 ist. Weitere besonders bevorzugte Alkohole der Struktur AA-1 sind solche, bei denen mindestens einer der Reste R¹, R², R³, R⁴, R⁵ oder R⁶ ≠ H ist. Weitere besonders bevorzugte Alkohole der Struktur AA-1 sind solche, bei denen m = 0 und mindestens einer der Reste R¹, R², R³, R⁴, R⁵ oder R⁶ ≠ H ist. Beispiele für die als Substituenten in den erfindungsgemäß verwendeten Verbindungen genannten C₁- bis C₅-Alkylreste sind die Gruppen Methyl, Ethyl, Propyl, Isopropyl, n-Butyl, tert.-Butyl, n-Pentyl, Amyl, Isoamyl. Ethyl, Methyl, Propyl, Isopropyl, n-Butyl, tert.-Butyl, n-Pentyl, Amyl, Isoamyl sind bevorzugte Alkylreste, besonders bevorzugt sind Methyl und n-Pentyl. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₅-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Erfindungsgemäß bevorzugte Beispiele für Halogenatome sind F-, Cl- oder Br-Atome, Cl-Atome sind ganz besonders bevorzugt. Erfindungsgemäß besonders bevorzugte Mikrokapseln a) sind dadurch gekennzeichnet, dass der deodorierend wirkende aromatische Alkohol der Struktur (AA-1) ausgewählt ist aus Anisalkohol, 2-Methyl-5-phenyl-pentan-1-ol, 1,1-Dimethyl-3-phenyl-propan-1-ol, 3-Phenylpropan-1-ol, 4-Phenylbutan-1-ol, 5-Phenylpentan-1-ol, 2-Benzylheptan-1-ol, 2,2-Dimethyl-3-phenylpropan-1-ol und 2-n-Pentyl-3-phenylpropan-1-ol. Außerordentlich bevorzugt ist 2-Benzylheptan-1-ol. Erfindungsgemäß sind Zusammensetzungen bevorzugt, die als desodorierendes oder schweißhemmendes Produkt konfektioniert sind und mindestens einen schweißhemmenden Wirkstoff, ausgewählt aus Aluminiumchlorhydroxiden, Aluminiumzirconiumchlorhydraten und Mischungen hiervon und Mikrokapseln a) enthalten, die mindestens einen deodorierend wirkenden aromatischen Alkohol der Struktur (AA-1) umfassen.

Die erfindungsgemäßen Mittel, die als Deodorantien oder Antitranspirantien formuliert werden, können variierende Wassergehalte aufweisen. Der Einsatz der Mikrokapseln ist sowohl in wasserarmen bis wasserfreien als auch in wässrigen Mitteln problemlos möglich. Wasserarm oder wasserfrei formulierte Deodorantien oder Antitranspirantien enthalten dabei, bezogen auf ihr Gewicht, bevorzugt null bis weniger als 5 Gew.-%, insbesondere null bis weniger als 3 Gew.-% freies Wasser. Unter freiem Wasser versteht man im Sinne der vorliegenden Anmeldung das Wasser, das nicht als Kristallwasser, Hydratationswasser oder ähnlich molekular gebundenes Wasser in den eingesetzten Bestandteilen, insbesondere in den schweißhemmenden Wirkstoffen, enthalten ist. Deodorantien oder Antitranspirantien, die in Form von Emulsionen oder Hydrogelen formuliert sind, enthalten vorzugsweise deutlich höhere Mengen an freiem Wasser. Bevorzugte deodorierende oder schweißhemmende Emulsionen sind als Öl-in-Wasser-Emulsion oder als Wasser-in-Öl-Emulsion formuliert. Weitere bevorzugte deodorierende oder schweißhemmende Emulsionen sind als Öl-in-Wasser-Mikroemulsion oder als Wasser-in-ÖI-Mikroemulsion formuliert. Derartige Emulsionen und Mikroemulsionen eignen sich sowohl zur Applikation in Stiftform als auch zur Applikation mit einer Rollkugel als auch zur Applikation mit einem Pumpzerstäuber oder einer Aerosolvorrichtung. Für die Applikation einer schweißhemmenden Emulsion mit einer Aerosolvorrichtung sind wegen der geringeren Korrosionsgefahr Wasser-in-ÖI-Emulsionen und Wasser-in-Öl-Mikroemulsionen bevorzugt.

Weiterhin sind erfindungsgemäße Zusammensetzungen bevorzugt, die als deodorierende oder schweißhemmende, zur Roll-on-Applikation oder zum Versprühen geeignete Öl-in-Wasser-Emulsionen formuliert sind und deren Wassergehalt mindestens 60 Gew.-%, bevorzugt 65 - 90 Gew.-%, besonders bevorzugt 70 - 85 Gew.-%, außerordentlich bevorzugt 75 - 80 Gew.-%, jeweils bezogen auf freies Wasser in der Gesamtzusammensetzung, beträgt.

Weiterhin sind erfindungsgemäße Zusammensetzungen bevorzugt, die als deodorierende oder schweißhemmende, zur Roll-on-Applikation oder zum Versprühen geeignete Hydrogele formuliert sind und deren Wassergehalt mindestens 60 Gew.-%, bevorzugt 65 - 90 Gew.-%, besonders bevorzugt 70 - 85 Gew.-%, außerordentlich bevorzugt 75 - 80 Gew.-%, jeweils bezogen auf freies Wasser in der Gesamtzusammensetzung, beträgt.

Weiterhin sind erfindungsgemäße Zusammensetzungen bevorzugt, die als deodorierende oder schweißhemmende Öl-in-Wasser-Emulsionsstifte formuliert sind und deren Wassergehalt 6 - 60 Gew.-%, bevorzugt 10 - 40 Gew.-%, besonders bevorzugt 20 - 30 Gew.-%, jeweils bezogen auf freies Wasser in der Gesamtzusammensetzung, beträgt.

Weiterhin sind erfindungsgemäße Zusammensetzungen bevorzugt, die als deodorierende oder schweißhemmende Wasser-in-ÖI-Emulsionsstifte formuliert sind und deren Wassergehalt 6 - 50 Gew.-%, bevorzugt 10 - 40 Gew.-%, besonders bevorzugt 20 - 30 Gew.-%, jeweils bezogen auf freies Wasser in der Gesamtzusammensetzung, beträgt.

Weiterhin sind erfindungsgemäße Zusammensetzungen bevorzugt, die als deodorierende oder schweißhemmende Wasser-in-ÖI-Emulsionssprays formuliert sind und deren Wassergehalt 6 - 50 Gew.-%, bevorzugt 10 - 40 Gew.-%, besonders bevorzugt 20 - 35 Gew.-%, außerordentlich bevorzugt 25 - 30 Gew.-%, jeweils bezogen auf freies Wasser in der Gesamtzusammensetzung, beträgt.

Weiterhin sind erfindungsgemäße Zusammensetzungen bevorzugt, die als deodorierende oder schweißhemmende Wasser-in-ÖI-Emulsionsgele formuliert sind und deren Wassergehalt 6 - 40 Gew.-%, bevorzugt 7 - 30 Gew.-%, besonders bevorzugt 8 - 22 Gew.-%, außerordentlich bevorzugt 25 - 30 Gew.-%, jeweils bezogen auf freies Wasser in der Gesamtzusammensetzung, beträgt.

Für Zusammensetzungen, die mit einem Treibgas versprüht werden, beziehen sich diese Mengenangaben auf die treibgasfreie Gesamtzusammensetzung.

Die erfindungsgemäßen Mittel können auch als Haarpflegemittel (Conditioner) formuliert werden. In diesem Falle enthalten sie mindestens einen Pflegestoff. Erfindungsgemäß bevorzugte Zusammensetzungen sind dadurch gekennzeichnet, dass sie als Haarpflegemittel konfektioniert sind und - bezogen auf ihr Gewicht - 0,05 bis 30 Gew.-% mindestens eines Pflegestoffes aus der Gruppe der QAV und/oder der kationischen Polymere und/oder der Silikone enthalten.

Die quartären Ammoniumverbindungen (QAV) wurden z.T. bereits weiter oben beschrieben. Einsetzbar sind vor allem kationische Tenside vom Typ der quartären Ammoniumverbindungen, der Esterquats und der Amidoamine, die in der dieser Anmeldung zu Grunde liegenden deutschen Prioritätsanmeldung DE 102010040567.1 auf den ursprünglich eingereichten Seiten 75, Zeile 34 bis Seite 76, Zeile 4, beschrieben sind. Dieser und die folgenden Verweise auf die Prioritätsanmeldung DE 102010040567.1 dienen lediglich der Illustration der Gegenstände der Anmeldung/des Patents, ergänzen diese aber nicht.

Besonders bevorzugte erfindungsgemäße Haarpflegemittel enthalten als kationischen Pflegestoff - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Tensid(e) aus der Gruppe der quartären Ammoniumverbindungen und/oder der Esterquats und/oder der Amidoamine, wobei bevorzugte kationische(s) Tensid(e) ausgewählt ist/sind aus Alkyltrimethylammoniumchloriden mit vorzugsweise 10 bis 18 Kohlenstoffatomen im Alkylrest und/oder Dialkyldimethylammoniumchloride mit vorzugsweise 10 bis 18 Kohlenstoffatomen im Alkylrest und/oder Trialkylmethylammoniumchloride mit vorzugsweise 10 bis 18 Kohlenstoffatomen im Alkylrest und/oder Cetyltrimethylammoniumchlorid und/oder Stearyltrimethylammoniumchlorid und/oder Distearyldimethylammoniumchlorid und/oder Lauryldimethylammoniumchlorid und/oder Lauryldimethylbenzylammoniumchlorid und/oder Tricetylmethylammoniumchlorid Quaternium-27 und/ oder Quaternium-83 und/oder N-Methyl-N(2-hydroxyethyl)-N,N-(ditalgacyloxyethyl)ammonium-methosulfat und/oder N-Methyl-N(2-hydroxyethyl)-N,N-(distearoyloxyethyl)ammonium-methosulfat und/oder N,N-Dimethyl-N,N-distearoyloxyethyl-ammoniumchlorid und/oder N,N-Di-(2-hydroxyethyl)-N,N-(fettsäureesterethyl)-ammoniumchlorid.

Zusätzlich zu oder anstelle von QAV können auch kationische Polymere in den erfindungsgemäßen Mitteln eingesetzt werden. Geeignete kationische Polymere sind solche, die in der dieser Anmeldung zu Grunde liegenden deutschen Prioritätsanmeldung DE 102010040567.1 auf den ursprünglich eingereichten Seiten 76, Zeile 36 bis Seite 77, Zeile 27, beschrieben sind.

Die erfindungsgemäßen Zusammensetzungen können auch als Hautbehandlungsmittel, beispielsweise Cremes, Lotionen oder Gele formuliert werden. Solche Zusammensetzungen enthalten neben den Mikrokapseln a) bevorzugt mindestens einen hautaufhellenden und/oder hautberuhigenden und/oder feuchtigkeitsspendenden und/oder sebumregulierenden Wirkstoff. Erfindungsgemäß bevorzugte hautaufhellende Wirkstoffe sind ausgewählt aus solchen, die in der dieser Anmeldung zu Grunde liegenden deutschen Prioritätsanmeldung DE 102010040567.1 auf den ursprünglich eingereichten Seiten 77, Zeile 34 bis Seite 78, Zeile 6, beschrieben sind. Die hautaufhellenden Wirkstoffe sind vorzugsweise in einer Menge von 0,05 bis 5 Gew.-%, bevorzugt von 0,1 - 2 Gew-%, jeweils bezogen auf die gesamte Zusammensetzung, enthalten.

In einer weiteren bevorzugten Ausführungsform enthalten die erfindungsgemäßen Zusammensetzungen mindestens einen hautberuhigenden Wirkstoff. Erfindungsgemäß bevorzugte hautberuhigende Wirkstoffe sind ausgewählt aus Allantoin, α-Bisabolol und α-Liponsäure. Die hautberuhigenden Wirkstoffe sind vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten.

In einer weiteren bevorzugten Ausführungsform enthalten die erfindungsgemäßen Zusammensetzungen mindestens einen sebumregulierenden Wirkstoff. Erfindungsgemäß bevorzugte sebumregulierende Wirkstoffe sind ausgewählt aus Acnacidol, Azelainsäure, Azeloglycina und einem Extrakt aus Spiraea Ulmaria. Der Extrakt ist z.B. im Produkt Seburegul der Firma Silab enthalten. Die sebumregulierenden Wirkstoffe sind vorzugsweise in Mengen von 0,001 - 5 Gew.-%, bevorzugt 0,01 - 2 Gew.-% und besonders bevorzugt 0,1 - 1 Gew.-%, jeweils bezogen auf die gesamte Zusammensetzung, enthalten.

In einer weiteren bevorzugten Ausführungsform enthalten die erfindungsgemäßen Zusammensetzungen mindestens eine alpha-Hydroxycarbonsäure, alpha-Ketocarbonsäure oder beta-Hydroxycarbonsäure oder deren Ester-, Lacton- oder Salzform. Erfindungsgemäß geeignete alpha-Hydroxycarbonsäuren oder alpha-Ketocarbonsäuren und deren Ester sind solchen, die in der dieser Anmeldung zu Grunde liegenden deutschen Prioritätsanmeldung DE 102010040567.1 auf den ursprünglich eingereichten Seiten 78, Zeile 29 bis Seite 79, Zeile 2, beschrieben sind. Die alpha-Hydroxycarbonsäuren, alpha-Ketocarbonsäuren oder beta-Hydroxycarbonsäuren oder ihre Derivate sind vorzugsweise in Mengen von 0,1 - 10 Gew.-%, bevorzugt 0,5 - 5 Gew.-%, jeweils bezogen auf die gesamte Zusammensetzung, enthalten.

In einer weiteren bevorzugten Ausführungsform enthalten die erfindungsgemäßen Zusammensetzungen mindestens ein Flavonoid oder mindestens einen Flavonoid-reichen Pflanzenextrakt, wie sie in der dieser Anmeldung zu Grunde liegenden deutschen Prioritätsanmeldung DE 102010040567.1 auf den ursprünglich eingereichten Seiten 79, Zeilen 9 bis 24, beschrieben sind. Erfindungsgemäß werden die Flavonoide vorzugsweise in Mengen von 0,0001 - 1 Gew.-%, bevorzugt 0,0005 - 0,5 Gew.-% und besonders bevorzugt 0,001 - 0,1 Gew.-%, jeweils bezogen auf die Flavonoidaktivsubstanz in der gesamten kosmetischen Zusammensetzung, eingesetzt.

In einer weiteren bevorzugten Ausführungsform enthalten die erfindungsgemäßen Zusammensetzungen mindestens ein Isoflavonoid oder mindestens einen Isoflavonoid-reichen Pflanzenextrakt, wie sie in der dieser Anmeldung zu Grunde liegenden deutschen Prioritätsanmeldung DE 102010040567.1 auf den ursprünglich eingereichten Seiten 79, Zeile 33 bis Seite 80, Zeile 23, beschrieben sind. Erfindungsgemäß werden die Isoflavonoide vorzugsweise in Mengen von 0,00001 - 1 Gew.-%, bevorzugt 0,0005 - 0,5 Gew.-% und besonders bevorzugt 0,001 - 0,1 Gew.-%, jeweils bezogen auf die Isoflavonoidaktivsubstanz in der gesamten kosmetischen Zusammensetzung, eingesetzt.

In einer weiteren bevorzugten Ausführungsform enthalten die erfindungsgemäßen Zusammensetzungen mindestens ein Polyphenol oder einen Polyphenol-reichen Pflanzenextrakt, wie in der dieser Anmeldung zu Grunde liegenden deutschen Prioritätsanmeldung DE 102010040567.1 auf den ursprünglich eingereichten Seiten 80, Zeile 31 bis Seite 81, Zeile 13, beschrieben ist. Erfindungsgemäß werden die Polyphenole vorzugsweise in Mengen von 0,001 - 10 Gew.-%, bevorzugt 0,005 - 5 Gew.-% und besonders bevorzugt 0,01 - 3 Gew.-%, jeweils bezogen auf die gesamte kosmetische Zusammensetzung, eingesetzt.

In einer weiteren bevorzugten Ausführungsform enthalten die erfindungsgemäßen Zusammensetzungen mindestens ein Ubichinon oder ein Ubichinol oder deren Derivate gemäß der dieser Anmeldung zu Grunde liegenden deutschen Prioritätsanmeldung DE 102010040567.1, ursprünglich eingereichte Seite 81, Zeilen 19 bis 36.

In einer weiteren bevorzugten Ausführungsform enthalten die erfindungsgemäßen Zusammensetzungen Silymarin gemäß der dieser Anmeldung zu Grunde liegenden deutschen Prioritätsanmeldung DE 102010040567.1, ursprünglich eingereichte Seite 82, Zeilen 1 bis 8.

In einer weiteren bevorzugten Ausführungsform enthalten die erfindungsgemäßen Zusammensetzungen mindestens ein natürlich vorkommendes Xanthin-Derivat gemäß der dieser Anmeldung zu Grunde liegenden deutschen Prioritätsanmeldung DE 102010040567.1, ursprünglich eingereichte Seite 82, Zeilen 10 - 15.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß ist Ectoin vorzugsweise in Mengen von 0,0001 - 1 Gew.-%, bevorzugt 0,001 - 0,5 Gew.-% und besonders bevorzugt 0,005 - 0,01 Gew.-%, jeweils bezogen auf die gesamte Zusammensetzung, enthalten.

In einer weiteren bevorzugten Ausführungsform enthalten die erfindungsgemäßen Zusammensetzungen mindestens eine anorganische und/oder mindestens eine organische UV-Filtersubstanz gemäß der dieser Anmeldung zu Grunde liegenden deutschen Prioritätsanmeldung DE 102010040567.1, ursprünglich eingereichte Seite 82, Zeile 25, bis Seite 84, Zeile 14.

In einer weiteren bevorzugten Ausführungsform enthalten die erfindungsgemäßen Zusammensetzungen mindestens einen selbstbräunenden Wirkstoff. Erfindungsgemäß bevorzugte selbstbräunende Wirkstoffe sind ausgewählt aus Dihydroxyaceton und Erythrulose. Erfindungsgemäß sind die selbstbräunenden Wirkstoffe vorzugsweise in Mengen von 0,1 - 15 Gew.-%, bevorzugt 0,5 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid gemäß der dieser Anmeldung zu Grunde liegenden deutschen Prioritätsanmeldung DE 102010040567.1, ursprünglich eingereichte Seite 84, Zeilen 26 bis 31.

Die erfindungsgemäßen Mittel können auch als Stylingmittel konfektioniert werden. In diesem Falle enthalten sie mindestens einen Inhaltsstoff aus der Gruppe der haarfestigenden oder Haarstyling-Wirkstoffe gemäß der dieser Anmeldung zu Grunde liegenden deutschen Prioritätsanmeldung DE 102010040567.1, ursprünglich eingereichte Seite 85, Zeilen 1 bis 22.

Weitere vorteilhafte Eigenschaften der erfindungsgemäßen Mittel können durch den Zusatz weiterer Inhaltsstoffe erzielt werden. Nachfolgend werden diese Inhaltsstoffe beschrieben, wobei zum Teil exemplarisch "Färbemittel", "Stylingmittel", "Deodorantien" usw. genannt werden. Diese Bezeichnungen sind jeweils exemplarisch und nicht beschränkend zu verstehen. Vielmehr kann ein optionaler Inhaltsstoff eines exemplarisch beschriebenen "Stylingmittels" selbstverständlich auch in einem erfindungsgemäßen Färbemittel enthalten sein.

Als weiterhin vorteilhaft hat es sich gezeigt, wenn Polymere in den erfindungsgemäßen Mitteln enthalten sind. In einer bevorzugten Ausführungsform werden den erfindungsgemäßen Mitteln daher Polymere zugesetzt, wobei sich sowohl kationische, anionische, amphotere als auch nichtionische Polymere als wirksam erwiesen haben.

Erfindungsgemäß einsetzbar sind vorzugsweise kationische bzw. amphotere Polymere gemäß der dieser Anmeldung zu Grunde liegenden deutschen Prioritätsanmeldung DE 102010040567.1, ursprünglich eingereichte Seite 85, Zeile 36 bis Seite 90, Zeile 21. Die kationischen bzw. amphoteren Polymere sind in den erfindungsgemäßen Mitteln bevorzugt in Mengen von 0,05 - 10 Gew.-%, besonders bevorzugt 0,1 - 5 Gew.-%, jeweils bezogen auf das gesamte Mittel, enthalten.

Bei den anionischen Polymeren handelt es sich um anionische Polymere, die Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann. Besonders bevorzugt ist das Homopolymer der 2-Acrylamido-2-methylpro-pansulfonsäure, das beispielsweise unter der Bezeichnung Rheothik®11-80 im Handel erhältlich ist. Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind die, die in der dieser Anmeldung zu Grunde liegenden deutschen Prioritätsanmeldung DE 102010040567.1, ursprünglich eingereichte Seite 91, Zeilen 9 bis 30 offenbart sind.

Die erfindungsgemäßen Mittel können in einer weiteren Ausführungsform nichtionogene Polymere enthalten. Geeignete nichtionogene Polymere sind die, die in der dieser Anmeldung zu Grunde liegenden deutschen Prioritätsanmeldung DE 102010040567.1, ursprünglich eingereichte Seite 91, Zeile 34 bis Seite 92, Zeile 16, offenbart sind.

Es ist erfindungsgemäß auch möglich, dass die Zubereitungen mehrere, insbesondere zwei verschiedene Polymere gleicher Ladung und/oder jeweils ein ionisches und ein amphoteres und/oder nicht ionisches Polymer enthalten. Die Polymere sind in den erfindungsgemäßen Mitteln bevorzugt in Mengen von 0,05 - 10 Gew.-%, besonders bevorzugt 0,1 - 5 Gew.-%, außerordentlich bevorzugt 0,1 - 3 Gew.-%, jeweils bezogen auf das gesamte Mittel, enthalten.

Die erfindungsgemäßen Mittel können weiterhin eine 2-Pyrrolidinon-5-carbonsäure und deren Derivate gemäß der dieser Anmeldung zu Grunde liegenden deutschen Prioritätsanmeldung DE 102010040567.1, ursprünglich eingereichte Seite 92, Zeile 26 bis 31, enthalten.

Schließlich können die erfindungsgemäßen Mittel auch Pflanzenextrakte gemäß der dieser Anmeldung zu Grunde liegenden deutschen Prioritätsanmeldung DE 102010040567.1, ursprünglich eingereichte Seite 92, Zeile 33 bis Seite 93, Zeile 35, enthalten.

Zusätzlich kann es sich als vorteilhaft erweisen, wenn in den erfindungsgemäßen Mitteln Penetrationshilfsstoffe und/ oder Quellmittel gemäß der dieser Anmeldung zu Grunde liegenden deutschen Prioritätsanmeldung DE 102010040567.1, ursprünglich eingereichte Seite 94, Zeilen 1 bis 8, enthalten sind.

Eine besonders bevorzugte Gruppe von Inhaltsstoffen stellen die Silikone dar. Erfindungsgemäße bevorzugte Mittel enthalten mindestens ein Silicon, bevorzugt ein Silicon gemäß der dieser Anmeldung zu Grunde liegenden deutschen Prioritätsanmeldung DE 102010040567.1, ursprünglich eingereichte Seite 94, Zeile 12 bis Seite 103, Zeile 5.

Ferner können die erfindungsgemäßen Mittel weitere Wirk-, Hilfs- und Zusatzstoffe, gemäß der dieser Anmeldung zu Grunde liegenden deutschen Prioritätsanmeldung DE 102010040567.1, ursprünglich eingereichte Seite 103, Zeile 7 bis Seite 104, Zeile 25, enthalten.

Als weiteren optionalen Bestandteil können die erfindungsgemäßen Mittel 0,01 bis 10 Gew.-% Chitosan und/oder Chitosanderivat(e) gemäß der dieser Anmeldung zu Grunde liegenden deutschen Prioritätsanmeldung DE 102010040567.1, ursprünglich eingereichte Seite 104, Zeile 27 bis Seite 106, Zeile 10, enthalten.

Eine weitere bevorzugte Gruppe von Inhaltsstoffen der erfindungsgemäßen Mittel sind Vitamine, Provitamine oder Vitaminvorstufen gemäß der dieser Anmeldung zu Grunde liegenden deutschen Prioritätsanmeldung DE 102010040567.1, ursprünglich eingereichte Seite 104, Zeile 12 bis Seite 109, Zeile 36.

Bevorzugt ist auch der zusätzlich Einsatz von Bisabolol und/oder Bisabololoxiden in den erfindungsgemäßen Mitteln. Hier sind erfindungsgemäße Mittel bevorzugt, die zusätzlich 0,001 - 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol enthalten.

Abhängig vom Verwendungszweck der erfindungsgemäßen Mittel können diese variierende Wassergehalte aufweisen. Der Einsatz der Mikrokapseln ist sowohl in wasserarmen bis wasserfreien als auch in wässrigen Mitteln problemlos möglich. Wasserarm oder wasserfrei formulierte Mittel wie beispielsweise bestimmte Deodorantien oder Antitranspirantien enthalten dabei bezogen auf ihr Gewicht vorzugsweise weniger als 5 Gew.-%, insbesondere weniger als 2 Gew.-% Wasser. Dabei ist freies Wasser gemeint, wie vorstehend definiert.

Kosmetische Reinigungsmittel wie Shampoos, Duschgele, etc., aber auch Konditioniermittel, Entwickleremulsionen für die oxidative Haarfärbung, Sonnenschutzmittel, Hautcremes etc. werden bevorzugt wasserbasiert formuliert und enthalten dann deutlich höhere Mengen an Wasser. Hier sind erfindungsgemäße Zusammensetzungen bevorzugt, deren Wassergehalt mindestens 40 Gew.-%, bevorzugt 45 bis 90 Gew.-%, besonders bevorzugt 50 - 85 Gew.-%, außerordentlich bevorzugt 55 - 80 Gew.-%, jeweils bezogen auf die Gesamtzusammensetzung, beträgt.

Unabhängig davon, für welchen Verwendungszweck die erfindungsgemäßen kosmetischen Produkte konfektioniert werden, haben sich bestimmte Inhaltsstoffe als besonders geeignet für die Verkapselung erwiesen. Bevorzugte erfindungsgemäße Zusammensetzungen enthalten entweder Mikrokapseln ohne Inhalt (aus Gründen der optischen Differenzierung, z.B. bei Mund- und Zahnpflegemitteln), deren Wandung vorzugsweise eingefärbt ist, oder Mikrokapseln, die mindestens einen der folgenden Inhaltsstoffe verkapselt enthalten:
- Alkohole (s.o. zu desodorierenden Alkoholen, insbesondere solchen der Formel AA-1)
- Parfümöle,
- Pflegestoffe,
- natürliche oder synthetische Öle,
- Silikonöle,
- Farbstoffe,
- kühlende Substanzen.

Erfindungsgemäß bevorzugte Zusammensetzungen sind gekennzeichnet durch einen Gehalt an erfindungsgemäßen Mikrokapseln a), die in ihrem Inneren mindestens eine kühlende Substanz enthalten. Unter einer kühlenden Substanz im Sinne der vorliegenden Anmeldung werden Wirkstoffe verstanden, die bei Applikation auf die Haut oder Schleimhaut infolge Oberflächenanästhesierung und Reizung der kälteempfindlichen Nerven ein Kältegefühl erzeugen, auch wenn die behandelten Hautpartien tatsächlich normale bzw. erhöhte Temperatur zeigen. Bevorzugte kühlende Substanzen sind insbesondere Menthol, Isopulegol sowie Mentholderivate, z. B. Menthyllactat, Menthylpyrrolidoncarbonsäure, Menthylmethylether, Menthoxypropandiol, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol. Als kühlende Substanzen besonders bevorzugt sind Menthol, Isopulegol, Menthyllactat, Menthoxypropandiol und Menthylpyrrolidoncarbonsäure. Bevorzugte erfindungsgemäße Zusammensetzungen enthalten mindestens eine kühlende Substanz in einer Gesamtmenge von 0,01 - 1,5 Gew.-%, bevorzugt 0,1 - 1 Gew.-% und besonders bevorzugt 0,2 - 0,5 Gew.-%, jeweils bezogen auf das Gesamtgewicht der kühlenden Substanz(en) in der (treibmittelfreien) Zusammensetzung, wobei die kühlende Substanz in den erfindungsgemäßen Mikrokapseln a) verkapselt vorliegt.

Eine weitere besonders bevorzugte Gruppe zu verkapselnder Inhaltsstoffe stellen die Duftstoffe dar. Der Geruch eines Produkts und der Geruch des Anwenders bei und nach der Anwendung stellen einen nicht unerheblichen Grund für die Wahl eines speziellen kosmetischen Mittels dar. Zudem erlaubt der Duft dem Verbraucher, ein typisches und unverwechselbares Produkt zu kaufen.

Als Parfümöle bzw. Duftstoffe können erfindungsgemäß einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan, zu den Aldehyden z.B. die linearen Alkanale mit 8 - 18 C-Atomen, Citral, Citronellal, Citronellyloxyacetaldehyd, Cyclamenaldehyd, Lilial und Bourgeonal, zu den Ketonen z.B. die Jonone, alpha-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Bestimmte Riechstoffverbindungen können in Form ihrer hydrolysierbaren Derivate eingesetzt werden. Diese Derivate weisen meist eine geringere Flüchtigkeit und/oder eine höhere Lagerstabilität auf als die underivatisierten Riechstoffverbindungen. Durch Hydrolyse der Derivate werden die Riechstoffverbindungen zeitverzögert freigesetzt, wodurch beispielsweise eine länger andauernde Beduftung und/oder variierende Dufteffekte erzielt werden. Erfindungsgemäß bevorzugte hydrolysierbare Derivate von Riechstoffverbindungen mit einer alkoholischen Funktion sind Kieselsäureester. Durch die Aufteilung des Gesamt-Parfümgehaltes in Parfüm, welches Kieselsäureester-verestert ist und Parfüm, welches unverestert vorliegt ist, läßt sich eine Vielzahl von Produktcharakteristiken realisieren. So ist es beispielsweise denkbar und möglich, den Gesamt-Parfümgehalt der erfindungsgemäßen Mittel in zwei Portionen x und y aufzuteilen, wobei der Anteil x aus haftfesten, d.h. weniger flüchtigen und der Anteil y aus leichter flüchtigen Parfümölen besteht. Die allgemeine Beschreibung der einsetzbaren Parfüme (siehe oben) stellte allgemein die unterschiedlichen Substanzklassen von Riechstoffen dar. Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Die oben beschriebene Ausführungsform der vorliegenden Erfindung, in der die leichter flüchtigen Riechstoffe bzw. Duftstoffe in die erfindungsgemäße Mikrokapsel a) inkorporiert werden, ist eine solche Methode zur Riechstoffixierung. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt. Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undecalacton, Vanilin, Veratrumaldehyd, Zimtaldehyd, Zimtalkohol, Zimtsäure, Zimtsäureethylester, Zimtsäurebenzylester.

Zu den leichter flüchtigen Riechstoffen zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Bevorzugte erfindungsgemäße Zusammensetzungen enthalten mindestens eine Riechstoffverbindung in einer Gesamtmenge von 0,01 - 5 Gew.-%, bevorzugt 0,1 - 2 Gew.-% und besonders bevorzugt 0,2 - 1 Gew.-%, jeweils bezogen auf das Gesamtgewicht der Riechstoffverbindung(en) in der Zusammensetzung, wobei die Riechstoffverbindung(en) in den erfindungsgemäßen Mikrokapseln a) verkapselt vorliegt bzw. vorliegen.

Weitere bevorzugte erfindungsgemäße Zusammensetzungen enthalten mindestens eine Riechstoffverbindung in einer Gesamtmenge von 0,01 - 5 Gew.-%, bevorzugt 0,1 - 2 Gew.-% und besonders bevorzugt 0,2 - 1 Gew.-%, jeweils bezogen auf das Gesamtgewicht der Riechstoffverbindung(en) in der Zusammensetzung, wobei die Riechstoffverbindung(en) unverkapselt vorliegt bzw. vorliegen.

Weitere bevorzugte erfindungsgemäße Zusammensetzungen enthalten mindestens eine in den erfindungsgemäßen Mikrokapseln a) verkapselte Riechstoffverbindung in einer Gesamtmenge von 0,01 - 5 Gew.-%, bevorzugt 0,1 - 2 Gew.-% und besonders bevorzugt 0,2 - 1 Gew.-%, und mindestens eine unverkapselte Riechstoffverbindung in einer Gesamtmenge von 0,01 - 5 Gew.-%, bevorzugt 0,1 - 2 Gew.-% und besonders bevorzugt 0,2 - 1 Gew.-%, jeweils bezogen auf das Gesamtgewicht der Riechstoffverbindung(en) in der Zusammensetzung.

Zusätzlich zu oder anstelle von Duftstoffen können Ölkörper verkapselt und in die erfindungsgemäßen Zusammensetzungen inkorporiert werden. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimer-diol oder Trimertriol) und/ oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/ oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN = C12-15 Alkylbenzoat), Dialkylether, Ringöffnungsprodukte von epoxidierten Fettsäureestern mit Polyolen, Siliconöle und/oder aliphatische bzw. naphthenische Kohlenwasserstoffe in Betracht. Eine weitere bevorzugte Gruppe von Ölen, die verkapselt werden können, sind die Silikonöle, die in der dieser Anmeldung zu Grunde liegenden deutschen Prioritätsanmeldung DE 102010040567.1, ursprünglich eingereichte Seite 94, Zeile 12 bis Seite 103, Zeile 5, ausführlich beschrieben sind. Bevorzugte erfindungsgemäße Zusammensetzungen enthalten mindestens ein natürliches oder synthetisches Öl oder ein Silikonöl in einer Gesamtmenge von 0,01 - 5 Gew.-%, bevorzugt 0,1 - 2 Gew.-% und besonders bevorzugt 0,2 - 1 Gew.-%, jeweils bezogen auf das Gesamtgewicht des Öls in der Zusammensetzung, wobei dieses Öl in den erfindungsgemäßen Mikrokapseln a) verkapselt vorliegt.

### Beispiele:

### I. Synthesebeispiele:

### Beispiel 1: Herstellung von Copolymeren

### a) AMPS-Hydroxybutylacrylat

Für den 1500 g Ansatz werden 891 g demineralisiertes Wasser zusammen mit 585 g AMPS (50-% wässrige Lösung) und 7,5 g 4-Hydroxybutylacrylat (HBA) in den Reaktor gefüllt und unter Schutzgas-Atmosphäre gesetzt. Das Reaktionsgemisch wird unter Rühren (400 rpm) auf 75 °C aufgeheizt. Von dem wasserlöslichen Initiator Natriumperoxodisulfat werden 0,03 g in 15 g Wasser gelöst und mittels einer Spritze in den Reaktor injiziert, wenn die Reaktionstemperatur erreicht ist. Nach Erreichen der Maximaltemperatur beginnt eine Stunde Nachreaktion. Anschließend wird der Ansatz auf Raumtemperatur abgekühlt und mit 1,5 g Konservierungsmittel versetzt. Die wässrige Lösung wird charakterisiert über die Viskosität, Festkörper-Gehalt und den pH-Wert. Die Viskosität beträgt 540 mPas (gemessen bei 20 rpm Brookfield), der Festkörper-Gehalt beträgt 21 % und der pH-Wert liegt bei 3,3. Es werden 3 g Copolymer auf eine Petrischale gegeben und 24 Stunden bei 160 °C im Trockenschrank getrocknet. Die Auswaage beträgt 0,69 g, was einer Ausbeute von 21,6 % entspricht.

### b) AMPS-Polyakylenglykolmonomethacrylat

Die Vorlage besteht aus 912 g demineralisiertem Wasser, 240 g AMPS und 7,5 g Poly(ethylen/ propylen)glykolmonomethacrylat (Bisomer PEM 63P HD von Cognis, CAS-Nr. 589-75-9). Die Mischung wird unter Schutzgas-Atmosphäre gesetzt. Das Reaktionsgemisch wird unter Rühren (400 rpm) auf 75 °C aufgeheizt. 1,5 g Natriumperoxodisulfat werden in 15 g Wasser gelöst und mit einer Spritze in den Reaktor überführt. Nachdem die Temperatur im Reaktor ein Maximum erreicht hat und zu sinken beginnt, werden 240 g AMPS mit 83 g PEM 63P HD mittels Schlauchpumpe über einen Zeitraum von einer Stunde zu dosiert. Anschließend erfolgt eine halbstündige Nachreaktion. Anschließend wird der Ansatz auf Raumtemperatur abgekühlt und mit 1,5 g Konservierungsmittel versetzt.

Die wässrige Lösung wird charakterisiert über die Viskosität, Festkörper-Gehalt und den pH-Wert. Die Viskosität beträgt 110 mPas (gemessen bei 20 rpm Brookfield), der Festkörper-Gehalt beträgt 23 % und der pH-Wert ist 3,1. Es werden 3 g Copolymer auf eine Petrischale gegeben und 24 Stunden bei 160 °C im Trockenschrank getrocknet. Die Auswaage beträgt 0,68 g, was einer Ausbeute von 21,6 % entspricht.

### Beispiel 2: Resorcin-Kapsel

In einem 400 ml-Becherglas werden 5,5 g Resorcin unter Rühren (Rührgeschwindigkeit: etwa 1500 U/min) in 70 g Wasser gelöst und anschließend mit 2,0 g Natriumcarbonat-Lösung (20 Gew.-%ig) versetzt, worauf der pH-Wert bei ca. 7,9 liegt. Diese Lösung wird auf eine Temperatur von etwa 52 °C erwärmt. Anschließend werden 25,5 g Glutardialdehyd hinzugegeben.

Das Gemisch wird weitere ca. 10 Minuten bei einer Rührgeschwindigkeit von etwa 1500 U/min und einer Temperatur von etwa 52 °C gerührt (Vorkondensationszeit). Danach werden etwa 20 g Wasser ergänzt und ca. 2 Minuten später 1 g eines der Schutzkolloide a) Copolymer 1a, b) Copolymer 1b und c) Poly-AMPS (AMPS-Homopolymer) und wieder ca. 2 Minuten später 55 g Phenoxyethanol hinzugefügt. Direkt im Anschluss wird die Rührgeschwindigkeit auf etwa 4000 U/min erhöht und etwa zur gleichen Zeit erfolgt die Zugabe von 20,0 g Natriumcarbonat-Lösung (20 Gew.-%ig). Danach liegt der pH-Wert der Mischung bei etwa 9,7. In der Folgezeit nehmen die Viskosität und das Volumen der Mischung zu. Es wird so lange bei einer Rührgeschwindigkeit von etwa 4000 U/min weitergerührt, bis die Viskosität wieder gesunken ist. Erst danach wird die Rührgeschwindigkeit auf etwa 1500 U/min abgesenkt. Bei einer Temperatur von etwa 52 °C und etwa gleichbleibender Rührgeschwindigkeit wird der Ansatz weitere ca. 60 Minuten gerührt. Diese Phase nennt man auch Ruhephase. Im Anschluss daran wird die Mischung auf ca. 80 °C aufgeheizt und die Kapseln bei dieser Temperatur über einen Zeitraum von 3 Stunden ausgehärtet. Kapselgrößenverteilung - D (90) 5 - 10 µm; Verkapselungseffizienz ca. 90 %; Trocknungsausbeute > 90 %; Festkörper der Slurry ca. 40 Gew %.

Die erzeugten Kapseln sind formaldehydfrei und lassen sich als stabile Kern/Schale-Mikrokapseln aus der wässrigen Slurry heraus ohne Probleme zu einem trockenen fließfähigen Pulver verarbeiten.

Die Beladung der Kapseln kann anstelle von beispielsweise Phenoxyethanol auch mit anderen gasförmigen, flüssigen oder festen hydrophoben Materialien und Substanzklassen erfolgen, die unter den gegebenen Reaktionsbedingungen keine Neben- oder Parallelreaktionen eingehen. Zusätzlich zu den Phenoxyethanol-haltigen Resorcinmikrokapseln des Beispiels I.2. wurden weitere Mikrokapseln nach analogen Verfahren hergestellt:
- Beispiel I.3.: Phenoxyisopropanol-(3-Phenoxy-propan-2-ol)-haltige Resorcinmikrokapseln,
- Beispiel I.4.: Anisalkohol- haltige Resorcinmikrokapseln,
- Beispiel I.5.: 2-Methyl-5-phenyl-pentan-1-ol-haltige Resorcinmikrokapseln,
- Beispiel I.6.: 1,1-Dimethyl-3-phenyl-propan-1-ol-haltige Resorcinmikrokapseln,
- Beispiel I.7.: Benzylalkohohaltige Resorcinmikrokapseln I,
- Beispiel I.8.: 2-Phenylethan-1-ol-haltige Resorcinmikrokapseln,
- Beispiel I.9.: 3-Phenylpropan-1-ol-haltige Resorcinmikrokapseln,
- Beispiel I.10.: 4-Phenylbutan-1-ol-haltige Resorcinmikrokapseln,
- Beispiel I.11:: 5-Phenylpentan-1-ol-haltige Resorcinmikrokapseln,
- Beispiel I.12.: 2-Benzylheptan-1-ol-haltige Resorcinmikrokapseln,
- Beispiel I.13.: 2,2-Dimethyl-3-phenylpropan-1-ol-haltige Resorcinmikrokapseln,
- Beispiel I.14.: 2,2-Dimethyl-3-(3'-methylphenyl)-propan-1-ol-haltige Resorcinmikrokapseln,
- Beispiel I.15.: 2-Ethyl-3-phenylpropan-1-ol-haltige Resorcinmikrokapseln,
- Beispiel I.16.: 2-Ethyl-3-(3'-methylphenyl)-propan-1-ol-haltige Resorcinmikrokapseln,
- Beispiel I.17.: 3-(3'-Chlorphenyl)-2-ethylpropan-1-ol-haltige Resorcinmikrokapseln,
- Beispiel I.18.: 3-(2'-Chlorphenyl)-2-ethylpropan-1-ol-haltige Resorcinmikrokapseln,
- Beispiel I.19.: 3-(4'-Chlorphenyl)-2-ethylpropan-1-ol-haltige Resorcinmikrokapseln,
- Beispiel I.20.: 3-(3',4'-Dichlorphenyl)-2-ethylpropan-1-ol-haltige Resorcinmikrokapseln,
- Beispiel I.21.: 2-Ethyl-3-(2'-methylphenyl)-propan-1-ol-haltige Resorcinmikrokapseln,
- Beispiel I.22.: 2-Ethyl-3-(4'-methylphenyl)-propan-1-ol-haltige Resorcinmikrokapseln,
- Beispiel I.23.: 3-(3',4'-Dimethylphenyl)-2-ethylpropan-1-ol haltige Resorcinmikrokapseln,
- Beispiel I.24.: 2-Ethyl-3-(4'-methoxyphenyl)-propan-1-ol-haltige Resorcinmikrokapseln,
- Beispiel I.25.: 3-(3',4'-Dimethoxyphenyl)-2-ethylpropan-1-ol-haltige Resorcinmikrokapseln,
- Beispiel I.26.: 2-Allyl-3-phenylpropan-1-ol-haltige Resorcinmikrokapseln,
- Beispiel I.27.: 2-n-Pentyl-3-phenylpropan-1-ol-haltige Resorcinmikrokapseln.

In einer weiteren Beispielserie wurden Phloroglucin-Mikrokapseln hergestellt. Analog zum Verfahren gemäß Beispiel I.2. wurden die dort eingesetzten 5,5 g Resorcin vollständig durch 6,3 g Pholoroglucin ersetzt. Auf diese Weise erhielt man:
- Beispiel I.28.: Phenoxyethanol-haltige Phloroglucinmikrokapseln
- Beispiel I.29.: (3-Phenoxy-propan-2-ol)-haltige Phloroglucinmikrokapseln,
- Beispiel I.30.: Anisalkohol- haltige Phloroglucinmikrokapseln,
- Beispiel I.31.: 2-Methyl-5-phenyl-pentan-1-ol -haltige Phloroglucinmikrokapseln,
- Beispiel I.32.: 1,1-Dimethyl-3-phenyl-propan-1-ol-haltige Phloroglucinmikrokapseln,
- Beispiel I.33.: Benzylalkoho- haltige Phloroglucinmikrokapseln I,
- Beispiel I.34.: 2-Phenylethan-1-ol-haltige Phloroglucinmikrokapseln,
- Beispiel I.35.: 3-Phenylpropan-1-ol-haltige Phloroglucinmikrokapseln,
- Beispiel I.36.: 4-Phenylbutan-1-ol-haltige Phloroglucinmikrokapseln,
- Beispiel I.37:: 5-Phenylpentan-1-ol-haltige Phloroglucinmikrokapseln,
- Beispiel I.38.: 2-Benzylheptan-1-ol-haltige Phloroglucinmikrokapseln,
- Beispiel I.39.: 2,2-Dimethyl-3-phenylpropan-1-ol-haltige Phloroglucinmikrokapseln,
- Beispiel I.40.: 2,2-Dimethyl-3-(3'-methylphenyl)-propan-1-ol-haltige Phloroglucinmikrokapseln,
- Beispiel I.41.: 2-Ethyl-3-phenylpropan-1-ol-haltige Phloroglucinmikrokapseln,
- Beispiel I.42.: 2-Ethyl-3-(3'-methylphenyl)-propan-1-ol-haltige Phloroglucinmikrokapseln,
- Beispiel I.43.: 3-(3'-Chlorphenyl)-2-ethylpropan-1-ol-haltige Phloroglucinmikrokapseln,
- Beispiel I.44.: 3-(2'-Chlorphenyl)-2-ethylpropan-1-ol-haltige Phloroglucinmikrokapseln,
- Beispiel I.45.: 3-(4'-Chlorphenyl)-2-ethylpropan-1-ol-haltige Phloroglucinmikrokapseln,
- Beispiel I.46.: 3-(3',4'-Dichlorphenyl)-2-ethylpropan-1-ol-haltige Phloroglucinmikrokapseln,
- Beispiel I.47.: 2-Ethyl-3-(2'-methylphenyl)-propan-1-ol-haltige Phloroglucinmikrokapseln,
- Beispiel I.48.: 2-Ethyl-3-(4'-methylphenyl)-propan-1-ol-haltige Phloroglucinmikrokapseln,
- Beispiel I.49.: 3-(3',4'-Dimethylphenyl)-2-ethylpropan-1-ol haltige Phloroglucinmikrokapseln,
- Beispiel I.50.: 2-Ethyl-3-(4'-methoxyphenyl)-propan-1-ol-haltige Phloroglucinmikrokapseln,
- Beispiel I.51.: 3-(3',4'-Dimethoxyphenyl)-2-ethylpropan-1-ol-haltige Phloroglucinmikrokapseln,
- Beispiel I.52.: 2-Allyl-3-phenylpropan-1-ol-haltige Phloroglucinmikrokapseln,
- Beispiel I.53.: 2-n-Pentyl-3-phenylpropan-1-ol-haltige Phloroglucinmikrokapseln.

In den beiden Beispielserien I.3 bis I.27 (Resorcin) bzw. I.28 bis I.53 (Phloroglucin) kann bei der Synthese der Mikrokapseln 25,5 g Glutardialdehyd durch 21,9 g Succindialdehyd ersetzt werden. Entsprechende Resorcin- und Phloroglucin-Mikrokapseln, die auf Succialdehyd basieren, wurden in den Beispielserien I.54 bis I.79 (Resorcin und Glutardialdehyd) sowie I. 80 bis I.105 (Phloroglucin und Glutardialdehyd) hergestellt.

### II. Anwendungsbeispiele:

Alle Mengenangaben beziehen sich, soweit nicht anders vermerkt, auf Gew.-%.

**Tabelle 1: Antitranspirant-Wachsstifte**

| | 1.1 | 1.2 | 1.3 | 1.4 | 1.5 | 1.6 | 1.7 |
|---|---|---|---|---|---|---|---|
| | | | | | | | |
| PPG-14 Butylether | 15,00 | 18,00 | 12,00 | 19,00 | 6,00 | 10,50 | 10,50 |
| Hydrogenated Castor Oil | 1,00 | 1,50 | 2,00 | 1,50 | 1,80 | 3,00 | 3,00 |
| Stearyl alcohol | 20,00 | 18,00 | 15,00 | 18,00 | 17,00 | 23,50 | 23,50 |
| Ceteareth-30 | 3,00 | 2,00 | 4,00 | - | 3,00 | 3,00 | -- |
| Isoceteth-20 | - | - | - | 2,50 | - | -- | -- |
| Parfum | 1,00 | 1,20 | 0,80 | 1,50 | 1,00 | 1,50 | 1,50 |
| Aluminumchlorohydrate | 20,00 | 22,00 | 18,00 | - | 20,00 | -- | -- |
| Aluminum Zirconium tetrachlorohydrate Gly | -- | -- | -- | 22,00 | -- | 20,00 | -- |
| Aluminum Zirconium pentachlorohydrate Gly | -- | -- | -- | -- | -- | -- | 20,00 |
| Allantoin | 0,10 | -- | -- | 0,10 | -- | -- | -- |
| Cocoglycerides (FP 30 - 32 °C) | 4,00 | 6,00 | 3,00 | 5,00 | 6,00 | -- | --- |
| Myristylmyristat | - | - | - | - | - | 1,70 | 1,40 |
| Silica | - | - | - | - | - | 0,80 | -- |
| Silica Dimethyl Silylate | - | - | - | - | - | 1,00 | -- |
| Talc | 3,00 | 2,00 | 5,00 | 3,00 | 3,00 | - | -- |
| Mikrokapseln I.2. | 0,20 | -- | -- | 1,00 | -- | -- | -- |
| Mikrokapseln I.12 | -- | 1,50 | -- | -- | 0,50 | -- | 2,00 |
| Mikrokapseln I.30 | | | 0,75 | -- | | 2,00 | -- |
| Tocopherylacetat | 0,20 | -- | 0,50 | 0,10 | -- | -- | -- |
| Cyclopentasiloxan | ad 100 | ad 100 | ad 100 | ad 100 | ad 100 | ad 100 | ad 100 |

**Tabelle 2: Antitranspirant-Wachsstifte**

| | 2.1 | 2.2 | 2.3 | 2.4 | 2.5 |
|---|---|---|---|---|---|
| | | | | | |
| Hexyldecanol | 10,00 | 12,00 | 10,00 | 8,00 | 8,00 |
| PPG-14 Butylether | 6,00 | 5,00 | 6,00 | 8,00 | 8,00 |
| Hydrogenated Castor Oil | 4,00 | 5,00 | 6,00 | 5,00 | 5,00 |
| Stearyl alcohol | 12,00 | 14,00 | 11,00 | 16,00 | 16,00 |
| Cetyl alcohol | 6,00 | 5,00 | 6,00 | 3,00 | 3,00 |
| PEG-20 Glyceryl stearate | 5,00 | 4,00 | 6,00 | 4,00 | 4,00 |
| Ceteareth-30 | 3,00 | 1,00 | 3,00 | -- | -- |
| Parfum | 1,00 | 1,20 | 0,80 | 1,00 | 1,00 |
| Aluminumchlorohydrate | 20,00 | 20,00 | 18,00 | -- | -- |
| Aluminium Zirconium tetrachlorohydrate Glyc | -- | -- | -- | 23,00 | 23,00 |
| Talc | 8,00 | 5,00 | 8,00 | 7,00 | 7,00 |
| Mikrokapseln I.12. | 0,20 | -- | -- | -- | -- |
| Mikrokapseln I.3 | -- | 0,50 | -- | -- | -- |
| Mikrokapseln I.38 | -- | -- | 1,0 | -- | -- |
| Mikrokapseln I.4 | -- | -- | -- | 0,5 | -- |
| Mikrokapseln I.5 | -- | -- | -- | -- | 0,2 |
| Cyclopentasiloxane | ad 100 | ad 100 | ad 100 | ad 100 | ad 100 |

**Tabelle 3: Wasserfreie Antitranspirant-Wachsstifte ohne Cyclomethicone**

| | 3.1 | 3.2 | 3.3 | 3.4 | 3.5 | 3.6 |
|---|---|---|---|---|---|---|
| | | | | | | |
| Stearyl Alcohol | 16 | 18 | 18 | 18 | 18 | 18 |
| Behenyl Alcohol | -- | -- | 0,2 | -- | -- | -- |
| Ceteareth-30 | 3 | 3 | 3 | 3 | 3 | 3 |
| PPG-14 Butyl Ether | 15 | 5 | 10 | 15 | 10 | 7,5 |
| Dimethicone (2 cSt) | -- | -- | 18,4 | 23 | 24,3 | 24,3 |
| Dimethicone (5 cSt) | 33 | 44,3 | 17,4 | 11 | -- | -- |
| Mikrokapseln I.2. | 0,5 | -- | -- | -- | -- | -- |
| Mikrokapseln I.7 | -- | 0,2 | -- | -- | -- | -- |
| Mikrokapseln I.9 | -- | -- | 1 | -- | -- | -- |
| Mikrokapseln I.12. | -- | -- | -- | 0,5 | -- | -- |
| Mikrokapseln 1.15 | -- | -- | -- | -- | 0,2 | -- |
| Mikrokapseln I.38 | -- | - | -- | -- | -- | 0,7 |
| Hydrogenated Polydecene | -- | -- | -- | -- | 15 | 15 |
| Cocoglycerides (FP 30 - 32 °C) | 5 | 4 | 4 | 4 | 4 | 4 |
| Hydrogenated Castor Oil | 1,5 | 1,5 | 2 | 1,5 | 1,5 | 1,5 |
| Aluminum Chlorohydrate | 20 | 20 | 20 | 20 | 20 | 20 |
| Talc | 5 | 3 | 5 | 3 | 3 | 5 |
| Parfum | 1 | 1 | 1 | 1 | 1 | 1 |

**Fortsetzung Tabelle 3: Wasserfreie Antitranspirant-Wachsstifte ohne Cyclomethicone**

| | 3.7 | 3.8 | 3.9 | 3.10 | 3.11 | 3.12 |
|---|---|---|---|---|---|---|
| | | | | | | |
| Stearyl Alcohol | 16 | 18 | 18 | 18 | 18 | 18 |
| Behenyl Alcohol | -- | -- | 0,2 | -- | -- | -- |
| Ceteareth-30 | 3 | 3 | 3 | 3 | 3 | 3 |
| PPG-14 Butyl Ether | 15 | 5 | 10 | 15 | 10 | 7,5 |
| Dimethicone (2 cSt) | -- | -- | 18,4 | 23 | 24,3 | 24,3 |
| Dimethicone (5 cSt) | 33 | 44,3 | 17,4 | 11 | -- | -- |
| Mikrokapseln I.2. | 0,5 | -- | -- | -- | -- | -- |
| Mikrokapseln I.8 | -- | 0,2 | -- | -- | -- | -- |
| Mikrokapseln I.12 | -- | -- | 1 | -- | -- | -- |
| Mikrokapseln I.49 | -- | -- | -- | 0,5 | -- | -- |
| Mikrokapseln I.100 | -- | -- | -- | -- | 0,2 | -- |
| Mikrokapseln I.105 | -- | -- | -- | -- | -- | 0,7 |
| Hydrogenated Polydecene | -- | -- | -- | -- | 15 | 15 |
| Cocoglycerides (FP 30 - 32 °C) | 5 | 4 | 4 | 4 | 4 | 4 |
| Hydrogenated Castor Oil | 1,5 | 1,5 | 2 | 1,5 | 1,5 | 1,5 |
| Aluminum Chlorohydrate | 20 | 20 | 20 | 20 | 20 | 20 |
| Talc | 5 | 3 | 5 | 3 | 3 | 5 |
| Parfum | 1 | 1 | 1 | 1 | 1 | 1 |

**Tabelle 4: Wasserfreie Antitranspirant-Wachsstifte mit zwei Phasen**

| **Erste (äußere) Phase** | **4.1** | **4.2** | **4.3** |
|---|---|---|---|
| C₁₂₋₁₅ Alkyl Benzoate | 9.15 | 28.60 | 31.30 |
| Isopropyl myristate | - | - | 15.62 |
| Phenyl trimethicone (DC 556) | -- | - | 2.60 |
| 12-Hydroxystearinsäure | 12.00 | 12.00 | 6.00 |
| N-lauroyl glutamic acid di-n-butylamide | 2.50 | 2.00 | 1.35 |
| C20-40 Pareth-10³ | 3.00 | 3.00 | 3.00 |
| Aktiviertes Al-Zr Chlorohydrate Gly; 17.5 Gew.-% USP Aktivsubstanz | 33.50 | 33.50 | 33.50 |
| Parfum | 3.85 | 2.50 | 4.03 |
| Parfum (verkapselt) | - | 1.50 | - |
| Cyclomethicone (DC 245) | 36.00 | 17.20 | 2.60 |

| **Zweite Phase (innerer Streifen oder Zylinder)** | **4.4** | **4.5** | **4.6** |
|---|---|---|---|
| C₁₂₋₁₅ Alkyl Benzoate | 46.55 | 51.02 | 51.78 |
| Isopropyl myristate | - | - | 25.85 |
| Phenyl trimethicone (DC 556) | - | - | 4.30 |
| 12-Hydroxystearinsäure | 15.00 | 15.44 | 10.00 |
| N-lauroyl glutamic acid di-n-butylamide | 1.50 | 1.50 | 2.00 |
| Farbstoff | 0.10 | 0.10 | 0.07 |
| Parfum | 0.85 | 1.24 | 1.70 |
| Mikrokapseln I.12. | 2.00 | 1.00 | - |
| Mikrokapseln I.27 | - | - | 1.50 |
| Cyclomethicone (DC 245) | 34.00 | 29.70 | 2.80 |

| **Zweite Phase (innerer Streifen oder Zylinder)** | **4.7** | **4.8** | **4.9** |
|---|---|---|---|
| C₁₂₋₁₅ Alkyl Benzoate | 46.55 | 51.02 | 51.78 |
| Isopropyl myristate | - | - | 25.85 |
| Phenyl trimethicone (DC 556) | - | - | 4.30 |
| 12-Hydroxystearinsäure | 15.00 | 15.44 | 10.00 |
| N-lauroyl glutamic acid di-n-butylamide | 1.50 | 1.50 | 2.00 |
| Farbstoff | 0.10 | 0.10 | 0.07 |
| Parfum | 0.85 | 1.24 | 1.70 |
| Mikrokapseln I.2. | 2.00 | 1.00 | -- |
| Mikrokapseln I.38 | - | - | 1.50 |
| Cyclomethicone (DC 245) | 34.00 | 29.70 | 2.80 |

**Tabelle 5: Antiperspirant-Creme wasserfrei (Soft Solid)**

| | 5.1 | 5.2 | 5.3 | 5.4 |
|---|---|---|---|---|
| | | | | |
| Aluminum Chlorohydrate | 20,00 | 22,00 | 20,00 | |
| Aluminium Zirconium tetrachlorohydrate Glyc | | | | 24,00 |
| Hexyldecanol | 5,00 | 4,50 | 5,50 | 6,00 |
| Dicaprylylether | 3,00 | 4,00 | 3,50 | 5,00 |
| Cocoglycerides | 5,00 | 6,00 | 7,00 | 3,00 |
| C18-C36 Triglyceride | 6,00 | 5,00 | 4,00 | 3,00 |
| Ceteareth-30 | 3,00 | 2,00 | 2,50 | 4,00 |
| PEG-20 Glycerylstearate | 5,00 | 6,00 | 3,00 | 2,00 |
| Cellulose | 3,00 | 2,00 | 5,00 | 1,00 |
| Aluminum-Starch-Octenylsuccinate | 5,00 | 4,00 | 6,00 | 5,00 |
| Silica | 1,00 | 2,00 | 0,50 | |
| Talc | 10,00 | 5,00 | 7,00 | 12,00 |
| Allantoin | 0,10 | 0,10 | | |
| Parfum | 1,00 | 1,50 | 2,00 | 0,80 |
| Mikrokapseln I.2. | 0,5 | -- | -- | -- |
| Mikrokapseln I.17 | -- | 0,2 | -- | -- |
| Mikrokapseln I.27 | -- | -- | 1 | -- |
| Mikrokapseln I.38 | -- | -- | -- | 0,5 |
| Cyclopentasiloxan | ad 100 | ad 100 | ad 100 | ad 100 |

**Tabelle 6: Puder (Deodorant / Antitranspirant)**

| 6 | 6.1 | 6.2 | 6.3 | 6.4 |
|---|---|---|---|---|
| | | | | |
| Aluminum Chlorohydrate | 20,00 | -- | -- | -- |
| Aluminium Zirconium tetrachlorohydrate Glyc | -- | -- | -- | 24,00 |
| Silica | 2,00 | 2,00 | 1,00 | 1,00 |
| Triclosan | -- | 0,30 | 0,10 | -- |
| Sensiva SC 50 | -- | -- | 1,00 | -- |
| Parfum | 1,00 | 0,50 | 2,00 | 1,00 |
| Parfum (verkapselt) | - | 1,00 | -- | 1,00 |
| Menthyllactat (verkapselt) | - | - | 1,00 | 1,00 |
| Mikrokapseln I.12. | 0,5 | -- | -- | -- |
| Mikrokapseln I.58 | -- | 0,3 | -- | -- |
| Mikrokapseln I.60 | -- | -- | 1,0 | -- |
| Mikrokapseln I.62. | -- | -- | -- | 0,3 |
| Talc | ad 100 | ad 100 | ad 100 | ad 100 |

Die Puderbeispiele 6.1 - 6.3 können sowohl als loser Puder als auch als gepresster Puder konfektioniert sein.

**Tabelle 7: Dibenzylidensorbitol-basierte Antitranspirant-Gelstifte**

| | 7.1 | 7.2 | 7.3 | 7.4 (Deodorant) |
|---|---|---|---|---|
| 1,2-Propylenglycol | 85 | 84,4 | 83,85 | 91,2 |
| Al/Zr tetrachloro-hydrate-gly | 11 | 11 | 11 | 3 |
| Dibenzylidensorbitol | 1 | 1,3 | 1,1 | 1,3 |
| Hydroxypropyl-cellulose | 0,3 | 0,3 | 0,35 | 0,5 |
| Na₄EDTA | 0,2 | 0,2 | 0,2 | 0,2 |
| Diisopropylsebacat | -- | 1 | 1 | -- |
| Glycereth-7-diisononanoat | 0,5 | -- | -- | -- |
| PEG/PPG-14/4 Dimethicone (Abil B 8851) | 0,25 | 0,25 | 0,25 | -- |
| Oleth-10 | -- | -- | -- | 0,75 |
| PPG-10-butandiol | -- | -- | -- | 0,75 |
| PPG-3-myristylether | -- | -- | -- | 0,75 |
| Mikrokapseln I.2. | 0,5 | -- | -- | -- |
| Mikrokapseln I.12 | -- | 0,3 | -- | -- |
| Mikrokapseln I.66 | -- | -- | 1,0 | -- |
| Mikrokapseln I.77 | -- | -- | -- | 0,3 |
| Parfüm | 1,25 | 1,25 | 1,25 | 1,25 |

**Tabelle 8: Wasserfreie Roll-on-Zusammensetzungen**

| | 8.1 | 8.2 | 8.3 | 8.4 | 8.5 |
|---|---|---|---|---|---|
| Quaternium-18 hectorite | 4,0 | 2,5 | 2,5 | 3,2 | 3,6 |
| Parfum | 0,5 | 0,5 | 0,5 | 1,0 | 1,0 |
| Parfum (verkapselt) | 0,5 | 0,5 | 0,5 | 1,0 | 1,0 |
| Al/Zr tetrachlorohydrate-gly (aktiviert) | 20 | 20 | 20 | 20 | 20 |
| Mikrokapseln I.2. | - | - | - | - | 2,0 |
| Mikrokapseln I.90 | 0,5 | -- | -- | -- | -- |
| Mikrokapseln I.91 | -- | 0,3 | -- | -- | -- |
| Mikrokapseln I.92 | -- | -- | 1,0 | -- | -- |
| Mikrokapseln I.38 | -- | -- | -- | 1,3 | -- |
| Propylencarbonat | 1,0 | 1,0 | - | - | 1,0 |
| Dimethicone (50 cSt) | - | - | 5,0 | 3,0 | |
| Aerosil 300 (Fumed Silica) | - | 0,2 | 0,4 | - | - |
| Cyclomethicone | ad 100 | ad 100 | ad 100 | ad 100 | ad 100 |

**Tabelle 9: Wasserfreie Roll-on-Zusammensetzungen**

| | 9.1 | 9.2 | 9.3 | 9.4 | 9.5 |
|---|---|---|---|---|---|
| Quaternium-18 hectorite | 4,0 | 2,5 | 2,5 | 3,2 | 3,6 |
| Parfum | 0,5 | 0,5 | 0,5 | 1,0 | 1,0 |
| Parfum (verkapselt) | 0,5 | 0,5 | 0,5 | 1,0 | 1,0 |
| Al/Zr pentachlorohydrategly (aktiviert) | 20 | 20 | 20 | 20 | 20 |
| Mikrokapseln I.12. | 0,5 | 1,0 | 1,5 | 1,7 | 2,0 |
| Propylencarbonat | 1,0 | 1,0 | - | - | 1,0 |
| Dimethicone (50 cSt) | - | - | 5,0 | 3,0 | |
| Aerosil 300 (Fumed Silica) | - | 0,2 | 0,4 | - | - |
| Cyclomethicone | ad 100 | ad 100 | ad 100 | ad 100 | ad 100 |

**Tabelle 10: Wasserfreie Roll-on-Zusammensetzungen mit Siliconelastomer**

| | 10.1 | 10.2 | 10.3 | 10.4 | 10.5 |
|---|---|---|---|---|---|
| Dow Corning 9040 (90 Gew.-% Cyclomethicone, 10 Gew.-% Dimethicone Crosspolymer) | 5,0 | 20,0 | 5,0 | 10,0 | 10,0 |
| Abil EM 90 (CETYL PEG/PPG-10/1 DIMETHICONE) | -- | -- | 1,0 | -- | -- |
| Mikrokapseln I.2. | - | - | - | 1,3 | - |
| Mikrokapseln I.55 | 0,5 | -- | -- | -- | - |
| Mikrokapseln I.30 | -- | 0,3 | -- | -- | - |
| Mikrokapseln I.72. | -- | -- | 1,0 | -- | - |
| Mikrokapseln I.38 | -- | -- | -- | -- | 2,0 |
| Cyclomethicone | ad 100 | ad 100 | ad 100 | ad 100 | ad 100 |

**Tabelle 11: erfindungsgemäße Antitranspirant-Emulsionen (O/W)**

| **INGREDIENTS (EU-INCI)** | **A** | **B** | **C** |
|---|---|---|---|
| ALUMINUM CHLOROHYDRATE | 13,0 | 13,0 | 13,0 |
| TALC | 1,0 | -- | -- |
| BENTONITE | -- | 1,0 | -- |
| HECTORITE | -- | -- | 5,0 |
| STEARETH-2 | 2,5 | 2,5 | 2,5 |
| STEARETH-21 | 1,5 | 1,5 | 1,5 |
| PARFUM | 1,1 | 1,1 | 1,1 |
| PPG-15 STEARYL ETHER | 0,5 | 0,5 | 0,5 |
| BISABOLOL | 0,1 | 0,1 | 0,1 |
| ALUMINUM STARCH OCTENYLSUCCINATE | 0,1 | 0,1 | 0,1 |
| BIS-PEG-18 METHYL ETHER DIMETHYL SILANE | 0,1 | 0,1 | 0,1 |
| TOCOPHERYL ACETATE | 0,1 | 0,1 | 0,1 |
| Mikrokapseln I.12. | 1,5 | 1,5 | 1,5 |
| AQUA | ad 100 | ad 100 | ad 100 |

Die Emulsion gemäß Beispiel 11 wies am ersten Tag nach der Herstellung eine Viskosität von 1800 mPas auf, gemessen mit einem Brookfield-Viskosimeter, Spindel RV 4, 20 s-1, ohne Helipath, bei 20 °C Umgebungstemperatur und 20 °C Probentemperatur.

**Beispiel 12: erfindungsgemäße Antitranspirant-Emulsion (O/W)**

| **INGREDIENTS (EU-INCI)** | **A** | **B** | **C** |
|---|---|---|---|
| ALUMINUM CHLOROHYDRATE | 26,0 | 26,0 | 26,0 |
| TALC | 1,0 | -- | -- |
| BENTONITE | -- | 1,0 | -- |
| HECTORITE | -- | -- | 2,0 |
| STEARETH-2 | 2,4 | 2,4 | 2,4 |
| STEARETH-21 | 1,5 | 1,5 | 1,5 |
| PARFUM | 1,0 | 1,0 | 1,0 |
| PPG-15 STEARYL ETHER | 0,5 | 0,5 | 0,5 |
| ALLANTOIN | 0,1 | 0,1 | 0,1 |
| ALUMINUM STARCH OCTENYLSUCCINATE | 0,1 | 0,1 | 0,1 |
| ALOE BARBADENSIS | 0,1 | 0,1 | 0,1 |
| Mikrokapseln I.12. | 1,0 | 1,0 | 1,0 |
| AQUA | ad 100 | ad 100 | ad 100 |

Die Emulsion gemäß Beispiel 12 wies am ersten Tag nach der Herstellung eine Viskosität von 2000 mPas auf, gemessen mit einem Brookfield-Viskosimeter, Spindel RV 4, 20 s-1, ohne Helipath, bei 20 °C Umgebungstemperatur und 20 °C Probentemperatur.

**Beispiel 13: erfindungsgemäße Antitranspirant-Emulsion (O/W)**

| **INGREDIENTS (EU)** | **A** | **B** | **C** |
|---|---|---|---|
| ALUMINUM ZIRCONIUM TETRACHLOROHYDREX GLY | 23,7 | 23,7 | 23,7 |
| TALC | 2,0 | -- | -- |
| BENTONITE | -- | 0,5 | -- |
| HECTORITE | -- | -- | 3,0 |
| STEARETH-2 | 2,4 | 2,4 | 2,4 |
| STEARETH-21 | 1,6 | 1,6 | 1,6 |
| PARFUM | 1,2 | 1,2 | 1,2 |
| PPG-15 STEARYL ETHER | 0,5 | 0,5 | 0,5 |
| ALUMINUM STARCH OCTENYLSUCCINATE | 0,1 | 0,1 | 0,1 |
| TOCOPHERYL ACETATE | 0,5 | 0,5 | 0,5 |
| Mikrokapseln I.38. | 2,0 | 2,0 | 2,0 |
| AQUA | ad 100 | ad 100 | ad 100 |

Die Emulsion gemäß Beispiel 13 wies am ersten Tag nach der Herstellung eine Viskosität von 2200 mPas auf, gemessen mit einem Brookfield-Viskosimeter, Spindel RV 4, 20 s-1, ohne Helipath, bei 20 °C Umgebungstemperatur und 20 °C Probentemperatur.

**Beispiel 14: erfindungsgemäße Antitranspirant-Emulsion (O/W)**

| **INGREDIENTS (EU)** | **A** | **B** | **C** |
|---|---|---|---|
| ALUMINUM CHLOROHYDRATE | 20,0 | 20,0 | 20,0 |
| TALC | 0,5 | -- | -- |
| BENTONITE | -- | 1,5 | -- |
| HECTORITE | -- | -- | 1,0 |
| STEARETH-2 | 2,3 | 2,3 | 2,3 |
| STEARETH-21 | 1,5 | 1,5 | 1,5 |
| PARFUM | 1,0 | 1,0 | 1,0 |
| PPG-15 STEARYL ETHER | 0,5 | 0,5 | 0,5 |
| ALUMINUM STARCH OCTENYLSUCCINATE | 0,1 | 0,1 | 0,1 |
| ALLANTOIN | 0,1 | 0,1 | 0,1 |
| ISOPROPYL MYRISTATE | 0,1 | 0,1 | 0,1 |
| Mikrokapseln I.38. | 2,0 | 2,0 | 2,0 |
| AQUA | ad 100 | ad 100 | ad 100 |

Die Emulsion gemäß Beispiel 14 wies am ersten Tag nach der Herstellung eine Viskosität von 1700 mPas auf, gemessen mit einem Brookfield-Viskosimeter, Spindel RV 4, 20 s-1, ohne Helipath, bei 20 °C Umgebungstemperatur und 20 °C Probentemperatur.

**Beispiel 15: erfindungsgemäße Antitranspirant-Emulsion (O/W)**

| **INGREDIENTS (EU)** | **A** | **B** | **C** |
|---|---|---|---|
| ALUMINUM CHLOROHYDRATE | 20,0 | 20,0 | 20,0 |
| TALC | 1,0 | -- | -- |
| BENTONITE | -- | 1,0 | -- |
| HECTORITE | -- | -- | 5,0 |
| STEARETH-2 | 2,3 | 2,3 | 2,3 |
| STEARETH-21 | 1,5 | 1,5 | 1,5 |
| PARFUM | 1,0 | 1,0 | 1,0 |
| PPG-15 STEARYL ETHER | 0,5 | 0,5 | 0,5 |
| DISTARCH PHOSPHATE | 0,1 | 0,1 | 0,1 |
| ALLANTOIN | 0,1 | 0,1 | 0,1 |
| ISOPROPYL MYRISTATE | 0,1 | 0,1 | 0,1 |
| Mikrokapseln I.12. | 2,0 | 2,0 | 2,0 |
| AQUA | ad 100 | ad 100 | ad 100 |

Die Emulsion gemäß Beispiel 15 wies am ersten Tag nach der Herstellung eine Viskosität von 1800 mPas auf, gemessen mit einem Brookfield-Viskosimeter, Spindel RV 4, 20 s-1, ohne Helipath, bei 20 °C Umgebungstemperatur und 20 °C Probentemperatur. Die Emulsionen gemäß den Beispielen 11 - 15 wurden jeweils in eine Flasche mit einem Rollapplikator eingefüllt und waren so verkaufsfertig.

**Tabelle 16: Suspensionen zum Versprühen als Antitranspirant-Spray**

| | | | 16.1 | 16.2 | 16.3 | 16.4 | 16.5 | 16.6 |
|---|---|---|---|---|---|---|---|---|
| | | | | | | | | |
| Parfum | | | 5,00 | 5,00 | 5,00 | 5,00 | 5,00 | 5,00 |
| Aluminumchlorohydrat (aktiviert) | | | 30,00 | 35,00 | 35,00 | 35,00 | 28,00 | 32,00 |
| Mikrokapseln I.12. | | | 2,00 | 2,00 | 2,00 | 2,00 | 2,00 | 2,00 |
| Isopropylpalmitat | | | 5,00 | 5,00 | - | - | - | 6,00 |
| C12-15-Alkylbenzoat | | | -- | -- | 5,00 | 5,00 | -- | -- |
| Cosmacol PLG | | | - | - | - | - | 5,00 | -- |
| Disteardimonium Hectorite | | | 4,50 | 3,90 | 4,00 | 3,80 | 5,00 | 4,50 |
| Propylencarbonat | | | 1,50 | 1,30 | 1,30 | 1,30 | 1,70 | 1,50 |
| Ethylhexylpalmitat | | | ad 100 | ad 100 | ad 100 | ad 100 | ad 100 | ad 100 |

| | 16.7 | 16.8 | 16.9 | 16.10 | 16.11 | 16.12 | 16.12a | 16.13 |
|---|---|---|---|---|---|---|---|---|
| | | | | | | | | |
| Parfum | 5,00 | 5,00 | 5,00 | 5,00 | 5,00 | 5,00 | 5,00 | 5,00 |
| Aluminumchlorohydrat (aktiviert) | 32,00 | 28,00 | 30,00 | 35,00 | 32,00 | 32,00 | 32,00 | 28,00 |
| Mikrokapseln I.12. | 2,00 | 2,00 | 2,00 | 2,00 | 2,00 | 2,00 | 2,00 | 2,00 |
| Isopropylmyristat | - | - | - | - | - | - | - | 5,00 |
| Isopropylpalmitat | 5,00 | 5,00 | - | - | - | - | - | -- |
| C12-15-Alkylbenzoat | -- | -- | 5,00 | 5,00 | -- | -- | -- | -- |
| Cosmacol PLG | - | - | - | - | 5,00 | 5,00 | 5,00 | - |
| Disteardimonium Hectorite | 4,50 | 3,90 | 4,00 | 3,80 | 5,00 | 4,50 | - | 3,90 |
| Propylencarbonat | 1,50 | 1,30 | 1,30 | 1,30 | 1,70 | 1,50 | - | 1,30 |
| Silica | - | - | - | - | - | 0,80 | 0,80 | - |
| Silica Dimethyl Silylate | - | - | - | - | - | 1,00 | 1,00 | - |
| Tocopherylacetat | 0,20 | -- | 0,50 | 0,10 | -- | -- | -- | -- |
| Cyclopentasiloxan | ad 100 | ad 100 | ad 100 | ad 100 | ad 100 | ad 100 | ad 100 | ad 100 |

| Cosmacol PLG (INCI: DI-C12-13 ALKYL TARTRATE, TRI-C12-13 ALKYL CITRATE, SILICA) | | | | | | | | |
|---|---|---|---|---|---|---|---|---|
| | | 16.14 | 16.15 | 16.16 | 16.16a | 16.17 | 16.18 | 16.19 |
| | | | | | | | | |
| Parfum | | 5,00 | 5,00 | 5,00 | 5,00 | 5,00 | 5,00 | 5,00 |
| Aluminumchlorohydrat (aktiviert) | | 32,00 | 28,00 | 30,00 | 30,00 | 35,00 | 32,00 | 32,00 |
| Verkapseltes 2-Benzylheptan-1-ol | | 0,90 | 1,00 | 2,00 | 2,00 | 2,00 | -- | -- |
| Mikrokapseln I.2. | | 1,50 | 1,50 | 1,50 | 1,50 | 1,50 | 1,50 | 1,50 |
| Disteardimonium Hectorite | | 4,50 | 3,90 | 4,00 | - | 3,80 | 5,00 | 4,50 |
| Propylencarbonat | | 1,50 | 1,30 | 1,30 | - | 1,30 | 1,70 | 1,50 |
| Silica | | - | - | 0,80 | 0,80 | - | - | -- |
| Silica Dimethyl Silylate | | - | - | 1,00 | 1,00 | - | - | -- |
| Tocopherylacetat | | 0,20 | -- | 0,50 | 0,50 | 0,10 | -- | -- |
| 2-Ethylhexylpalmitat | | ad 100 | ad 100 | ad 100 | ad 100 | ad 100 | ad 100 | ad 100 |

| | | 16.20 | 16.21 | 16.22 | 16.23 | 16.24 | 16.25 | 16.25 |
|---|---|---|---|---|---|---|---|---|
| | | | | | | | | |
| Parfum | | 5,00 | 5,00 | 5,00 | 5,00 | 5,00 | 5,00 | 5,00 |
| Aluminumchlorohydrat (aktiviert) | | 30,00 | 35,00 | 35,00 | 35,00 | 28,00 | 32,00 | 32,00 |
| Mikrokapseln I.2. | | 1,50 | 1,50 | 1,50 | 1,50 | 1,50 | 1,50 | 1,50 |
| Isopropylpalmitat | | 5,00 | 5,00 | - | - | - | 6,00 | 6,00 |
| C12-15-Alkylbenzoat | | -- | -- | 5,00 | 5,00 | -- | -- | -- |
| Cosmacol PLG | | - | - | - | - | 5,00 | -- | -- |
| Disteardimonium Hectorite | | 4,50 | 3,90 | 4,00 | 3,80 | 5,00 | 4,50 | 4,50 |
| Propylencarbonat | | 1,50 | 1,30 | 1,30 | 1,30 | 1,70 | 1,50 | 1,50 |
| Cyclopentasiloxan | | ad 100 | ad 100 | ad 100 | ad 100 | ad 100 | ad 100 | ad 100 |

| | | 16.26 | 16.27 | 16.28 | 16.29 | 16.30 | 16.31 | 16.32 |
|---|---|---|---|---|---|---|---|---|
| | | | | | | | | |
| Parfum | | 6,00 | 7,00 | 6,00 | 6,00 | 6,50 | 7,00 | 5,50 |
| Aluminumchlorohydrat (aktiviert) | | 30,00 | 35,00 | 35,00 | 35,00 | 28,00 | 32,00 | 32,00 |
| Mikrokapseln I.38. | | 1,50 | 1,50 | 1,50 | 1,50 | 1,50 | 1,50 | 1,50 |
| Isopropylpalmitat | | 5,00 | 5,00 | 7,00 | 7,00 | 7,00 | 6,00 | 6,00 |
| La Toja Salz | | -- | -- | -- | 0,05 | 0,1 | 0,05 | 0,5 |
| Parfumkapseln (INCI: Parfum (Fragrance), Sodium Starch Octenylsuccinate, Mannitol, Silica | | 1,5 | 2,0 | - | 2,0 | -- | -- | 2,0 |
| Talkum | | 0,5 | 1,0 | -- | -- | -- | -- | -- |
| Bentonit | | -- | -- | 0,5 | 1,0 | -- | -- | -- |
| Hectorit | | -- | -- | -- | -- | 0,5 | 1,0 | - |
| Disteardimonium Hectorite | | 4,50 | 6,00 | 4,50 | 5,00 | 5,50 | 6,00 | 6,00 |
| Propylencarbonat | | 1,50 | 1,30 | 1,00 | 0,90 | 1,50 | 1,30 | 1,20 |
| Cyclopentasiloxan | | ad 100 | ad 100 | ad 100 | ad 100 | ad 100 | ad 100 | ad 100 |

| | | 16.33 | 16.34 | 16.35 | 16.36 | 16.37 | 16.38 | 16.39 |
|---|---|---|---|---|---|---|---|---|
| | | | | | | | | |
| Parfum | | 6,00 | 7,00 | 6,00 | 6,00 | 6,50 | 7,00 | 5,50 |
| Aluminumchlorohydrat (aktiviert) | | 30,00 | 35,00 | 35,00 | 35,00 | 28,00 | 32,00 | 32,00 |
| Mikrokapseln I.12. | | 1,50 | 1,50 | 1,50 | 1,50 | 1,50 | 1,50 | 1,50 |
| Isopropylpalmitat | | 5,00 | 5,00 | 7,00 | 7,00 | 7,00 | 6,00 | 6,00 |
| La Toja Salz | | -- | -- | -- | 0,5 | 0,1 | 0,5 | 0,5 |
| Deodorantkapseln (INCI: Sodium Starch Octenyl Succinate, 2-Benzylheptanol, Phenoxyethanol, Mannitol, Silica | | 1,5 | 2,0 | 1,5 | 2,0 | -- | -- | -- |
| Talkum | | 5,0 | 1,0 | -- | -- | -- | -- | -- |
| Bentonit | | -- | -- | 5,0 | 1,0 | -- | -- | -- |
| Hectorit | | -- | -- | -- | -- | 5,0 | 1,0 | -- |
| Disteardimonium Hectorite | | 4,50 | 6,00 | 4,50 | 5,00 | 5,50 | 6,00 | 6,00 |
| Propylencarbonat | | 1,50 | 1,30 | 1,00 | 0,90 | 1,50 | 1,30 | 1,20 |
| Cyclopentasiloxan | | ad 100 | ad 100 | ad 100 | ad 100 | ad 100 | ad 100 | ad 100 |

Die Zusammensetzungen 16.1 bis 16.39 wurden in Spraydosen aus innen lackiertem Aluminium abgefüllt und mit einer Isobutan/Butan/Propan-Treibmittel-Mischung im Gewichtsverhältnis Suspension : Treibmittel von 25 : 75, 22 : 78, 20 : 80, 18 : 82, 15 : 85 und 13 : 87 beaufschlagt; die erfindungsgemäßen Zusammensetzungen 16.12a und 16.16a wurden mit einer Butan/1,1-Difluorethan-Treibmittel-Mischung im Gewichtsverhältnis Suspension : Treibmittel von 45:55, 40:60 und 30:70 beaufschlagt.

**Beispiel 17: Erfindungsgemäße Duschgele**

| | 17.1 | 17.2 | 17.3 | 17.4 | 17.5 |
|---|---|---|---|---|---|
| | Gew.-% telquel | Gew.-% telquel | Gew.-% telquel | Gew.-% telquel | Gew.-% telquel |
| Texapon®¹ N 70 | 12 | 10 | 8 | 10 | 14 |
| Dehyton®² PS | 9 | - | 4 | 10 | 2 |
| Rewoteric®³ AMC | - | 7 | 6 | - | - |
| Plantacare®⁴ 818 | 2 | 1,5 | - | - | 5 |
| Conditioner®⁵ P7 | 1 | 0,5 | - | 2 | - |
| Panthenol | 0,2 | - | 0,1 | - | - |
| Euperlan®⁶ PK 810 | 0,5 | - | - | 2 | 2 |
| Acusol®⁷ OP 301 | 2 | - | 3 | 0,5 | - |
| Mikrokapseln I.5 | 1,5, davon 0,7 Gew.-% Parfüm | - | - | - | - |
| Mikrokapseln I.10 | - | 1,5, davon 0,7 Gew.-% Parfüm | - | - | - |
| Mikrokapseln I.11 | - | - | 1,5, davon 0,5 Gew.-% Parfüm und 0,2 Gew.-% Menthol | - | - |
| Mikrokapseln I.48 | - | - | - | 2,0, davon 1,0 Gew.-% Parfüm | - |
| Mikrokapseln I.50 | - | - | - | - | 2,5, davon 1,3 Gew.-% Parfüm |
| Aloe Vera Extrakt | - | - | - | - | 0,1 |
| Tocopherylacetat | - | - | 0,2 | - | - |
| Joghurt Protein | 0,5 | 0,2 | 0,1 | 0,3 | 0,4 |
| Wasser | ad 100 | ad 100 | ad 100 | ad 100 | ad 100 |

| | | | | | |
|---|---|---|---|---|---|
| ¹ Natriumlaurylethersulfat, 2 EO (INCI: SODIUM LAURETH SULFATE); Aktivsubstanz: 68 Gew.-%; BASF ² Kokosfettsäureamidoethyl-N-2-hydroxyethylglycin-Natriumsalz (INCI: DISODIUM COCOAMPHODIACETATE); Aktivsubstanz: 37,5 Gew.-%; BASF ³ Hydroxyethyl-N-kokosalkylamidoethyl-carboxymethylglycinat-Natriumsalz (INCI: SODIUM COCOAMPHOACETATE); Aktivsubstanz: 30,5 - 32 Gew.-%; Evonik ⁴ C₈-C₁₄-Alkylpolyglucosid (INCI: COCO-GLUCOSIDE); Aktivsubstanz: 51 - 53 Gew.-%; BASF ⁵ Acrylamid Dimethyldiallylammoniumchlorid Copolymer (INCI: POLYQUATERNIUM-7); 3V Sigma ⁶ Perlglanzmittel (INCI: AQUA; GLYCOL DISTEARATE; SODIUM LAURETH SULFATE; COCAMIDE MEA; LAURETH-10; FORMIC ACID); BASF ⁷ Styrol Acryl Copolymer (INCI: STYRENE/ACRYLATES COPOLYMER); Aktivsubstanz: 39 - 41 Gew.-%; Rohm & Haas | | | | | |

**Beispiel 18: Erfindungsgemäße Haarconditioner (Angaben in Gew.-%**

| | 18.1 | 18.2 | 18.3 |
|---|---|---|---|
| Cetearylalkohol | 3,00 | 3,00 | 6,00 |
| Quaternium-91 | 1,40 | 1,40 | 4,50 |
| Panthenol | 0,20 | 0,20 | 0,50 |
| Polyurethan-32 | 0,50 | 0,50 | 1,00 |
| Silicone Quaternium-22 | -- | 1,00 | -- |
| Isopropylpalmitat | -- | -- | -- |
| Coenzym Q-10 | -- | -- | -- |
| Methylparaben | 0,20 | 0,20 | 0,20 |
| Parfüm | 0,30 | 0,30 | 0,30 |
| Phenoxyethanol | 0,40 | 0,40 | 0,30 |
| Mikrokapseln I.9 | 1,5, davon 0,7 Gew.-% Parfüm | | |
| Mikrokapseln I.13 | - | 2,0, davon 1,0 Gew.-% Parfüm | |
| Mikrokapseln I.39 | - | | 2,0, davon 1,0 Gew.-% Parfüm |
| Wasser | ad 100 | ad 100 | ad 100 |

**Beispiel 19: Erfindungsgemäße Haarshampoos (Angaben in Gew.-%**

| | 19.1 | 19.2 |
|---|---|---|
| Texapon®¹ N70 | 15 | 15 |
| Dehyton®² K | 10 | - |
| Dehyton®³ G | - | 15 |
| Plantacare®⁴ 818 UP | 4 | - |
| Dehyquart®⁵ A CA | - | 0,5 |
| D-Panthenol | - | 0,2 |
| Nicotinsäureamid | 0,3 | - |
| Cutina®⁶HR | 0,4 | 0,6 |
| Cetiol®⁷ HE | 1 | 0,8 |
| Polymer JR®⁸ 400 | 0,3 | 0,5 |
| Polyquaternium-7 | 0,1 | - |
| Guar Hydroxypropyltrimonium Chloride | - | 0,1 |
| Mikrokapseln I.15 | 1,5, davon 0,7 Gew.-% Parfüm | - |
| Mikrokapseln I.40 | - | 2,0, davon 1,0 Gew.-% Parfüm |
| Dow Coming®⁹ 200 | 0,1 | 0,1 |
| Aprikosenkernöl | 0,1 | - |
| Citronensäure | 0,5 | 0,6 |
| NaCl | 1,3 | 1 |
| Zink Pyrithion | - | 0,5 |
| Parfum (unverkapselt) | 1,0 | 1,0 |
| Konservierungsmittel | 0,5 | 0,5 |
| Wasser | ad 100 | ad 100 |

| | | |
|---|---|---|
| In den zuvor genannten Haarshampoos wurden die folgenden Handelsprodukte eingesetzt: 1 INCI-Bezeichnung: Sodium Laureth Sulfate; AS 68-73%; Cognis, 2 INCI-Bezeichnung: Cocamidopropyl Betaine; AS 29-32%; Cognis, 3 INCI-Bezeichnung: Disodium Cocoamphodiacetate; AS 30%; Cognis, 4 INCI-Bezeichnung: Coco Glucoside; AS 51-53%; Cognis, 5 INCI-Bezeichnung: Aqua, Cetrimonium Chloride, AS 24-26%; Cognis, 6 INCI-Bezeichnung: Hydrogenated Castor Oil; Cognis 7 INCI-Bezeichnung: PEG-7 Glyceryl Cocoate; Cognis 8 INCI-Bezeichnung: Polyquaternium-10; Dow 9 INCI-Bezeichnung: Dimethicone (30,000 cst); Dow Corning | | |

## Patentansprüche

1. Kosmetische Zusammensetzung, enthaltend - bezogen auf ihr Gewicht -
a) Mikrokapseln, deren Kapselwände ein Harz umfassen, welches durch Umsetzung
i) mindestens eines aromatischen Alkohols oder dessen Ether oder Ester und
ii) mindestens einer aldehydischen Komponente, die mindestens zwei C-Atome pro Molekül aufweist, und
iii) in Gegenwart mindestens eines Homo- oder Copolymers von 2-Acrylamido-2-methyl-propansulfonsäure oder dessen Salzen,
erhältlich ist
b) 0,01 bis 70 Gew.-% mindestens eines kosmetischen Wirkstoffes aus der Gruppe
b1) der Oxidationsfarbstoffvorprodukte und/oder
b2) der direktziehenden Farbstoffe und/oder
b3) der Oxidationsmittel ausgewählt aus Wasserstoffperoxid und dessen Anlagerungsverbindungen an feste Träger und/oder
b4) der Tenside und/oder Emulgatoren und/oder
b5) der haarkonditionierenden Wirkstoffe und/oder
b6) der desodorierenden und/oder der schweißhemmenden Wirkstoffe und/oder
b7) der hautaufhellenden und/oder hautberuhigenden und/oder feuchtigkeitsspendenden Wirkstoffe und/oder
b8) der anorganischen und/oder organischen UV-Filtersubstanzen und/oder
b9) der sebumregulierenden Wirkstoffe und/oder der mechanischen Exfoliationsmittel und/oder der antimikrobiellen Wirkstoffe und/oder
b10) der haarfestigenden oder Haarstyling-Wirkstoffe und/oder
b11) der Antikaries-Wirkstoffe und/oder
b12) der Wirkstoffe gegen Zahnsteinbildung und/oder
b13) der Mischungen dieser Wirkstoffe b1) - b12).

2. Zusammensetzung nach Anspruch 1, **dadurch gekennzeichnet, dass** der mindestens eine aromatische Alkohol a)i) ausgewählt ist aus Phenol, den Kresolen (*o*-, *m*- und *p*-Kresol), den Naphtholen (α- und β-Naphthol), Thymol, Brenzcatechin, Resorcin, Hydrochinon und 1,4-Naphthohydrochinon, Phloroglucin, Pyrogallol, Hydroxyhydrochinon.

3. Zusammensetzung nach einem der vorherigen Ansprüche, **dadurch gekennzeichnet, dass** die aldehydische Komponente a)ii) ausgewählt ist aus Valeraldehyd, Capronaldehyd, Caprylaldehyd, Decanal, Succindialdehyd, Cyclohexancarbaldehyd, Cyclopentancarbaldehyd, 2-Methyl-1-propanal , 2-Methylpropionaldehyd, Acetaldehyd, Acrolein, Aldosteron, Antimycin A, 8'-Apo-β-caroten-8'-al, Benzaldehyd, Butanal, Chloral, Citral, Citronellal, Crotonaldehyd, Dimethylaminobenzaldehyd, Folinsäure, Fosmidomycin, Furfural, Glutaraldehyd, Glycerinaldehyd, Glykolaldehyd, Glyoxal, Glyoxylsäure, Heptanal, 2-Hydroxybenzaldehyd, 3-Hydroxybutanal, Hydroxymethylfurfural, 4-Hydroxynonenal, Isobutanal, Isobutyraldehyd, Methacrolein, 2-Methylundecanal, Mucochlorsäure, N-Methylformamid, 2-Nitrobenzaldehyd, Nonanal, Octanal, Oleocanthal, Orlistat, Pentanal, Phenylethanal, Phycocyanin, Piperonal, Propanal, Propenal, Protocatechualdehyd, Retinal, Salicylaldehyd, Secologanin, Streptomycin, Strophanthidin, Tylosin, Vanillin, Zimtaldehyd.

4. Zusammensetzung nach einem der vorherigen Ansprüche, **dadurch gekennzeichnet, dass** das molare Verhältnis von dem mindestens einen aromatischen Alkohol a)i) zu der mindestens einen aldehydischen Komponente a)ii), die mindestens zwei C-Atome pro Molekül aufweist, zwischen 1 zu 2 und 1 zu 3,5, bevorzugt zwischen 1 zu 2,4 und 1 zu 2,8 und besonders bevorzugt bei 1 zu 2,6 liegt.

5. Zusammensetzung nach einem der vorherigen Ansprüche, **dadurch gekennzeichnet, dass** sie - bezogen auf ihr Gewicht - 0,1 bis 25 Gew.-%, vorzugsweise 0,25 bis 20 Gew.-%, weiter bevorzugt 0,5 bis 15 Gew.-% und insbesondere 1 bis 10 Gew.-% Mikrokapseln a) enthält.

6. Zusammensetzung nach einem der vorherigen Ansprüche, **dadurch gekennzeichnet, dass** sie als Mittel zum Färben und/oder Aufhellen keratinischer Fasern konfektioniert ist und als Oxidationsfarbstoffvorprodukt mindestens eine Entwicklerkomponente und/oder mindestens eine Kupplerkomponente enthält, wobei bevorzugte Entwicklerkomponenten ausgewählt sind aus p-Phenylendiamin, p-Toluylendiamin, N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'-diaminophenyl)-1,4,7,10-tetraoxadecan, 4-Aminophenol, 4-Amino-3-methylphenol, Bis-(5-amino-2-hydroxyphenyl)methan, 2,4,5,6- Tetraaminopyrimidin, 2-Hydroxy-4,5,6-triaminopyrimidin, 4,5-Diamino-1-(2-hydroxyethyl)-pyrazol und bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

7. Zusammensetzung nach einem der vorherigen Ansprüche, **dadurch gekennzeichnet, dass** sie als desodorierendes oder schweißhemmendes Produkt konfektioniert ist und mindestens einen schweißhemmenden Wirkstoff, ausgewählt aus Aluminiumchlorhydroxiden, Aluminiumzirconiumchlorhydraten und Mischungen hiervon und/oder mindestens einen in einer Mikrokapsel a) verkapselten deodorierend wirkenden aromatischen Alkohol der Struktur (AA-1) enthält, 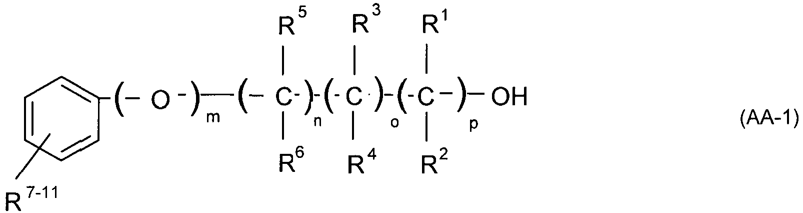
wobei R¹ - R⁶ = unabhängig voneinander ein Wasserstoffatom, eine Alkylgruppe mit 1 bis 10 Kohlenstoffatomen, die linear oder verzweigt sein kann und die substituiert sein kann mit OH-Gruppen oder Alkoxy-Gruppen mit 1 bis 5 Kohlenstoffatomen, oder eine Alkenylgruppe mit 2 bis 10 Kohlenstoffatomen, die linear oder verzweigt sein kann und die substituiert sein kann mit OH-Gruppen oder Alkoxy-Gruppen mit 1 bis 5 Kohlenstoffatomen,
R⁷ - R¹¹ = unabhängig voneinander ein Wasserstoffatom, ein Halogenatom, insbesondere ein Chloratom, oder eine Alkylgruppe mit 1 bis 10 Kohlenstoffatomen, die linear oder verzweigt sein kann und die substituiert sein kann mit OH-Gruppen oder Alkoxy-Gruppen mit 1 bis 5 Kohlenstoffatomen, insbesondere mit einer Methoxygruppe, m = 0 oder 1 ist, n, o, p = unabhängig voneinander ganze Zahlen von 0 bis 10 sein können, wobei mindestens einer der Werte n, o, p ≠ 0 ist.

8. Zusammensetzung nach einem der vorherigen Ansprüche, **dadurch gekennzeichnet, dass** sie als Haarreinigungsmittel konfektioniert ist und - bezogen auf ihr Gewicht - 0,05 bis 30 Gew.-% mindestens eines anionischen und/oder amphoteren Tensids enthält.

9. Zusammensetzung nach einem der vorherigen Ansprüche, **dadurch gekennzeichnet, dass** sie als Haarpflegemittel konfektioniert ist und - bezogen auf ihr Gewicht - 0,05 bis 30 Gew.-% mindestens eines Pflegestoffes aus der Gruppe der QAV und/oder der kationischen Polymere und/oder der Silikone enthält.

10. Zusammensetzung nach einem der vorherigen Ansprüche, **dadurch gekennzeichnet, dass** die Mikrokapseln a) mindestens einen der folgenden Inhaltsstoffe verkapselt enthalten:
- Alkohole, insbesondere solche der Formel AA-1,
- Parfümöle,
- Pflegestoffe,
- natürliche oder synthetische Öle,
- Silikonöle,
- Farbstoffe,
- kühlende Substanzen.

11. Zusammensetzung nach einem der vorherigen Ansprüche, **dadurch gekennzeichnet, dass** mindestens eine Riechstoffverbindung in einer Gesamtmenge von 0,01 - 5 Gew.-%, bevorzugt 0,1 - 2 Gew.-% und besonders bevorzugt 0,2 - 1 Gew.-%, jeweils bezogen auf das Gesamtgewicht der Riechstoffverbindung(en) in der Zusammensetzung, enthalten ist, wobei die Riechstoffverbindung(en) in den erfindungsgemäßen Mikrokapseln a) verkapselt vorliegt bzw. vorliegen.

12. Zusammensetzung nach einem der vorherigen Ansprüche, **dadurch gekennzeichnet, dass** mindestens eine in den erfindungsgemäßen Mikrokapseln a) verkapselte Riechstoffverbindung in einer Gesamtmenge von 0,01 - 5 Gew.-%, bevorzugt 0,1 -2 Gew.-% und besonders bevorzugt 0,2 - 1 Gew.-%, und mindestens eine unverkapselte Riechstoffverbindung in einer Gesamtmenge von 0,01 - 5 Gew.-%, bevorzugt 0,1 - 2 Gew.-% und besonders bevorzugt 0,2 - 1 Gew.-%, jeweils bezogen auf das Gesamtgewicht der Riechstoffverbindung(en) in der Zusammensetzung, enthalten sind.

13. Zusammensetzung nach einem der vorherigen Ansprüche, **dadurch gekennzeichnet, dass** ihr Wassergehalt mindestens 40 Gew.-%, bevorzugt 45 bis 90 Gew.-%, besonders bevorzugt 50 - 85 Gew.-%, außerordentlich bevorzugt 55 - 80 Gew.-%, jeweils bezogen auf die Gesamtzusammensetzung, beträgt.

14. Zusammensetzung nach einem der vorherigen Ansprüche, **dadurch gekennzeichnet, dass** sie als Körperreinigungsmittel, insbesondere als Duschgel, Duschbad oder Flüssigseife, konfektioniert sind, und - jeweils bezogen auf ihr Gewicht - 5 bis 30, bevorzugt 7 bis 20 und insbesondere 8 bis 14 Gew.-% Tenside, die bevorzugt aus der Gruppe der anionischen, amphoteren, zwitterionischen und nichtionischen Tenside sowie aus Gemischen dieser Tensidklassen ausgewählt sind, sowie 0,1 bis 10 Gew.-%, vorzugsweise 0,25 bis 5 Gew.-%, weiter bevorzugt 0,5 bis 3 Gew.-% und insbesondere 1 bis 2 Gew.-% erfindungsgemäße Mikrokapseln a) enthalten.

## Claims

1. A cosmetic composition, containing, based on its weight,
a) microcapsules of which the capsule walls comprise a resin that can be obtained by reacting
i) at least one aromatic alcohol or the ethers or esters thereof and
ii) at least one aldehydic component that comprises at least two C atoms per molecule, and
iii) in the presence of at least one homopolymer or copolymer of 2-acrylamido-2-methyl-propane sulfonic acid or the salts thereof
b) from 0.01 to 70 wt.% of at least one cosmetic active ingredient from the group of
b1) oxidation dye precursors and/or
b2) direct dyes and/or
b3) oxidizing agents selected from hydrogen peroxide, and the addition compounds thereof on solid carriers and/or
b4) surfactants and/or emulsifiers and/or
b5) hair-conditioning active ingredients and/or
b6) deodorizing and/or antiperspirant active ingredients and/or
b7) skin-lightening and/or skin-soothing and/or moisturizing active ingredients and/or
b8) inorganic and/or organic UV filter substances and/or
b9) sebum-regulating active ingredients and/or mechanical exfoliating agents and/or antimicrobial active ingredients and/or
b10) hair-setting or hair-styling active ingredients and/or
b11) anticariogenic active ingredients and/or
b12) active ingredients that prevent tartar formation and/or
b13) mixtures of these active ingredients b1) - b12).

2. The composition according to claim 1, **characterized in that** the at least one aromatic alcohol a)i) is selected from phenol, the cresols (*o*-, *m-* and *p*-cresol), the naphthols (α- and β-naphthol), thymol, pyrocatechol, resorcinol, hydroquinone and 1,4-naphthohydroquinone, phloroglucinol, pyrogallol, or hydroxyhydroquinone.

3. The composition according to one of the preceding claims, **characterized in that** the aldehydic component a)ii) is selected from valeraldehyde, capronaldehyde, caprylaldehyde, decanal, succindialdehyde, cyclohexanecarboxaldehyde, cyclopentanecarboxaldehyde, 2-methyl-1-propanal, 2-methylpropionaldehyde, acetaldehyde, acrolein, aldosterone, antimycin A, 8'-apo-β-carotene-8'-al, benzaldehyde, butanal, chloral, citral, citronellal, crotonaldehyde, dimethylaminobenzaldehyde, folinic acid, fosmidomycin, furfural, glutaraldehyde, glyceraldehyde, glycolaldehyde, glyoxal, glyoxylic acid, heptanal, 2-hydroxybenzaldehyde, 3-hydroxybutanal, hydroxymethylfurfural, 4-hydroxynonenal, isobutanal, isobutyraldehyde, methacrolein, 2-methylundecanal, mucochloric acid, N-methylformamide, 2-nitrobenzaldehyde, nonanal, octanal, oleocanthal, orlistat, pentanal, phenylethanal, phycocyanin, piperonal, propanal, propenal, protocatechuic aldehyde, retinal, salicylaldehyde, secologanin, streptomycin, strophanthidin, tylosin, vanillin, or cinnamaldehyde.

4. The composition according to one of the preceding claims, **characterized in that** the molar ratio of the at least one aromatic alcohol a)i) to the at least one aldehydic component a)ii), which comprises at least two C atoms per molecule, is between 1:2 and 1:3.5, preferably between 1:2.4 and 1:2.8, and is particularly preferably 1:2.6.

5. The composition according to one of the preceding claims, **characterized in that** it contains, based on its weight, from 0.1 to 25 wt.%, preferably from 0.25 to 20 wt.%, more preferably from 0.5 to 15 wt.%, and in particular from 1 to 10 wt.%, of microcapsules a).

6. The composition according to one of the preceding claims, **characterized in that** it is formulated as an agent for coloring and/or lightening keratin fibers and contains at least one developer component and/or at least one coupler component as an oxidation dye precursor, preferred developer components being selected from p-phenylenediamine, p-toluenediamine, N,N-bis-(2-hydroxyethyl)amino-p-phenylenediamine, 1,3-bis-[(2-hydroxyethyl-4'-aminophenyl)amino]-propan-2-ol, 1,10-bis-(2',5'-diaminophenyl)-1,4,7,10-tetraoxadecane, 4-aminophenol, 4-amino-3-methylphenol, bis-(5-amino-2-hydroxyphenyl)methane, 2,4,5,6-tetraaminopyrimidine, 2-hydroxy-4,5,6-triaminopyrimidine, 4,5-diamino-1-(2-hydroxyethyl)-pyrazole, and preferred coupler components being selected from resorcinol, 2-methylresorcinol, 5-methylresorcinol, 2,5-dimethylresorcinol, 4-chlororesorcinol, resorcinol monomethyl ether, 5-aminophenol, 5-amino-2-methylphenol, 5-(2-hydroxyethyl)amino-2-methylphenol, 3-amino-4-chloro-2-methylphenol, 3-amino-2-chloro-6-methylphenol, 3-amino-2,4-dichlorophenol, 2,4-diaminophenoxyethanol, 2-amino-4-(2'-hydroxyethyl)amino-anisole sulfate, 1,3-bis-(2,4-diaminophenoxy)propane, 2-amino-3-hydroxypyridine, 2-methylamino-3-amino-6-methoxypyridine, 2,6-dihydroxy-3,4-dimethylpyridine, 3,5-diamino-2,6-dimethoxypyridine, 1-naphthol, 2-methyl-1-naphthol, 1,5-dihydroxynaphthaline, 2,7-dihydroxynaphthaline, 1-phenyl-3-methylpyrazol-5-one, 2,6-bis-[(2'-hydroxyethyl)amino]-toluene, 4-hydroxyindole, 6-hydroxyindole, or 6-hydroxybenzomorpholine.

7. The composition according to one of the preceding claims, **characterized in that** it is formulated as a deodorizing or antiperspirant product and contains at least one antiperspirant active ingredient selected from aluminum chlorhydroxides, aluminum zirconium chlorohydrates and mixtures thereof and/or at least one aromatic alcohol of structure (AA-1) which is encapsulated in a microcapsule a) and has a deodorizing effect 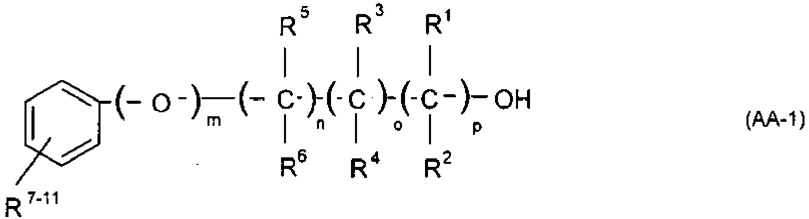 where R¹ - R⁶ are, independently of one another, a hydrogen atom, an alkyl group having 1 to 10 carbon atoms, which can be linear or branched and can be substituted with OH groups or alkoxy groups having 1 to 5 carbon atoms, or an alkenyl group having 2 to 10 carbon atoms, which can be linear or branched and can be substituted with OH groups or alkoxy groups having 1 to 5 carbon atoms, R⁷ - R¹¹ are, independently of one another, a hydrogen atom, a halogen atom, in particular a chlorine atom, or an alkyl group having 1 to 10 carbon atoms, which can be linear or branched and can be substituted with OH groups or alkoxy groups having 1 to 5 carbon atoms, in particular with a methoxy group, m is 0 or 1, and n, o and p can be, independently of one another, integers from 0 to 10, at least one of the values n, o or p not being equal to 0.

8. The composition according to one of the preceding claims, **characterized in that** it is formulated as a hair cleansing agent, and contains, based on its weight, from 0.05 to 30 wt.% of at least one anionic and/or amphoteric surfactant.

9. The composition according to one of the preceding claims, **characterized in that** it is formulated as a hair care agent and contains, based on its weight, from 0.05 to 30 wt.% of at least one care substance from the group of QACs and/or cationic polymers and/or silicones.

10. The composition according to one of the preceding claims, **characterized in that** the microcapsules a) contain at least one of the following ingredients in encapsulated form:
- alcohols, in particular those of formula AA-1,
- perfume oils,
- care substances,
- natural or synthetic oils,
- silicone oils,
- dyes,
- cooling substances.

11. The composition according to one of the preceding claims, **characterized in that** at least one odorant compound is contained in a total amount of from 0.01 to 5 wt.%, preferably from 0.1 to 2 wt.%, and particularly preferably from 0.2 to 1 wt.%, in each case based on the total weight of the odorant compound(s) in the composition, the odorant compound(s) being encapsulated in the microcapsules a) according to the invention.

12. The composition according to one of the preceding claims, **characterized in that** at least one odorant compound encapsulated in the microcapsules a) according to the invention is contained in a total amount of from 0.01 to 5 wt.%, preferably from 0.1 to 2 wt.%, and particularly preferably from 0.2 to 1 wt.%, and at least one unencapsulated odorant compound is contained in a total amount of from 0.01 to 5 wt.%, preferably from 0.1 to 2 wt.%, and particularly preferably from 0.2 to 1 wt.%, in each case based on the total weight of the odorant compound(s) in the composition.

13. The composition according to one of the preceding claims, **characterized in that** the water content thereof is at least 40 wt.%, preferably from 45 to 90 wt.%, particularly preferably from 50 to 85 wt.%, extremely preferably from 55 to 80 wt.%, in each case based on the total composition.

14. The composition according to one of the preceding claims, **characterized in that** it is formulated as a body cleansing agent, in particular a shower gel, bodywash or liquid soap, and contains, in each case based on its weight, from 5 to 30 wt.%, preferably from 7 to 20 wt.%, and in particular from 8 to 14 wt.%, of surfactants which are preferably selected from the group of anionic, amphoteric, zwitterionic and non-ionic surfactants and mixtures of these classes of surfactants, and from 0.1 to 10 wt.%, preferably from 0.25 to 5 wt.%, more preferably from 0.5 to 3 wt.%, and in particular from 1 to 2 wt.%, of microcapsules a) according to the invention.

## Revendications

1. Composition cosmétique contenant, par rapport à son poids total,
a) des microcapsules dont les parois comprennent une résine obtenue :
i) par réaction d'au moins un alcool aromatique ou de son éther ou ester,
ii) par réaction d'au moins un composant aldéhyde composé d'au moins deux atomes de carbone par molécule, et
iii) en présence d'au moins un homopolymère ou copolymère d'acide 2-acrylamido-2-méthylpropanesulfonique ou de ses sels ;
b) 0,01 à 70 % en poids d'au moins un principe actif cosmétique du groupe constitué par :
b1) des précurseurs de colorants d'oxydation et/ou
b2) des colorants directs et/ou
b3) des agents oxydants choisis parmi le peroxyde d'hydrogène et ses composés d'addition à des supports solides et/ou
b4) des tensioactifs et/ou des émulsifiants et/ou
b5) des substances de conditionnement capillaire et/ou
b6) des principes actifs désodorisants et/ou anti-transpirants actives et/ou
b7) des principes actifs éclaircissants et/ou apaisants et/ou hydratants pour la peau et/ou
b8) des filtres à rayons UV inorganiques et/ou organiques et/ou
b9) des principes actifs régulateurs de sébum et/ou des agents d'exfoliation mécaniques et/ou des principes actifs antimicrobiens et/ou
b10) des principes actifs fixant les cheveux ou coiffants et/ou
b11) des principes actifs anti-caries et/ou
b12) des principes actifs contre la formation de tartre et/ou
b13) des mélanges de ces principes actifs b1) à b12).

2. Composition selon la revendication 1, **caractérisée en ce que** l'au moins un alcool aromatique a) i) est choisi parmi le phénol, les crésols (*o*-, *m*-et *p-crésol*), les naphtols (α- et β-naphtol), le thymol, le pyrocatéchol, le résorcinol, l'hydroquinone et la 1,4-naphtohydroquinone, le phloroglucinol, le pyrogallol et l'hydroxyhydroquinone.

3. Composition selon l'une des revendications précédentes, **caractérisée en ce que** le composant aldéhydique a) ii) est choisi parmi le valéraldéhyde, le capronaldéhyde, le caprylaldéhyde, le décanal, le succindialdéhyde, le cyclohexane carbaldéhyde, le cyclopentane carbaldéhyde, le 2-méthyl-1-propanal, le 2-méthylpropionaldéhyde, l'acétaldéhyde, l'acroléine, l'aldostérone, l'antimycine A, le 8'-apo-β-carotène-8'-al, le benzaldéhyde, le maisanal, le chloral, le citral, le citronellal, le crotonaldéhyde, le diméthylaminobenzaldéhyde, l'acide folique, le fosmidomycine, le furfural, le glutaraldéhyde, le glycéraldéhyde, l'aldéhyde glycolique, le glyoxal, l'acide glyoxylique, l'heptanal, le 2-hydroxybenzaldéhyde, le 3-hydroxybutanal, l'hydroxyméthylfurfural, le 4-hydroxynonenal, l'isobutanal, l'isobutyraldéhyde, la méthacroléine, le 2-méthylundecanal, l'acide mucochlorique, le N-methylformamide, le 2-nitrobenzaldehyde, le nonanal, l'octanal, l'oléocanthal, l'orlistate, le pentanal, le phényléthanal, la phycocyanine, le pipéronal, le propanal, le propénal, le protocatechualdéhyde, le rétinal, la salicylaldéhyde, la sécologanine, la streptomycine, la strophanthidine, la tylosine, la vanilline, l'aldéhyde cinnamique.

4. Composition selon l'une des revendications précédentes, **caractérisée en ce que** le rapport molaire de l'au moins un alcool aromatique a) i) à l'au moins un composant aldéhydique a) ii), lequel comporte au moins deux atomes de carbone par molécule, est compris entre 1 et 2 et 1 et 3,5, de préférence entre 1 et 2,4 et 1 et 2,8 et en particulier de préférence entre 1 et 2,6.

5. Composition selon l'une des revendications précédentes, **caractérisée en ce qu'**elle contient, par rapport à son poids, 0,1 à 25 % en poids, de préférence 0,25 à 20 % en poids, de manière davantage préférée 0,5 à 15 % en poids et en particulier 1 à 10 % en poids de microcapsules a).

6. Composition selon l'une des revendications précédentes, **caractérisée en ce qu'**elle est formulée sous forme d'agent de coloration et/ou d'éclaircissement de fibres kératiniques, et contient au moins un constituant révélateur et/ou au moins un constituant coupleur comme précurseur de colorant d'oxydation, les constituants révélateurs préférés étant choisis parmi la p-phénylènediamine, la p-toluylènediamine, la N,N-bis-(2-hydroxyéthyl)amino-p-phénylènediamine, le 1,3-bis-[(2-hydroxyéthyl-4'-aminophényl)amino]propane-2-ol, le 1,10-bis-(2',5'-diaminophényl)-1,4,7,10-tétraoxadécane, le 4-aminophénol, le 4-amino-3-méthylphénol, le bis-(5-amino-2-hydroxyphényl)méthane, la 2,4,5,6 tétraaminopyrimidine, la 2-hydroxy-4,5,6-triaminopyrimidine, le 4,5-diamino-1-(2-hydroxyéthyl)-pyrazole, et les constituants coupleurs préférés étant choisis parmi le résorcinol, le 2-méthylrésorcinol, le 5-méthylrésorcinol, le 2,5-diméthylrésorcinol, le 4-chlororésorcinol, le monométhyléther de résorcinol, le 5-aminophénol, le 5-amino-2-méthylphénol, le 5-(2-hydroxyéthyl)amino-2-méthylphénol, le 3-amino-4-chloro-2-méthylphénol, le 3-amino-2-chior-6-méthylphénol, le 3-amino-2,4-dichlorophénol, le 2,4-diaminophénoxyéthanol, 2-amino-4-(2'-hydroxyéthyl)amino-anisol, 1,3-bis(2,4-diaminophénoxy)propane, la 2-amino-3-hydroxypyridine, la 2-méthylamino-3-amino-6-méthoxypyridine, la 2,6-dihydroxy-3,4-diméthylpyridine, la 3,5-diamino-2,6-diméthoxypyridine, le 1-naphthol, le 2-méthyl-1-naphthol, le 1,5-dihydroxynaphtalène, le 2,7-dihydroxynaphtalène, le 1-phényl-3-méthylpyrazol-5-one, le 2,6-bis[(2'-hydroxyéthyl)amino]toluène, le 4-hydroxyindole, le 6-hydroxyindole et la 6-hydroxybenzomorpholine.

7. Composition selon l'une des revendications précédentes, **caractérisée en ce qu'**elle est formulée sous forme de produit désodorisant ou anti-transpirant, et contient au moins un principe actif anti-transpirant choisi parmi les chlorohydroxydes d'aluminium, les chlorhydrates d'aluminium-zirconium et leurs mélanges et/ou au moins un alcool aromatique désodorisant encapsulé dans une microcapsule a) et de structure (AA-1) 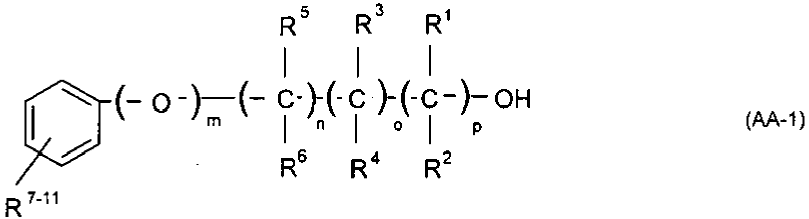 dans laquelle R¹ à R⁶ représentent indépendamment l'un de l'autre un atome d'hydrogène, un groupe alkyle ayant 1 à 10 atomes de carbone, lequel peut être linéaire ou ramifié et peut être substitué par des groupes OH ou des groupes alcoxy ayant 1 à 5 atomes de carbone, ou un groupe alcényle ayant 2 à 10 atomes de carbone, lequel peut être linéaire ou ramifié et peut être substitué par des groupes OH ou des groupes alcoxy ayant 1 à 5 atomes de carbone, R⁷ à R¹¹ représentent indépendamment l'un de l'autre un atome d'hydrogène, un atome d'halogène, notamment un atome de chlore, ou un groupe alkyle ayant 1 à 10 atomes de carbone, lequel peut être linéaire ou ramifié et peut être substitué par des groupes OH ou des groupes alkoxy ayant 1 à 5 atomes de carbone, notamment par un groupe méthoxy, m représente 0 ou 1, n, o et p peuvent représenter indépendamment l'un de l'autre des nombres entiers compris entre 0 et 10, l'une des valeurs n, o et p au moins étant différente de 0.

8. Composition selon l'une des revendications précédentes, **caractérisée en ce qu'**elle est formulée sous forme d'agent nettoyant capillaire, et contient, par rapport à son poids, 0,05 à 30 % en poids d'au moins un tensioactif anionique et/ou amphotère.

9. Composition selon l'une des revendications précédentes, **caractérisée en ce qu'**elle est formulée sous forme d'agent de soin capillaire, et contient, par rapport à son poids, 0,05 à 30 % en poids d'au moins une substance de soin du groupe constitué par des CAQ et/ou des polymères cationiques et/ou des silicones.

10. Composition selon l'une des revendications précédentes, **caractérisée en ce que** les microcapsules a) contiennent au moins un des ingrédients suivants encapsulés :
- des alcools, en particulier ceux de formule AA-1,
- des huiles de parfum,
- des substances de soin,
- des huiles naturelles ou synthétiques,
- des huiles de silicone,
- des colorants,
- des substances réfrigérantes.

11. Composition selon l'une des revendications précédentes, **caractérisée en ce qu'**au moins un composé de parfum est présente en une quantité totale située dans la plage allant de 0,01 à 5 % en poids, de préférence de 0,1 à 2 % en poids et de manière particulièrement préférée de 0,2 à 1 % en poids, par rapport au poids total du ou des composés de parfum dans la composition, le ou les composés de parfum étant présents dans les microcapsules a) encapsulées selon l'invention.

12. Composition selon l'une des revendications précédentes, **caractérisée en ce qu'**elle contient au moins un composé de parfum encapsulé dans les microcapsules a) selon l'invention en une quantité totale située dans la plage allant de 0,01 à 5 % en poids, de préférence de 0,1 à 2 % en poids et de manière particulièrement préférée de 0,2 à 1 % en poids, et au moins un composé de parfum non encapsulé en une quantité totale située dans la plage allant de 0,01 à 5 % en poids, de préférence de 0,1 à 2 % en poids et de manière particulièrement préférée de 0,2 à 1 % en poids, par rapport au poids total du ou des composés de parfum dans la composition.

13. Composition selon l'une des revendications précédentes, **caractérisée en ce que** sa teneur en eau est d'au moins 40 % en poids, de préférence de 45 à 90 % en poids, de manière particulièrement préférée de 50 à 85 % en poids et de manière préférée entre toutes de 55 à 80 % en poids, dans chaque cas par rapport à la composition totale.

14. Composition selon l'une des revendications précédentes, **caractérisée en ce qu'**elle est formulée sous forme de produit de nettoyage du corps, en particulier sous forme de gel douche, de bain de douche ou de savon liquide, et contient, par rapport à leur poids, 5 à 30 %, de préférence 7 à 20 % et en particulier 8 à 14 % en poids des tensioactifs, de préférence choisis dans le groupe des tensioactifs anioniques, amphotères, zwitterioniques et non-ioniques, et parmi des mélanges de ces classes de tensioactifs, et 0,1 à 10 % en poids, de préférence 0,25 à 5 % en poids, de manière davantage préférée 0,5 à 3 % en poids et en particulier 1 à 2 % en poids des microcapsules a) selon l'invention.
